# EUROPEAN PATENT APPLICATION

(11) **EP 4 706 518 A1**
(43) Date of publication of application: **11.03.2026**
(21) Application number: 24825221.5
(22) Date of filing: 18.06.2024
(51) Int. Cl.: A61B 5/0225, A61B 5/021

(54) **MEASUREMENT METHOD AND RELATED APPARATUS**

(30) Priority: 19.06.2023 CN 202310728736; 14.06.2024 CN 202410778424
(71) Applicant: Huawei Technologies Co., Ltd., Shenzhen, Guangdong 518129 (CN)
(72) Inventor: JIN, Junye, Shenzhen, Guangdong 518129 (CN); CHEN, Qing, Shenzhen, Guangdong 518129 (CN); HUANG, Yadong, Shenzhen, Guangdong 518129 (CN); WANG, Youhua, Shenzhen, Guangdong 518129 (CN); LU, Shiqiang, Shenzhen, Guangdong 518129 (CN); JIN, Yongfu, Shenzhen, Guangdong 518129 (CN)
(74) Representative: Isarpatent
(86) International application number: PCT/CN2024/099741
(87) International publication number: WO 2024/260326

(57) **Abstract**

This application discloses a measurement method and a related apparatus, applied to an electronic device including a micro air pump and an airbag. The electronic device may obtain an environment parameter, where the environment parameter includes any one or more of the following: a performance level of the micro air pump, a model of the airbag, and a measurement posture. The electronic device determines a first condition and a first threshold based on the environment parameter. The electronic device controls the micro air pump to inflate the airbag, and determines a first parameter when detecting that the first condition is satisfied. The first parameter is compared with the first threshold, and wearing tightness is determined based on a comparison result. In this way, wearing tightness of a user can be determined in a physiological parameter measurement, to avoid an error caused by the wearing tightness in the physiological parameter measurement process. In addition, the environment parameter is further considered in the wearing tightness measurement of the user, so that a measurement error caused by different environment parameters can be avoided.

## Description

This application claims priorities to Chinese Patent Application 202310728736.X, filed with the China National Intellectual Property Administration on June 19, 2023 and entitled "MEASUREMENT METHOD AND RELATED APPARATUS", and to Chinese Patent Application No. 202410778424.4, filed with the China National Intellectual Property Administration on June 14, 2024 and entitled "MEASUREMENT METHOD AND RELATED APPARATUS", both of which are incorporated herein by reference in their entireties.

### TECHNICAL FIELD

This application relates to the field of electronic technologies, and in particular, to a measurement method and a related apparatus.

### BACKGROUND

With continuous development of electronic technologies, functions of a wearable device are increasingly diversified, and a user may use the wearable device to measure physiological parameters such as blood pressure, blood oxygen, and a heart rate.

When the user wears the wearable device, the wearable device may collect a pulse wave signal of a wrist artery of the user according to an oscillographic method, and obtain a blood pressure value of the user through analysis. However, this measurement manner has a requirement on tightness of wearing the wearable device, and the wearing tightness of the user affects a measurement result.

### SUMMARY

This application provides a measurement method and a related apparatus, to measure wearing tightness of a user when the user wears an electronic device.

According to a first aspect, this application provides another measurement method, applied to an electronic device. The electronic device includes a micro air pump and an airbag, and the method includes: determining a first condition and a first threshold based on an environment parameter, where the environment parameter includes any one or more of the following: a performance level of the micro air pump, a model of the airbag, and a measurement posture of a user; controlling the micro air pump to inflate the airbag; determining a first parameter when the first condition is satisfied; and comparing the first parameter with the first threshold, and determining, based on a comparison result, that tightness of wearing the electronic device is first wearing tightness.

In this way, the wearing tightness of the user can be measured based on the environment parameter, to avoid a measurement error caused by different environment parameters, and improve measurement accuracy of the wearing tightness.

In a possible implementation, the wearing tightness includes proper wearing, slightly loose wearing, and excessively loose wearing.

It should be noted that, slightly loose wearing may also be referred to as little loose wearing.

In a possible implementation, the method further includes: determining, based on the environment parameter, that an operating parameter of the micro air pump is a first operating parameter; and controlling the micro air pump to inflate the airbag specifically includes: controlling the micro air pump to inflate the airbag based on the first operating parameter.

In this way, the operating parameter of the micro air pump can be set based on the environment parameter.

In a possible implementation, the electronic device further includes a Hall effect sensor; and before determining the first condition and the first threshold based on the environment parameter, the method further includes: measuring the model of the airbag based on the Hall effect sensor.

In this way, the model of the airbag can be measured.

In a possible implementation, the electronic device further includes an inertial measurement sensor, the inertial measurement sensor includes an acceleration sensor and/or a gyroscope sensor, and before determining the first condition and the first threshold based on the environment parameter, the method further includes: measuring the measurement posture of the user based on the inertial measurement sensor.

In this way, the measurement posture of the user can be obtained.

In a possible implementation, before determining the first condition and the first threshold based on the environment parameter, the method further includes: setting the operating parameter of the micro air pump to the first operating parameter; setting initial air pressure of the airbag to a first air pressure value, and setting end air pressure of the airbag to a second air pressure value; controlling the micro air pump to inflate the airbag based on the first operating parameter, and obtaining first duration consumed for air pressure of the airbag to change from the first air pressure value to the second air pressure value; and determining the performance level of the micro air pump based on the first operating parameter, the first air pressure value, the second air pressure value, and the first duration.

In this way, the performance level of the micro air pump can be measured.

In a possible implementation, measuring the model of the airbag based on the Hall effect sensor specifically includes: when a first measurement condition is satisfied, measuring the model of the airbag based on the Hall effect sensor, where the first measurement condition includes any one or more of the following: The first wearing tightness is measured for a first time, and the user removes and mounts the airbag.

In this way, when the first measurement condition is satisfied, the measurement of the model of the airbag may be triggered. There is no need to measure the model of the airbag each time the wearing tightness is measured, so that power consumption is reduced.

In a possible implementation, obtaining the performance level of the micro air pump specifically includes: when a second measurement condition is satisfied, obtaining the performance level of the micro air pump, where the second measurement condition includes any one or more of the following: The first wearing tightness is measured for a first time, and a time interval from measuring the performance level of the micro air pump last time reaches a first time interval.

In this way, when the second measurement condition is satisfied, the measurement of the performance level of the micro air pump may be triggered. There is no need to measure the performance level of the micro air pump each time the wearing tightness is measured, so that power consumption is reduced.

In a possible implementation, the first condition is that inflation time of the micro air pump reaches fifth duration, the first threshold includes a fifth air pressure value, and the first parameter is air pressure of the airbag when the first condition is satisfied.

In a possible implementation, the first condition is that the air pressure of the airbag reaches a seventh air pressure value, the first threshold includes sixth duration, and the first parameter is inflation time of the micro air pump when the first condition is satisfied.

In a possible implementation, if the first wearing tightness is proper wearing, the method further includes: controlling the micro air pump to inflate the airbag to obtain a first blood pressure measurement value; and outputting the first blood pressure measurement value.

In a possible implementation, the wearing tightness includes proper wearing, slightly loose wearing, and excessively loose wearing; and if the first wearing tightness is slightly loose wearing, the method further includes: obtaining a maximum fluctuation value of a pulse wave amplitude; and determining second wearing tightness based on the maximum fluctuation value of the pulse wave amplitude and a preset fluctuation threshold.

In this way, in the case of slightly loose wearing, the wearing tightness of the user may be further determined based on fluctuation of the pulse wave amplitude.

In a possible implementation, if the first wearing tightness is slightly loose wearing, the method further includes: displaying a slightly-loose-wearing prompt, where the slightly-loose-wearing prompt includes a first control, the first control is used to trigger the electronic device not to display the slightly-loose-wearing prompt when slightly loose wearing is detected, and the slightly-loose-wearing prompt is used to prompt the user that the first wearing tightness is slightly loose wearing.

In this way, in the case of slightly loose wearing, the slightly-loose-wearing prompt may be output. In addition, the first control used to trigger the electronic device not to display the slightly-loose-wearing prompt may be further displayed.

In a possible implementation, the method further includes: receiving and responding to an operation on the first control, and stopping displaying the slightly-loose-wearing prompt; receiving an operation of measuring the tightness of wearing the electronic device; determining the first condition and the first threshold based on the environment parameter; controlling the micro air pump to inflate the airbag; when the first condition is satisfied, determining a second parameter; comparing the second parameter with the first threshold, and determining, based on a comparison result, that the tightness of wearing the electronic device is third wearing tightness; and if the third wearing tightness is slightly loose wearing, skipping displaying the slightly-loose-wearing prompt.

In this way, based on a user operation, the slightly-loose-wearing prompt may not be displayed.

In a possible implementation, the method further includes: before determining the first condition and the first threshold based on the environment parameter, receiving an operation of starting a wearing tightness measurement; detecting that the electronic device has a not-worn state between the current wearing tightness measurement and a previous wearing tightness measurement; and if the first tightness is tighter than fourth tightness measured last time, skipping displaying the slightly-loose-wearing prompt.

In this way, if the electronic device is removed by the user between two wearing tightness measurements, and the wearing tightness measured this time is tighter than that measured last time, no slightly-loose-wearing prompt is output regardless of whether the wearing tightness measured this time is slightly loose wearing.

In a possible implementation, the method further includes: if the first tightness is tighter than the fourth tightness measured last time, measuring and outputting a second blood pressure measurement value.

In this way, when the wearing tightness measured this time is tighter than that measured last time, the electronic device determines that the electronic device is worn properly, and measures and outputs a blood pressure value.

In a possible implementation, duration of the not-worn state of the electronic device is less than preset removal duration.

In this way, the electronic device may determine, based on a value relationship between the not-worn duration and the preset removal duration, whether the user adjusts the wearing tightness. If the not-worn duration is less than the preset removal duration, the electronic device may determine that the user adjusts the wearing tightness. If the not-worn duration is greater than or equal to the preset removal duration, the electronic device may determine that the user removes the electronic device.

According to a second aspect, this application provides a measurement method, applied to an electronic device. The electronic device includes a micro air pump and an airbag, and the measurement method includes: determining a first condition and a first threshold based on a performance level of the micro air pump; controlling the micro air pump to inflate the airbag; determining a first parameter when the first condition is satisfied; and comparing the first parameter with the first threshold, and determining wearing tightness based on a comparison result.

In this way, wearing tightness of a user can be determined in a physiological parameter measurement, to avoid an error caused by the wearing tightness in the physiological parameter measurement process. In addition, performance of the micro air pump is also considered in the wearing tightness measurement of the user, so that a measurement error caused by performance deterioration of the micro air pump can be avoided.

In a possible implementation, before controlling the micro air pump to inflate the airbag, the method further includes: obtaining a first signal; and determining, based on the first signal, that the electronic device is worn by the user, where the first signal includes a first pulse wave signal detected by a photoplethysmography PPG module and/or a first capacitance signal detected by a capacitive sensor.

In this way, whether the user wears the electronic device may be determined by using data detected by the PPG module or the capacitive sensor. When it is determined that the user wears the electronic device, the wearing tightness of the user may continue to be measured. When it is determined that the user does not wear the electronic device, a prompt may be output to prompt the user to wear the electronic device.

In a possible implementation, before determining the first condition and the first threshold based on the performance level of the micro air pump, the method further includes: setting an operating parameter of the micro air pump to a first operating parameter; setting initial air pressure of the airbag to a first air pressure value, and setting end air pressure of the airbag to a second air pressure value; controlling the micro air pump to inflate the airbag based on the first operating parameter, and obtaining first duration consumed for air pressure of the airbag to change from the first air pressure value to the second air pressure value; and determining the performance level of the micro air pump based on the first operating parameter, the first air pressure value, the second air pressure value, and the first duration.

In this way, when the initial air pressure of the airbag is set to the first air pressure value, the end air pressure is set to the second air pressure value, and the operating parameter of the micro air pump is set to the first operating parameter, the electronic device may determine the performance level of the micro air pump based on the duration consumed for the air pressure of the airbag to change from the initial air pressure to the end air pressure.

In a possible implementation, before determining the first condition and the first threshold based on the performance level of the micro air pump, the method further includes: setting an operating parameter of the micro air pump to a first operating parameter; setting initial air pressure of the airbag to a first air pressure value and inflation duration to second duration; controlling the micro air pump to inflate the airbag based on the first operating parameter, and obtaining a third air pressure value of the airbag when the inflation duration reaches the second duration; and determining the performance level of the micro air pump based on the first operating parameter, the second duration, the first air pressure value, and the third air pressure value.

In this way, after the initial air pressure, the operating parameter of the micro air pump, and the inflation duration are set, the electronic device may determine the performance level of the micro air pump based on the air pressure of the airbag when the inflation duration of the micro air pump reaches the preset duration.

In a possible implementation, before determining the first condition and the first threshold based on the performance level of the micro air pump, the method further includes: setting a first operating parameter and a second operating parameter; setting initial air pressure of the airbag to a first air pressure value, and setting end air pressure of the airbag to a second air pressure value; controlling the micro air pump to inflate the airbag based on the first operating parameter, and obtaining first duration consumed for air pressure of the airbag to change from the first air pressure value to the second air pressure value; controlling the airbag to deflate until the air pressure of the airbag reaches the first air pressure value; controlling the micro air pump to inflate the airbag based on the second operating parameter, and obtaining third duration consumed for the air pressure of the airbag to change from the first air pressure value to the second air pressure value; and determining the performance level of the micro air pump based on the first operating parameter, the second operating parameter, the first air pressure value, the second air pressure value, the first duration, and the third duration.

In this way, the performance level of the micro air pump may be determined by measuring data of duration consumed for the air pressure of the airbag to change from the initial air pressure to the end air pressure based on a plurality of groups (two or more groups) of different operating parameters. This can reduce an error caused by a single measurement and improve measurement accuracy.

In another possible implementation, the electronic device may alternatively determine the performance level of the micro air pump by measuring data of duration consumed for the air pressure of the airbag to change from same initial air pressure to same (or different) end air pressure based on a same operating parameter.

This can also reduce an error caused by a single measurement and improve measurement accuracy.

In a possible implementation, before determining the first condition and the first threshold based on the performance level of the micro air pump, the method further includes: setting a first operating parameter and a second operating parameter; setting initial air pressure of the airbag to a first air pressure value and inflation duration to second duration; controlling the micro air pump to inflate the airbag based on the first operating parameter, and obtaining a third air pressure value of the airbag when the inflation duration reaches the second duration; controlling the airbag to deflate until air pressure of the airbag reaches the first air pressure value; controlling the micro air pump to inflate the airbag based on the second operating parameter, and obtaining a fourth air pressure value of the airbag when the inflation duration reaches the second duration; and determining the performance level of the micro air pump based on the first operating parameter, the second operating parameter, the second duration, the first air pressure value, the third air pressure value, and the fourth air pressure value.

In this way, the performance level of the micro air pump may be determined by measuring data of air pressure of the airbag when the inflation duration of the micro air pump reaches the preset duration based on a plurality of groups (two or more groups) of different operating parameters. This reduces an error caused by a single measurement and improve measurement accuracy.

In a possible implementation, before controlling the micro air pump to inflate the airbag based on the first operating parameter, the method further includes: detecting a first operation of the user, where the first operation is used to trigger the electronic device to detect the performance level of the micro air pump; and displaying an operation guide, where the operation guide is used to prompt the user to place the electronic device on a plane.

In this way, the operation guide may be displayed in response to the operation of measuring performance of the micro air pump by the user, and the performance level of the micro air pump may be detected.

In a possible implementation, before controlling the micro air pump to inflate the airbag based on the first operating parameter, the method further includes: detecting that a performance detection condition is satisfied, where the performance detection condition includes any one or more of the following: time from previous performance detection reaches fourth duration, and a difference between a previous measurement result of a first physiological parameter and an average value of first physiological parameters is greater than a first difference; obtaining a second signal; and determining, based on the second signal, that the electronic device is not worn by the user, where the second signal includes a second pulse wave signal detected by the PPG module and/or a second capacitance signal detected by the capacitive sensor.

In this way, when it is detected that the performance detection condition is satisfied and the user does not wear the electronic device, the electronic device may detect the performance level of the micro air pump by itself.

In a possible implementation, determining the first condition and the first threshold based on the performance level of the micro air pump specifically includes: setting the operating parameter of the micro air pump to a third operating parameter; and determining the first condition and the first threshold based on the performance level and the third operating parameter of the micro air pump; and controlling the micro air pump to inflate the airbag specifically includes: controlling the micro air pump to inflate the airbag based on the third operating parameter.

In this way, the operating parameter of the micro air pump may be first set, and then the first condition and the first threshold are set based on the operating parameter and the performance level of the micro air pump.

In a possible implementation, determining the first condition and the first threshold based on the performance level of the micro air pump specifically includes: setting the first condition and the first threshold; and determining the operating parameter of the micro air pump as a fourth operating parameter based on the performance level of the micro air pump, the first condition, and the first threshold; and controlling the micro air pump to inflate the airbag specifically includes: controlling the micro air pump to inflate the airbag based on the fourth operating parameter.

In this way, the first condition and the first threshold may be first set, and then the operating parameter of the micro air pump is set based on the first condition, the first threshold, and the performance level of the micro air pump.

In a possible implementation, the first condition is that inflation time of the micro air pump reaches fifth duration, the first threshold includes a fifth air pressure value, and the first parameter is air pressure of the airbag when the first condition is satisfied.

When the first condition is the inflation duration of the micro air pump, the first threshold is air pressure of the airbag when the duration of inflating the airbag by the micro air pump reaches preset duration.

In a possible implementation, the method further includes: when the air pressure of the airbag is greater than the fifth air pressure value, continuing to control the micro air pump to inflate the airbag, to obtain a first detection result; and displaying the first detection result.

In this way, when wearing of the user is slightly loose (or proper), the electronic device may continue to measure the physiological parameter, and obtain a detection result of the physiological parameter.

In a possible implementation, obtaining the first detection result specifically includes: obtaining first measurement data; and determining the first detection result based on the first parameter, the first threshold, and the first measurement data.

In this way, the electronic device may calibrate the obtained measurement data, and an output detection result is calibrated measurement data.

In a possible implementation, the method further includes: displaying a first prompt when the air pressure of the airbag is less than or equal to the fifth air pressure value, where the first prompt is used to prompt the user to adjust the wearing tightness.

In this way, in a case of excessively loose wearing, the electronic device may output a wearing tightness prompt, to prompt the user to adjust the wearing tightness.

In a possible implementation, the first threshold further includes a sixth air pressure value, and the sixth air pressure value is greater than the fifth air pressure value. The method further includes: displaying a second prompt when the air pressure of the airbag is greater than the fifth air pressure value and less than or equal to the sixth air pressure value, where the second prompt is used to prompt the user that the first detection result is inaccurate.

In this way, in a case of slightly loose wearing, the electronic device may further output an error prompt, to prompt the user that the measurement result of the physiological parameter may be inaccurate.

In a possible implementation, the first condition is that the air pressure of the airbag reaches a seventh air pressure value, the first threshold includes sixth duration, and the first parameter is inflation time of the micro air pump when the first condition is satisfied.

When the first condition is an air pressure value of the airbag, the first threshold is duration of inflating the airbag by the micro air pump when the air pressure of the airbag reaches a preset air pressure value.

In a possible implementation, the method further includes: when the inflation time of the micro air pump is less than the sixth duration, continuing to control the micro air pump to inflate the airbag, to obtain a first detection result; and displaying the first detection result.

In this way, when wearing of the user is slightly loose (or proper), the electronic device may continue to measure the physiological parameter, and obtain a detection result of the physiological parameter.

In a possible implementation, obtaining the first detection result specifically includes: obtaining first measurement data; and determining the first detection result based on the first parameter, the first threshold, and the first measurement data.

In this way, the electronic device may calibrate the obtained measurement data, and an output detection result is calibrated measurement data.

In a possible implementation, the method further includes: displaying a first prompt when the inflation time of the micro air pump is greater than or equal to the sixth duration, where the first prompt is used to prompt the user to adjust the wearing tightness.

In this way, in the case of excessively loose wearing, the electronic device may output a wearing tightness prompt, to prompt the user to adjust the wearing tightness.

In a possible implementation, the first threshold further includes seventh duration, and the seventh duration is less than the sixth duration. The method further includes: displaying a second prompt when the inflation time of the micro air pump is greater than or equal to the seventh duration and less than the sixth duration, where the second prompt is used to prompt the user that the first detection result is inaccurate.

In this way, in the case of slightly loose wearing, the electronic device may further output an error prompt, to prompt the user that the measurement result of the physiological parameter may be inaccurate.

In a possible implementation, the method further includes: setting a first flow value; controlling a flow monitoring sensor to obtain a first air flow, where the first air flow is a sum of air flows detected by the flow monitoring sensor when the first condition is satisfied; and displaying a first prompt when the first air flow is greater than or equal to the first flow value or the inflation time of the micro air pump is greater than or equal to the sixth duration, where the first prompt is used to prompt the user to adjust the wearing tightness.

In this way, the wearing tightness of the user may be determined with reference to monitoring data of the flow monitoring sensor, to improve measurement accuracy. If it is determined, based on either the determining result of the flow monitoring sensor for the wearing tightness of the user or the determining result of the micro air pump for the wearing tightness of the user, that the wearing of the user is excessively loose, the electronic device determines that the wearing of the user is excessively loose.

In a possible implementation, the method further includes: when the first air flow is less than the first flow value, and the inflation time of the micro air pump is less than the sixth duration, continuing to control the micro air pump to inflate the airbag, to obtain a first detection result; and displaying the first detection result.

In this way, when neither of the determining results of the flow monitoring sensor and the micro air pump for the wearing tightness of the user is excessively loose wearing, the electronic device may continue to measure the physiological parameter, to obtain a detection result of the physiological parameter.

In a possible implementation, the method further includes: setting a second difference; controlling the PPG module to monitor a third pulse wave signal when the micro air pump inflates the airbag; determining a third difference between a maximum value and a minimum value of an amplitude of the third pulse wave signal; and displaying a first prompt when the second difference is greater than or equal to the third difference or the inflation time of the micro air pump is greater than or equal to the sixth duration, where the first prompt is used to prompt the user to adjust the wearing tightness.

In this way, the wearing tightness of the user may be determined with reference to monitoring data of the PPG module, to improve measurement accuracy. If it is determined, based on either the determining result of the PPG module for the wearing tightness of the user or the determining result of the micro air pump for the wearing tightness of the user, that the wearing of the user is excessively loose, the electronic device determines that the wearing of the user is excessively loose.

In a possible implementation, the method further includes: when the second difference is less than the third difference, and the inflation time of the micro air pump is less than the sixth duration, continuing to control the micro air pump to inflate the airbag, to obtain a first detection result; and displaying the first detection result.

In this way, when neither of the determining results of the PPG and the micro air pump for the wearing tightness of the user is excessively loose wearing, the electronic device may continue to measure the physiological parameter, to obtain a detection result of the physiological parameter.

In a possible implementation, the method further includes: setting a second difference; controlling the PPG module to monitor a third pulse wave signal when the micro air pump inflates the airbag; determining a third difference between a maximum value and a minimum value of an amplitude of the third pulse wave signal; and displaying a first prompt when the second difference is greater than or equal to the third difference or the air pressure of the airbag is less than or equal to the fifth air pressure value, where the first prompt is used to prompt the user to adjust the wearing tightness.

In this way, if it is determined, based on either the determining result of the PPG module for the wearing tightness of the user or a determining result of an air pressure sensor (configured to monitor the air pressure of the airbag) for the wearing tightness of the user, that the wearing of the user is excessively loose, the electronic device determines that the wearing of the user is excessively loose.

In a possible implementation, the method further includes: when the second difference is less than the third difference, and the air pressure of the airbag is greater than the fifth air pressure value, continuing to control the micro air pump to inflate the airbag, to obtain a first detection result; and displaying the first detection result.

In this way, when neither of the determining results of the PPG and the micro air pump for the wearing tightness of the user is excessively loose wearing, the electronic device may continue to measure the physiological parameter, to obtain a detection result of the physiological parameter.

In a possible implementation, the method further includes: controlling the Hall effect sensor to obtain magnetic field data, and determining a size of the airbag based on the magnetic field data; and determining the first condition and the first threshold based on the performance level of the micro air pump specifically includes: determining the first condition and the first threshold based on the performance level of the micro air pump and the size of the airbag.

In this way, when airbags of different sizes may be configured for the electronic device, the electronic device may determine the size of the airbag through the Hall effect sensor.

According to a third aspect, this application provides an electronic device, including one or more processors, one or more memories, a micro air pump, and an airbag. The one or more memories are coupled to the one or more processors, the one or more memories are configured to store computer instructions, and when the one or more processors execute the computer instructions, the measurement method in any possible implementation of either of the foregoing aspects is implemented.

According to a fourth aspect, this application provides a chip system, including a processing circuit and an interface circuit. The interface circuit is configured to receive code instructions and transmit the code instructions to the processing circuit. The processing circuit is configured to run the code instructions to perform the measurement method in any possible implementation of either of the foregoing aspects.

According to a fifth aspect, this application provides a computer storage medium, including computer instructions. When the computer instructions are executed by a processor, the measurement method in any one of the possible implementations of either of the foregoing aspects is implemented.

According to a sixth aspect, this application provides a computer program product. When the computer program product is executed by a processor, the measurement method in any possible implementation of either of the foregoing aspects is implemented.

For beneficial effect of the third aspect to the sixth aspect, refer to the beneficial effect of the first aspect and the second aspect.

### BRIEF DESCRIPTION OF DRAWINGS

FIG. 1A is a diagram of a device form of an electronic device 100 according to an embodiment of this application;
FIG. 1B is a diagram of a hardware structure of an electronic device 100 according to an embodiment of this application;
FIG. 1C is a diagram of a connection relationship of hardware related to a wearing tightness measurement according to an embodiment of this application;
FIG. 1D is a diagram of results of detecting airbags of different sizes by a Hall effect sensor according to an embodiment of this application;
FIG. 2A to FIG. 2H are diagrams of a group of interfaces of a measurement method in a blood pressure measurement scenario according to an embodiment of this application;
FIG. 3 is a schematic flowchart of measuring performance of a micro air pump by an electronic device 100 according to an embodiment of this application;
FIG. 4A to FIG. 4C are a schematic flowchart of a measurement method in a blood pressure measurement scenario according to an embodiment of this application;
FIG. 5 is a diagram in which air pressure of an airbag and a pulse wave signal change with time in a process in which an electronic device 100 inflates the airbag through a micro air pump according to an embodiment of this application;
FIG. 6A and FIG. 6B are diagrams of a correspondence between a performance level of a micro air pump and a wearing tightness threshold when an operating parameter is determined according to an embodiment of this application;
FIG. 6C is a diagram of a correspondence between a performance level of a micro air pump and a wearing tightness threshold when the wearing tightness threshold is determined according to an embodiment of this application;
FIG. 6D is a diagram of a correspondence between a performance level of a micro air pump and an operating parameter of the micro air pump when a wearing tightness threshold is determined according to an embodiment of this application;
FIG. 7 is a diagram of photoplethysmography signals in a process of inflating an airbag by a micro air pump at different wearing tightness according to an embodiment of this application;
FIG. 8 is a diagram of a proportion integral differential (proportion integral differential, PID) control principle according to an embodiment of this application;
FIG. 9A and FIG. 9B are diagrams of connection relationships between two flow monitoring sensors and another component related to a wearing tightness measurement in a watch body according to an embodiment of this application;
FIG. 10 is a diagram of functional modules of an electronic device 100 according to an embodiment of this application;
FIG. 11 is a schematic flowchart of a measurement method according to an embodiment of this application;
FIG. 12 is a diagram of a connection relationship of hardware related to a wearing tightness measurement according to an embodiment of this application;
FIG. 13A is a schematic flowchart of a measurement method according to an embodiment of this application;
FIG. 13B is a diagram of a correspondence between an inflation capability of a micro air pump and a pressure rise duty cycle according to an embodiment of this application;
FIG. 13C is a diagram of a relationship between different measurement postures and correction coefficients according to an embodiment of this application;
FIG. 14 is a schematic flowchart of determining, by an electronic device 100, current wearing tightness based on pre-inflation data and a wearing tightness threshold according to an embodiment of this application;
FIG. 15A and FIG. 15B are another schematic flowchart of determining, by an electronic device 100, current wearing tightness based on pre-inflation data and a wearing tightness threshold according to an embodiment of this application;
FIG. 16 is another schematic flowchart of determining, by an electronic device 100, current wearing tightness based on pre-inflation data and a wearing tightness threshold according to an embodiment of this application;
FIG. 17A and FIG. 17B are a schematic flowchart of measuring a physiological parameter 1 (for example, blood pressure) by an electronic device 100 according to an embodiment of this application;
FIG. 18A and FIG. 18B are a schematic flowchart of measuring a physiological parameter 1 and wearing tightness of a user by an electronic device 100 according to an embodiment of this application;
FIG. 19A and FIG. 19B are another schematic flowchart of measuring a physiological parameter 1 by an electronic device 100 according to an embodiment of this application;
FIG. 20A and FIG. 20B are a schematic flowchart of a blood pressure measurement method according to an embodiment of this application;
FIG. 21 is a schematic flowchart of a method for measuring not-worn duration according to an embodiment of this application;
FIG. 22A to FIG. 22F are diagrams of a group of interfaces for measuring blood pressure by an electronic device 100 according to an embodiment of this application;
FIG. 23 is a diagram of functional modules of an electronic device 100 according to an embodiment of this application; and
FIG. 24 is a schematic flowchart of another measurement method according to an embodiment of this application.

### DESCRIPTION OF EMBODIMENTS

The following clearly describes technical solutions in embodiments of this application in detail with reference to the accompanying drawings. In descriptions of embodiments of this application, unless otherwise specified, "/" indicates "or". For example, A/B may indicate A or B. The term "and/or" in this specification merely describes an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more.

In the following descriptions, terms such as "first" and "second" are merely used for description, and shall not be understood as an indication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language like Java or an extensible markup language (extensible markup language, XML). Interface source code is parsed and rendered on an electronic device, and is finally presented as content that can be identified by the user. The user interface is usually in a representation form of a graphical user interface (graphical user interface, GUI), which refers to a user interface related to a computer operation and displayed in a graphical manner. The user interface may be a visual interface element like a text, an icon, a button, a menu, a tab, a text box, a dialog box, a status bar, a navigation bar, or a widget that is displayed on a display of the electronic device.

The following describes a device form of a wearable device provided in embodiments of this application.

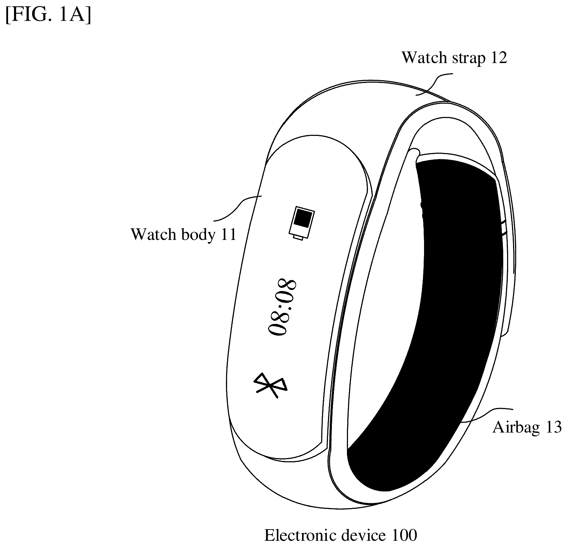

FIG. 1A is a diagram of a device form of an electronic device 100 according to an embodiment of this application.

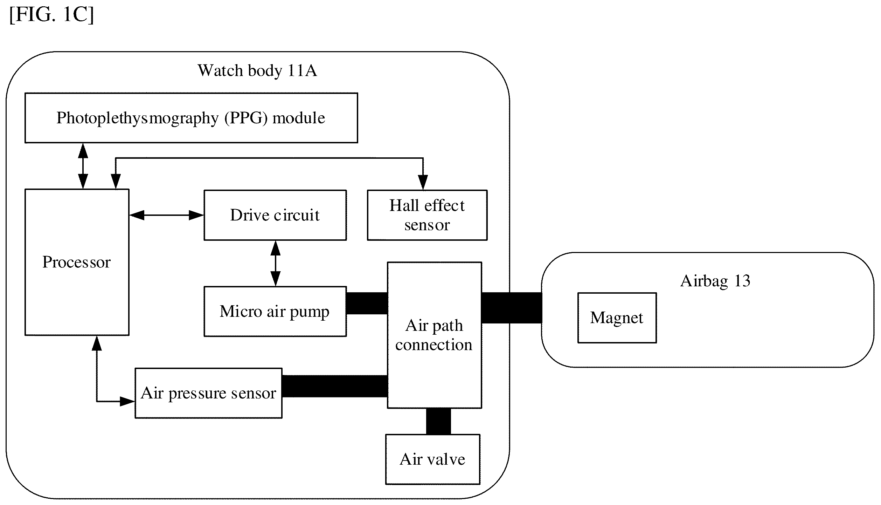

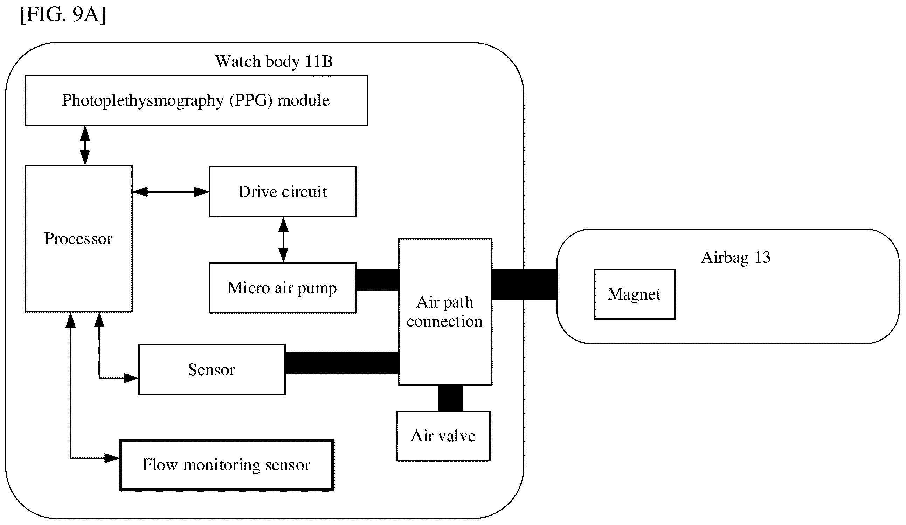

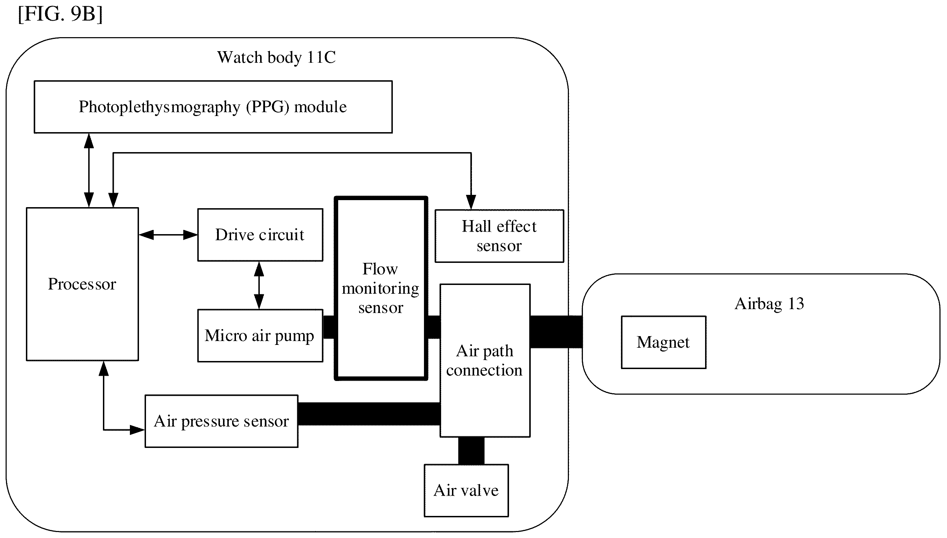

As shown in FIG. 1A, the electronic device 100 may include a watch body 11, a watch strap 12, and an airbag 13. The watch body 11 may include a display and a button, and the button may include one or more of a mechanical button, a touchscreen button, a watch crown, a joystick, and the like. For components inside the watch body 11, refer to related descriptions in the following embodiment shown in FIG. 1C, FIG. 9A, or FIG. 9B. Details are not described herein. The watch strap 12 may be connected to the watch body 11, and the airbag 13 may be disposed on an inner side of the watch strap 12. The inner side is a side that is in contact with a wrist of a user when the user wears the electronic device 100.

The electronic device 100 may be a band shown in FIG. 1A. It may be understood that in embodiments of this application, the electronic device 100 may alternatively be another wearable device like a watch. This is not limited in this application.

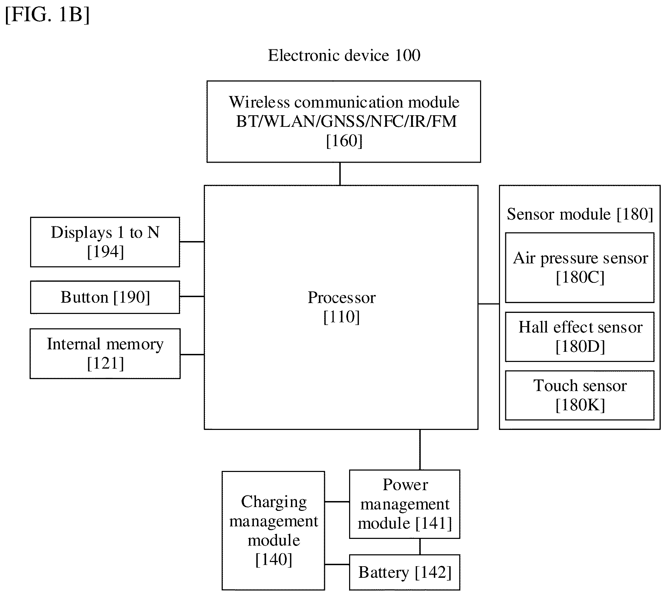

FIG. 1B is a diagram of a hardware structure of the electronic device 100 according to an embodiment of this application.

The electronic device 100 may be a wearable device, for example, a watch or a band.

The electronic device 100 may include a processor 110, an internal memory 121, a charging management module 140, a power management module 141, a battery 142, a wireless communication module 160, a sensor module 180, a button 190, a display 194, and the like. The sensor module 180 may include an air pressure sensor 180C and a touch sensor 180K. In some embodiments, the sensor module 180 may further include one or more of sensors such as a flow monitoring sensor, a Hall effect sensor 180D, a photoelectric sensor, a capacitive sensor, a gyroscope sensor, and an acceleration sensor. In some embodiments, the electronic device 100 may further include one or more of components such as an audio module, a motor, and an indicator.

It may be understood that the structure shown in this embodiment of this application does not constitute a specific limitation on the electronic device 100. In some other embodiments of this application, the electronic device 100 may include more or fewer components than those shown in the figure, or some components may be combined, or some components may be split, or there may be a different component arrangement. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent devices, or may be integrated into one or more processors. The controller may generate an operation control signal based on an instruction operation code and a time sequence signal, to complete control of instruction fetching and instruction execution. A memory may be further disposed in the processor 110, and is configured to store instructions and data. In some embodiments, the memory in the processor 110 is a cache memory. The memory may store instructions or data just used or cyclically used by the processor 110. If the processor 110 needs to use the instructions or the data again, the processor may directly invoke the instructions or the data from the memory. This avoids repeated access, reduces waiting time of the processor 110, and improves system efficiency.

The charging management module 140 is configured to receive a charging input from a charger. The charger may be a wireless charger or a wired charger. In some wired charging embodiments, the charging management module 140 may receive a charging input from the wired charger through a USB interface. In some wireless charging embodiments, the charging management module 140 may receive a wireless charging input through a wireless charging coil of the electronic device 100. The charging management module 140 supplies power to the electronic device through the power management module 141 while charging the battery 142.

The power management module 141 is configured to connect the battery 142, the charging management module 140, and the processor 110. The power management module 141 receives an input from the battery 142 and/or the charging management module 140, and supplies power to the processor 110, the internal memory 121, the display 194, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

The wireless communication module 160 may provide a wireless communication solution that is applied to the electronic device 100 and that includes any one or more of a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more devices integrating at least one communication processing module.

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs that execute program instructions to generate or change display information.

The display 194 is configured to display an image, a video, and the like. The display 194 includes a display panel. The display panel may be a liquid crystal display (liquid crystal display, LCD). The display panel may alternatively be an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini LED, a micro LED, a micro OLED, quantum dot light emitting diodes (quantum dot light emitting diodes, QLED), or the like. In some embodiments, the electronic device 100 may include one or more displays 194.

The internal memory 121 may include one or more random access memories (random access memories, RAMs) and one or more non-volatile memories (non-volatile memories, NVMs), may be directly read and written by the processor 110. The random access memory may be configured to store an executable program (for example, machine instructions) in an operating system or another running program, and may be configured to store data of a user, data of an application, and the like. The non-volatile memory may also store the executable program, the data of the user, the data of the application, and the like, which may be preloaded to the random access memory for the processor 110 to directly perform a read or write operation.

The audio module may be configured to convert digital audio information into an analog audio signal for output, and is also configured to convert an analog audio input into a digital audio signal. The audio module may be further configured to: encode and decode an audio signal. In some embodiments, the audio module may be disposed in the processor 110, or some functional modules in the audio module are disposed in the processor 110.

The air pressure sensor 180C (for example, a differential pressure sensor) may be configured to measure air pressure. In some embodiments, the electronic device 100 measures air pressure of the airbag through the air pressure sensor 180C. The electronic device 100 may also measure blood pressure of the user through the air pressure sensor 180C.

The touch sensor 180K is also referred to as a "touch device". The touch sensor 180K may be disposed on the display 194, and the touch sensor 180K and the display 194 constitute a touchscreen, which is also referred to as a "touch screen". The touch sensor 180K is configured to detect a touch operation performed on or near the touch sensor. The touch sensor may transfer the detected touch operation to the application processor to determine a type of a touch event. A visual output related to the touch operation may be provided through the display 194. In some other embodiments, the touch sensor 180K may alternatively be disposed on a surface of the electronic device 100 at a location different from that of the display 194.

The Hall effect sensor 180D may be configured to detect a magnetic field. In this embodiment of this application, the Hall effect sensor 180D may be configured to detect a size of the airbag.

In some embodiments, the sensor module 180 may further include one or more sensors of a gyroscope sensor and an acceleration sensor. These sensors may be configured to detect a gesture operation (for example, a wrist raising operation) of wearing the electronic device 100 by the user. The gyroscope sensor may be configured to determine a moving posture of the electronic device 100. In some embodiments, an angular velocity of the electronic device 100 around three axes (namely, axes x, y, and z) may be determined by using the gyroscope sensor. The acceleration sensor may detect magnitudes of accelerations of the electronic device 100 in various directions (generally on three axes). When the electronic device 100 is still, a magnitude and a direction of gravity may be detected. The acceleration sensor may be further configured to identify a posture of the electronic device. The magnetic sensor includes a Hall effect sensor, and may be configured to measure a magnetic field of the electronic device 100.

In some embodiments, the sensor module 180 may further include a photoelectric sensor and/or a flow monitoring sensor. The photoelectric sensor may be configured to transmit an optical signal (for example, an infrared signal), and receive an optical signal reflected by the user. The flow monitoring sensor may be configured to detect a volume of an air flow in the watch body, or configured to detect a volume of an air flow inflated by the micro air pump into the airbag.

The button 190 includes a power button, a volume button, and the like. The button 190 may be a mechanical button or a touch button. The electronic device 100 may receive a button input, and generate a button signal input related to a user setting and function control of the electronic device 100.

The following describes a connection relationship of hardware related to a wearing tightness measurement in the electronic device 100 provided in embodiments of this application.

FIG. 1C is a diagram of a connection relationship of hardware related to a wearing tightness measurement according to an embodiment of this application.

In this embodiment of this application, the watch body 11 shown in FIG. 1A may be a watch body 11A shown in FIG. 1C. As shown in FIG. 1C, the watch body 11A may include a processor, a drive circuit, a micro air pump, an air pressure sensor (for example, a differential pressure sensor), an air path connection, and the like. In some embodiments, the watch body 11A may further include any one or more of a photoplethysmography (photoplethysmography, PPG) module, a Hall effect sensor, and an air valve. The airbag 13 may be connected to the micro air pump in the watch body 11 through the air path connection. Optionally, a magnet may be disposed in the airbag 13, and the magnet may be disposed on a side that is of the airbag 13 and that is close to the watch body 11.

The processor may be the processor 110 shown in FIG. 1B. The processor may control the drive circuit to supply power to the micro air pump, so that the micro air pump inflates the airbag 13 based on a preset operating parameter through the air path connection. The processor may control turning-on and turning-off of the air valve, and may further control turning-on and turning-off of the air valve and a flow rate of air release based on a current air pressure requirement for the airbag 13. The processor may further control a sensor (for example, the air pressure sensor) to detect air pressure of the airbag 13. The processor may further control the PPG module to collect a PPG signal, and obtain a blood pressure measurement value of a user through analysis based on the PPG signal.

The drive circuit may supply power to the micro air pump under control of the processor, so that the micro air pump can operate with a preset operating parameter.

When the micro air pump operates, the micro air pump may inflate the airbag 13 through the air path connection.

The air pressure sensor may monitor the air pressure of the airbag. In some embodiments, the air pressure sensor may also collect a pulse wave signal of the user.

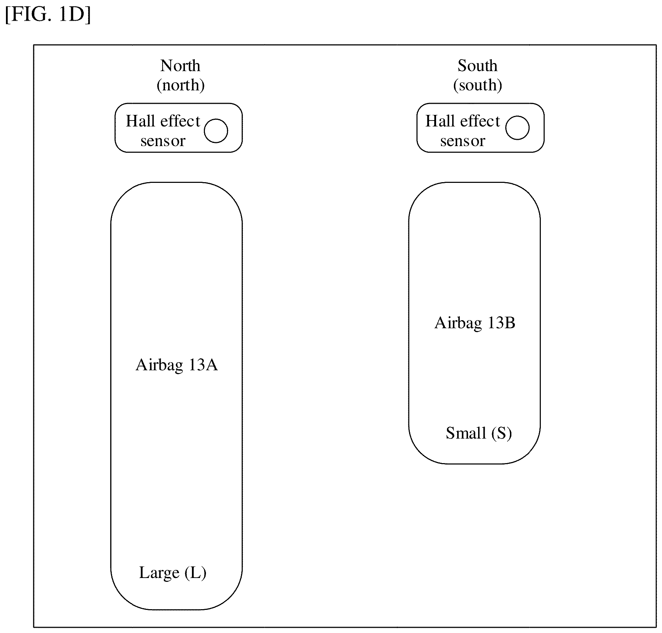

In some embodiments, the Hall effect sensor may be further disposed in the watch body 11, the magnet may be disposed in the airbag 13, and the Hall effect sensor may be configured to detect a magnetic field of the magnet in the airbag 13. For results of detecting magnets in airbags of different sizes by the Hall effect sensor, refer to related content in the following embodiment shown in FIG. 1D.

In some other embodiments, the watch body 11 may further include a flow monitoring sensor. The flow monitoring sensor may be configured to detect an air flow. For a connection relationship between the flow monitoring sensor and another component in the watch body 11A, refer to related descriptions in the following embodiments shown in FIG. 9A and FIG. 9B. Details are not described herein.

The air valve may include two states: on and off, and may further regulate a flow rate of air release of the airbag 13. In some embodiments, a micro air pump integrated an air release valve may alternatively be used in the watch body 11 to replace the air valve, and is configured to regulate the flow rate of air release of the airbag 13.

The PPG module may transmit an infrared signal (or another optical signal), and may further receive an infrared signal that is reflected back after being absorbed by a blood vessel of the user. The received signal may also be referred to as a PPG signal.

It may be understood that, the embodiment shown in FIG. 1C merely illustrates a part of function composition hardware related to a blood pressure measurement. For other hardware, refer to the related content in the embodiment shown in FIG. 1B.

In some embodiments, the magnet may be disposed in the airbag 13, and the electronic device 100 may include the Hall effect sensor. The electronic device 100 may detect the magnet in the airbag 13 through the Hall effect sensor, to determine a size of the airbag.

FIG. 1D is a diagram of results of detecting airbags of different sizes by the Hall effect sensor according to an embodiment of this application.

As shown in FIG. 1D, a size of an airbag 13A is large (large, L), a size of an airbag 13B is small (small, S), and the size of the airbag 13A is greater than that of the airbag 13B. When the size of the airbag is L, a detection result of the Hall effect sensor is a north (north) pole. When the size of the airbag is S, a detection result of the Hall effect sensor is a south (south) pole.

Therefore, in some embodiments of this application, the electronic device 100 may determine the size of the airbag based on a detection result of the Hall effect sensor.

When the user wears the electronic device 100, if the electronic device 100 is used to measure a physiological parameter (for example, blood pressure, a heart rate, or a body temperature) of the user, wearing tightness of the user affects a measurement result of the physiological parameter.

This application provides a measurement method, applied to the electronic device 100 including the micro air pump and the airbag. The electronic device 100 may detect a performance level of the micro air pump, and determine a first condition and a first threshold based on the performance level of the micro air pump. The electronic device 100 controls the micro air pump to inflate the airbag, and determines a first parameter when detecting that the first condition is satisfied. The first parameter is compared with the first threshold, and the wearing tightness is determined based on a comparison result.

In this way, the wearing tightness of the user can be determined in a physiological parameter measurement, to avoid an error caused by the wearing tightness in the physiological parameter measurement process. In addition, performance of the micro air pump is also considered in the wearing tightness measurement of the user, so that a measurement error caused by performance deterioration of the micro air pump can be avoided.

The measurement method provided in this application may be applied to a physiological parameter measurement scenario.

The following describes, with reference to specific scenarios, the measurement method provided in embodiments of this application.

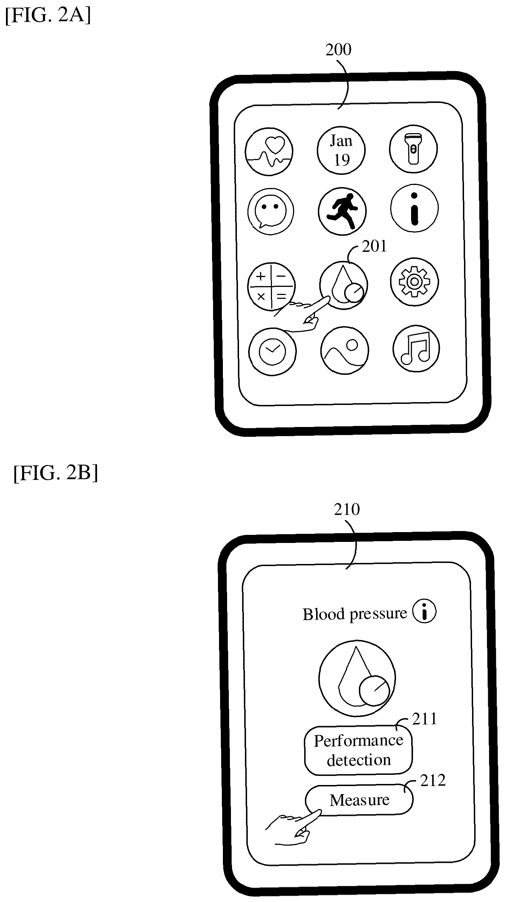

FIG. 2A to FIG. 2H are diagrams of a group of interfaces of the measurement method in a blood pressure measurement scenario according to an embodiment of this application.

For example, as shown in FIG. 2A, the electronic device 100 may display a home screen 200, and the home screen 200 may include one or more application icons, for example, a blood pressure application icon 201.

The electronic device 100 may receive and respond to a tap operation performed by the user on the blood pressure application icon 201, and display a blood pressure measurement interface 210 shown in FIG. 2B.

As shown in FIG. 2B, the blood pressure measurement interface 210 may include a performance detection control 211 and a measurement control 212. The performance detection control 211 may be used to trigger the electronic device 100 to detect performance of the micro air pump. The measurement control 212 may be used to trigger the electronic device 100 to measure current blood pressure of the user.

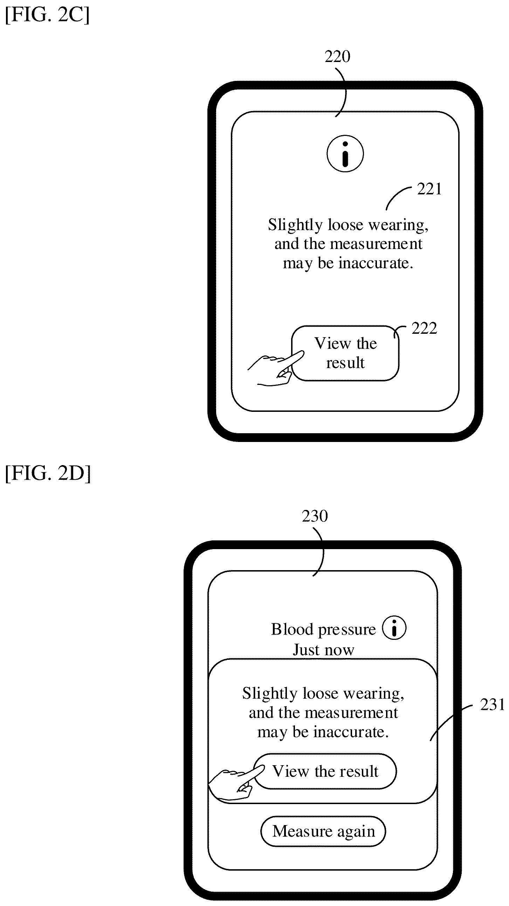

The electronic device 100 may receive and respond to a tap operation performed by the user on the measurement control 212. After detecting that the user wears the electronic device 100 slightly loosely and a blood pressure measurement is completed, the electronic device 100 may display an error prompt interface 220 shown in FIG. 2C.

As shown in FIG. 2C, the error prompt interface 220 may include an error prompt 221 and a result view control 222. The error prompt 221 may be used to prompt the user that a current measurement result may be inaccurate. For example, the error prompt 221 may include a text like "Slightly loose wearing, and the measurement may be inaccurate". The result view control 222 may be used to trigger the electronic device 100 to display a blood pressure measurement result.

In some embodiments, after it is detected that the user wears the electronic device 100 slightly loosely, and the blood pressure measurement is completed, as shown in FIG. 2D, the electronic device 100 may display a measurement result interface 230, and output a prompt window 231 on the measurement result interface 230. The measurement result interface 230 may be used to display the blood pressure measurement result. The prompt window 231 may include an error prompt and a result view control. For specific content of the result view control and the error prompt, refer to the related descriptions of the error prompt interface 220 shown in FIG. 2C.

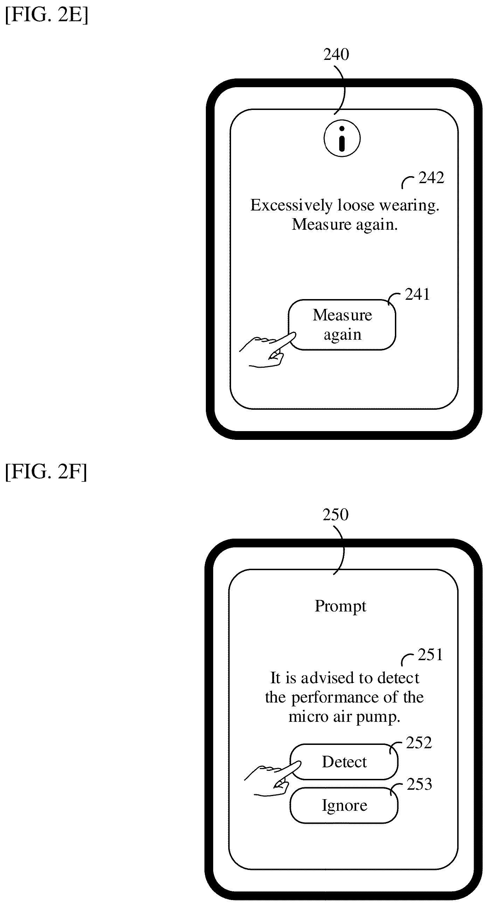

The electronic device 100 may receive and respond to a tap operation performed by the user on the measurement control 212, and when detecting that the wearing of the user is excessively loose, display a tightness prompt interface 240 shown in FIG. 2E.

As shown in FIG. 2E, the tightness prompt interface 240 may include a measurement control 241 and a wearing tightness prompt 242. The measurement control 241 is used to trigger the electronic device 100 to measure blood pressure of the user again. The wearing tightness prompt 242 may be used to prompt the user that the electronic device 100 is worn excessively loosely currently, and the blood pressure cannot be normally measured. For example, the wearing tightness prompt 242 may include a text like "Excessively loose wearing. Measure again".

In some embodiments, the electronic device 100 may also detect performance of the micro air pump. **In** this way, the electronic device 100 may set related data such as a wearing tightness threshold and an operating parameter of the micro air pump based on the performance of the micro air pump, to prevent performance deterioration caused by long-time use of the micro air pump from affecting the blood pressure measurement result.

For example, the electronic device 100 may receive and respond to a tap operation performed by the user on the measurement control 212 shown in FIG. 2B, and when determining that a performance detection condition of the micro air pump is satisfied (for example, a quantity of days since previous performance detection of the micro air pump reaches a preset quantity of days), the electronic device 100 may display a prompt interface 250 shown in FIG. 2F.

As shown in FIG. 2F, the prompt interface 250 may include a performance detection prompt 251, for example, a text "It is advised to re-detect the performance of the micro air pump". The performance detection prompt 251 is used to prompt the user to detect the performance of the micro air pump. The prompt interface 250 may further include a detection control 252 and an ignore control 253. The detection control 252 may be used to trigger the electronic device 100 to detect the performance of the micro air pump. The ignore control 253 may be used to trigger the electronic device 100 to perform wearing tightness detection.

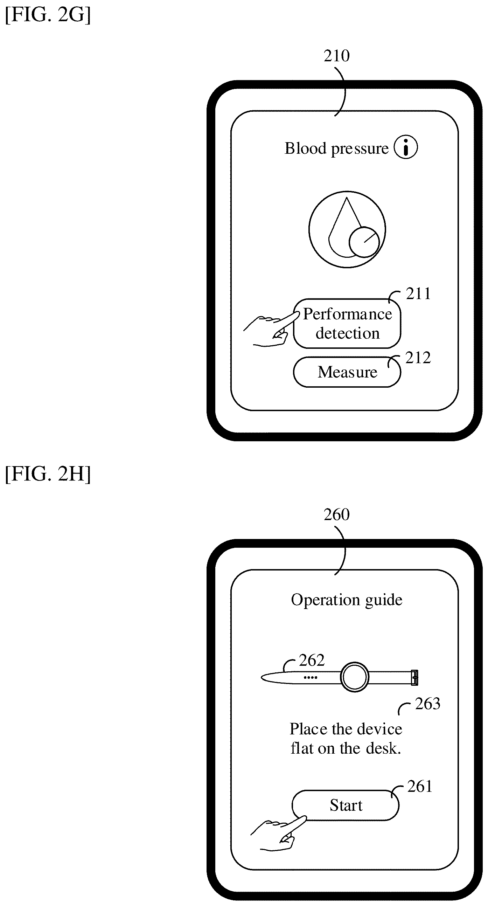

The electronic device 100 may receive and respond to a tap operation performed by the user on the detection control 252, and display an operation guide interface 260 shown in FIG. 2H.

For another example, as shown in FIG. 2G, the electronic device 100 may also display the blood pressure measurement interface 210, and the blood pressure measurement interface 210 may include the performance detection control 211 and the measurement control 212. The electronic device 100 may receive and respond to a tap operation performed by the user on the performance detection control 211, and display the operation guide interface 260 shown in FIG. 2H.

As shown in FIG. 2H, an operation guide may be displayed on the operation guide interface 260, including a picture guide 262 and a text guide 263, for example, a text "Place the device flat on the desk". The operation guide interface 260 may further include a start control 261. The electronic device 100 may receive and respond to a tap operation performed by the user on the start control 261, and detect the performance of the micro air pump.

It may be understood that FIG. 2A to FIG. 2H merely describe an example of application of the measurement method provided in embodiments of this application in the blood pressure measurement scenario. In some embodiments, the control used to trigger performance detection of the micro air pump may alternatively be displayed on an interface for measuring another physiological parameter (for example, the heart rate or the body temperature). This is not limited in this application.

The following describes a manner of detecting the performance of the micro pump provided in an embodiment of this application.

In this embodiment of this application, the performance of the micro air pump may be represented by a performance level.

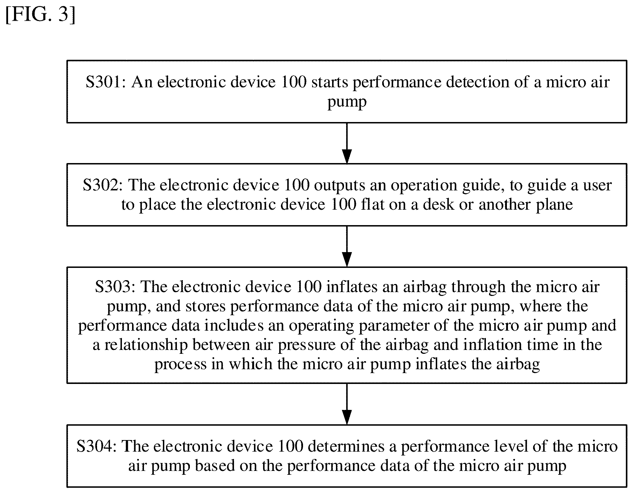

As shown in FIG. 3, a specific procedure of the manner of detecting the performance of the micro air pump may include the following steps.

S301: The electronic device 100 starts performance detection of the micro air pump.

In some embodiments, the electronic device 100 may start performance detection of the micro air pump when detecting that the performance detection condition of the micro air pump is satisfied and detecting that the electronic device 100 is not worn by the user. The performance detection condition of the micro air pump may include: Time from the previous performance detection of the micro air pump reaches the preset quantity of days, a deviation between one or more latest detected blood pressure measurement values and a historical blood pressure measurement value is greater than a deviation threshold, and the like.

In some other embodiments, the electronic device 100 may alternatively output a performance detection prompt for the micro air pump when detecting that the performance detection condition of the micro air pump is satisfied, where the performance detection prompt for the micro air pump is used to prompt the user to perform performance detection of the micro air pump. For example, the performance detection prompt for the micro air pump may be the performance detection prompt 251 on the prompt interface 250 shown in FIG. 2F.

In some embodiments, the electronic device 100 may receive and respond to an operation of detecting the performance of the micro air pump by the user, and start performance detection of the micro air pump. For example, the operation of detecting the performance of the micro air pump by the user may be the tap operation performed by the user on the performance detection control 211 on the blood pressure measurement interface 210 shown in FIG. 2G.

In some embodiments, when the performance detection of the micro air pump is started, the electronic device 100 may first determine whether the electronic device 100 is worn by the user.

The following describes two manners of determining whether the electronic device 100 is worn by the user provided in this embodiment of this application.

In some embodiments, the electronic device 100 may determine, based on whether the PPG module can detect a regular pulse wave signal, whether the user wears the electronic device 100.

In some other embodiments, a capacitive sensor may also be disposed on an inner side of the watch body 11 of the electronic device 100. When the user wears the electronic device 100, the capacitive sensor is in contact with skin of the user, causing a capacitance change. The electronic device 100 may alternatively determine, by detecting a capacitance change through the capacitive sensor, whether the user wears the electronic device 100.

It should be noted that, in this embodiment of this application, a pulse wave signal detected by the electronic device 100 based on the PPG module may also be referred to as a second pulse wave signal, and a capacitance signal detected based on the capacitive sensor may also be referred to as a second capacitance signal. When the performance detection of the micro air pump is started, the electronic device 100 may obtain a second signal, and determine, based on the second signal, whether the user wears the electronic device 100. The second signal may include the second pulse wave signal and/or the second capacitance signal.

S302: The electronic device 100 outputs an operation guide, to guide the user to place the electronic device 100 flat on a desk or another plane.

In the process of detecting the performance of the micro air pump, a strap needs to be unfastened, and the electronic device 100 needs to be placed flat on the desk or the another plane with an airbag side upward (or facing a side surface). After the performance detection of the micro air pump is started, the electronic device 100 may output the operation guide to guide the user to place the electronic device 100. For example, the operation guide may be the picture guide 262 and the text guide 263 in the embodiment shown in FIG. 2H.

In some embodiments, the electronic device 100 starts timing when the operation guide is output, and when the timing reaches preset time (for example, 5 seconds), the electronic device 100 may perform the following step S303.

In some other embodiments, when outputting the operation guide, the electronic device 100 may further display a measurement control. The measurement control may be used to trigger the electronic device 100 to measure the performance of the micro air pump. The electronic device 100 may receive and respond to an input of the user for the measurement control, and perform the following step S303.

S303: The electronic device 100 inflates the airbag through the micro air pump, and stores performance data of the micro air pump, where the performance data includes the operating parameter of the micro air pump and a relationship between the air pressure of the airbag and inflation time in the process in which the micro air pump inflates the airbag.

In some embodiments, the electronic device 100 may perform step S303 once. The electronic device 100 may record the air pressure of the airbag and consumed time in real time in the process of inflating the airbag, and store the performance data of the micro air pump. The performance data of the micro air pump may include the operating parameter of the micro air pump, initial air pressure, end air pressure, and time consumed for the air pressure of the airbag to change from the initial air pressure to the end air pressure.

In some embodiments, the electronic device 100 may set the operating parameter (also referred to as a first operating parameter) of the micro air pump, the initial air pressure (also referred to as a first air pressure value) of the airbag, and the end air pressure (also referred to as a second air pressure value) of the airbag. Then, the electronic device 100 may control the micro air pump to inflate the airbag based on the first operating parameter, to obtain time (also referred to as first duration) consumed for the air pressure of the airbag to change from the initial air pressure to the end air pressure.

In some other embodiments, the electronic device 100 may set the operating parameter (also referred to as a first operating parameter) of the micro air pump, the initial air pressure of the airbag, and inflation duration (also referred to as second duration) of the micro air pump. Then, the electronic device 100 may control the micro air pump to inflate the airbag based on the first operating parameter, to obtain an air pressure value (also referred to as a third air pressure value) of the airbag when the inflation duration reaches the second duration.

For example, Table 1 shows performance data that is of the micro air pump and that is stored in the electronic device 100.

**Table 1**

| Operating parameter | Initial air pressure (millimeters of mercury/mmHg) | End air pressure (millimeters of mercury/mmHg) | Consumed time (second/s) |
|---|---|---|---|
| An operating voltage is 15 V, and a duty cycle is 50% | 0 | 50 | 10 |
| An operating voltage is 15 V, and a duty cycle is 50% | 0 | 80 | 15 |
| An operating voltage is 15 V, and a duty cycle is 50% | 0 | 100 | 17 |

As shown in Table 1, the performance data that is of the micro air pump and that is stored in the electronic device 100 may include the operating parameter of the micro air pump, the initial air pressure (unit: millimeters of mercury/mmHg), the end air pressure (unit: millimeters of mercury/mmHg), and the consumed time (unit: second/s). It can be learned from Table 1 that, when the operating parameter is the operating voltage 15 V and the duty cycle 50%, the time consumed for the air pressure of the airbag to rise from the initial air pressure 0 to the end air pressure 50 is 10 seconds; when the operating parameter is the operating voltage 15 V and the duty cycle 50%, the time consumed for the air pressure of the airbag to rise from the initial air pressure 0 to the end air pressure 80 is 15 seconds; and when the operating parameter is the operating voltage 15 V and the duty cycle 50%, the time consumed for the air pressure of the airbag to rise from the initial air pressure 0 to the end air pressure 100 is 17 seconds.

It may be understood that Table 1 is merely an example for describing the performance data that is of the micro air pump and that is stored in the electronic device 100. In this embodiment of this application, the performance data of the micro air pump may include more or less data, or may include data whose value is different from the foregoing data value. This is not limited in this application.

In some embodiments, step S303 is a step that can be repeatedly performed.

The electronic device 100 may measure, for a plurality of times, time consumed for the air pressure of the airbag to rise from same initial air pressure to same end air pressure when the micro air pump operates with a same operating parameter, and calculate an average value; or measure time (for example, the first duration and the third duration) consumed for the air pressure of the airbag to rise from same initial air pressure to same end air pressure when the micro air pump operates with different operating parameters (for example, the first operating parameter and the second operating parameter); or measure time consumed for the air pressure of the airbag to rise from same initial air pressure to different end air pressure when the micro air pump operates with a same operating parameter, and store a plurality of groups of performance data of the micro air pump that are obtained through measurement; or when the micro air pump operates with different operating parameters (for example, the first operating parameter and the second operating parameter), measure the air pressure (for example, the third air pressure value and the fourth air pressure value) of the airbag when the inflation duration of the micro air pump reaches the preset duration (for example, the second duration). In the foregoing plurality of cases, for a storage form of the plurality of groups of performance data that are of the micro air pump and that are stored in the electronic device 100, refer to the content shown in Table 1.

In this way, the performance level of the micro air pump can be determined based on results of a plurality of measurements, to reduce an error caused by a single measurement and improve measurement accuracy.

S304: The electronic device 100 determines the performance level of the micro air pump based on the performance data of the micro air pump.

The performance level of the micro air pump may include a plurality of levels. For example, performance levels in descending order may include Po1, Po2, and Po3. The electronic device 100 may store a correspondence between the performance data and the performance level of the micro air pump. The electronic device 100 may determine the performance level of the micro air pump from the correspondence between the performance data and the performance level of the micro air pump based on the performance data of the micro air pump measured in step S302.

In some embodiments, the correspondence that is between the performance data and the performance level and that is stored in the electronic device 100 may be a relationship between the time consumed for the air pressure of the airbag to rise from the initial air pressure to the end air pressure and the performance level when the micro air pump operates with different operating parameters. In this case, the electronic device 100 may determine the performance level of the micro air pump based on the operating parameter (for example, the first operating parameter or the second operating parameter) of the micro air pump, the initial air pressure (for example, the first air pressure value) and the end air pressure (for example, the second air pressure value) of the airbag, and the inflation duration (for example, the first duration or the third duration) corresponding to the change of the air pressure of the airbag from the initial air pressure to the end air pressure.

For example, Table 2 shows a correspondence that is between the performance data and the performance level of the micro air pump and that is stored in the electronic device 100.

**Table 2**

| Operating parameter | Initial air pressure | End air pressure | Consumed time | Performance level |
|---|---|---|---|---|
| An operating voltage is 15 V, and a duty cycle is 50% | CS0 | CS1 | [0, ct1) | Po1 |
| An operating voltage is 15 V, and a duty cycle is 50% | CS0 | CS1 | [ct1, ct2) | Po2 |
| An operating voltage is 15 V, and a duty cycle is 50% | CS0 | CS1 | [ct2, +∞) | Po3 |
| An operating voltage is 13 V, and a duty cycle is 30% | CS0 | CS2 | [0, ct3) | Po1 |
| An operating voltage is 13 V, and a duty cycle is 30% | CS0 | CS2 | [ct3, ct4) | Po2 |
| An operating voltage is 13 V, and a duty cycle is 30% | CS0 | CS2 | [ct4, +∞) | Po3 |

As shown in Table 2, when the operating parameter is the operating voltage 15 V and the duty cycle 50%, if the time consumed for the air pressure of the airbag to rise from the initial air pressure CS0 to CS1 is less than ct1, the performance level of the micro air pump is Po1; if the consumed time is greater than or equal to ct1 and less than ct2, the performance level of the micro air pump is Po2; and if the consumed time is greater than or equal to ct2, the performance level of the micro air pump is Po3. When the operating parameter is the operating voltage 13 V and the duty cycle 30%, if the time consumed for the air pressure of the airbag to rise from the initial air pressure CS0 to CS2 is less than ct3, the performance level of the micro air pump is Pol; if the consumed time is greater than or equal to ct3 and less than ct4, the performance level of the micro air pump is Po2; and if the consumed time is greater than or equal to ct4, the performance level of the micro air pump is Po3.

It may be understood that Table 2 is merely an example. In this embodiment of this application, the electronic device 100 may alternatively store more or less content than that in Table 2, or store content different from that in Table 2. This is not limited in this application.

In some other embodiments, the correspondence that is between the performance data and the performance level and that is stored in the electronic device 100 may be a correspondence between the air pressure of the airbag and the performance level when the micro air pump operates with different operating parameters and the inflation time of the airbag reaches preset consumed time. In this case, the electronic device 100 may determine the performance level of the micro air pump based on the operating parameter (for example, the first operating parameter or the second operating parameter) of the micro air pump, the initial air pressure (for example, the first air pressure value) of the airbag, the duration (for example, the second duration) of inflating the airbag by the micro air pump, and the air pressure (for example, the third air pressure value or the fourth air pressure value) of the airbag when the inflation duration is satisfied.

For example, Table 3 shows another correspondence that is between the performance data and the performance level of the micro air pump and that is stored in the electronic device 100.

**Table 3**

| Operating parameter | Initial air pressure | End air pressure | Consumed time | Performance level |
|---|---|---|---|---|
| An operating voltage is 15 V, and a duty cycle is 50% | CS0 | [CS0, CSS1) | CT1 | Po3 |
| An operating voltage is 15 V, and a duty cycle is 50% | CS0 | [CSS1, CSS2) | CT1 | Po2 |
| An operating voltage is 15 V, and a duty cycle is 50% | CS0 | [CSS2, +∞) | CT1 | Po1 |
| An operating voltage is 13 V, and a duty cycle is 30% | CS0 | [CS0, CSS3) | CT2 | Po3 |
| An operating voltage is 13 V, and a duty cycle is 30% | CS0 | [CS3, CSS3) | CT2 | Po2 |
| An operating voltage is 13 V, and a duty cycle is 30% | CS0 | [CSS3, +∞) | CT2 | Po1 |

As shown in Table 3, when the operating parameter is the operating voltage 15 V and the duty cycle 50%, and inflation time for the air pressure of the airbag starting from the initial air pressure CS0 reaches CT1, if the air pressure of the airbag is greater than or equal to CS0 and less than CSS1, the performance level of the micro air pump is Po3; if the air pressure of the airbag is greater than or equal to CSS1 and less than CSS2, the performance level of the micro air pump is Po2; and if the air pressure of the airbag is greater than or equal to CSS2, the performance level of the micro air pump is Po1. When the operating parameter is the operating voltage 13 V and the duty cycle 30%, and inflation time for the air pressure of the airbag starting from the initial air pressure CS0 reaches CT2, if the air pressure of the airbag is greater than or equal to CS0 and less than CSS3, the performance level of the micro air pump is Po3; if the air pressure of the airbag is greater than or equal to CSS3 and less than CSS4, the performance level of the micro air pump is Po2; and if the air pressure of the airbag is greater than or equal to CSS4, the performance level of the micro air pump is Po1.

It may be understood that Table 3 is merely an example in embodiments of this application. In this embodiment of this application, the electronic device 100 may alternatively store more or less content than that in Table 3, or store content different from that in Table 3. This is not limited in this application.

In some embodiments, if airbags of different sizes may be configured for the electronic device 100, a size of the airbag currently used by the electronic device 100 needs to be determined before the performance of the micro air pump is detected. The electronic device 100 may detect the size of the airbag through the Hall effect sensor. For a detection manner, refer to the related content in the embodiment shown in FIG. 1D. In some other embodiments, the electronic device 100 may alternatively prompt the user to enter the size of the airbag when detecting removal or mounting of the airbag, and store the size of the airbag that is entered by the user.

It may be understood that, when airbags of different sizes may be configured for the electronic device 100, the electronic device 100 may store correspondences that are between the performance data and the performance level of the micro air pump when airbags of different sizes are used, and determine the performance level of the micro air pump based on the size of the airbag and the performance data of the micro air pump.

In this way, when airbags of different sizes may be configured for the electronic device 100, the size of the airbag may be determined through the Hall effect sensor, and the size of the airbag is included in a basis for determining the performance of the micro air pump, so that a measurement error caused by a size difference of the airbag can be avoided.

The following describes a schematic flowchart of a measurement method in a blood pressure measurement scenario according to an embodiment of this application.

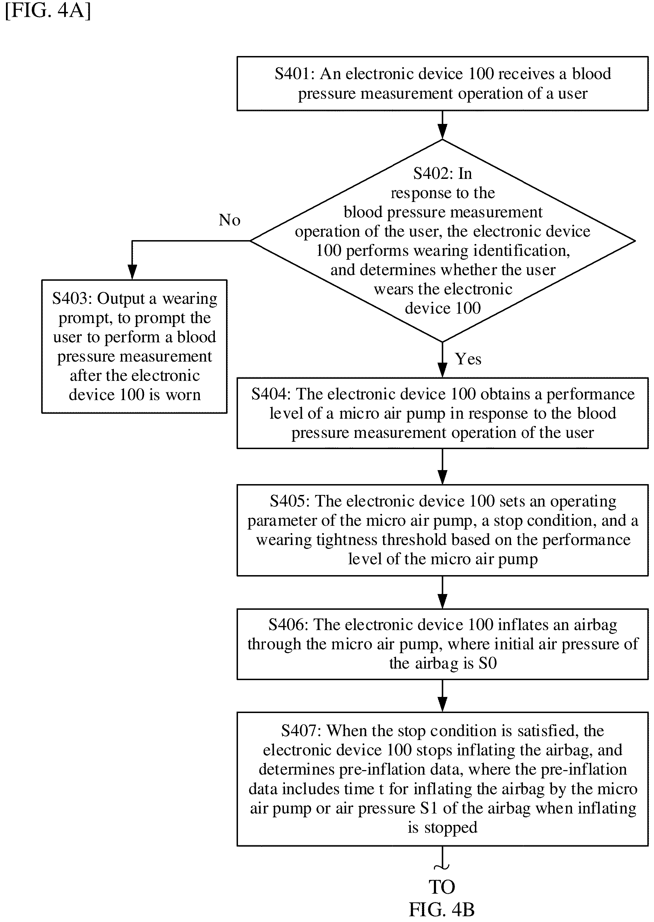

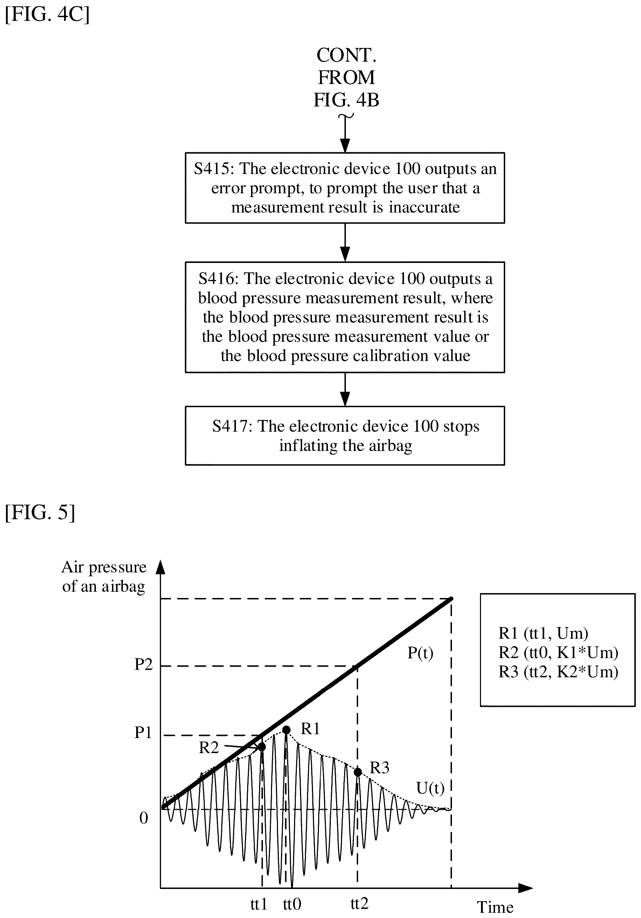

As shown in FIG. 4A to FIG. 4C, a specific procedure of the measurement method in the blood pressure measurement scenario may include the following steps.

S401: An electronic device 100 receives a blood pressure measurement operation of a user.

For example, the blood pressure measurement operation of the user may be a tap operation on the measurement control 212 on the blood pressure measurement interface 210 shown in FIG. 2B.

S402: In response to the blood pressure measurement operation of the user, the electronic device 100 performs wearing identification, and determines whether the user wears the electronic device 100.

In this embodiment of this application, the electronic device 100 may obtain a first signal, and determine, based on the first signal, whether the electronic device is worn by the user. The first signal may include a first pulse wave signal detected by a PPG module and/or a first capacitance signal detected by a capacitive sensor.

For a manner of determining, based on the second signal, whether the user wears the electronic device 100, refer to the related content of step S301 shown in FIG. 3. Details are not described herein again.

If the electronic device 100 determines that the user wears the electronic device 100, the electronic device 100 may perform the following step S404.

If the electronic device 100 determines that the user does not wear the electronic device 100, the electronic device 100 performs the following step S403.

S403: Output a wearing prompt, to prompt the user to perform a blood pressure measurement after the electronic device 100 is worn.

When it is determined that the user does not wear the electronic device 100, the electronic device 100 may output the wearing prompt, where the wearing prompt is used to prompt the user to perform a blood pressure measurement after the electronic device 100 is worn.

Step S402 and step S403 are optional steps. In some embodiments, the electronic device 100 may alternatively perform step S404 after performing step S401.

S404: The electronic device 100 obtains a performance level of a micro air pump in response to the blood pressure measurement operation of the user.

The performance level of the micro air pump may include a plurality of levels, for example, three levels: high (Po1), medium (Po2), and low (Po3), or may include more or fewer levels. The performance level of the micro air pump may be used to measure a rate at which the micro air pump inflates an airbag. It may be understood that the performance level of the micro air pump is merely a parameter used to measure performance of the micro air pump. In this embodiment of this application, the performance level may be replaced with another parameter that can be used to represent the performance. This is not limited in this application.

In some embodiments, the electronic device 100 may measure and store the performance level of the micro air pump before starting the blood pressure measurement. In this case, the electronic device 100 may obtain the performance level of the micro air pump in response to the blood pressure measurement operation of the user. When detecting that a performance detection condition of the micro air pump is satisfied, the electronic device 100 may measure and store the performance level of the micro air pump. For specific content of the performance detection condition of the micro air pump and a manner of detecting the performance of the micro air pump, refer to the related descriptions in the embodiment shown in FIG. 3.

In some other embodiments, the electronic device 100 may also measure the performance level of the micro air pump in response to the blood pressure measurement operation of the user.

Optionally, if airbags of different sizes may be configured for the electronic device 100, the electronic device 100 may further obtain a size of the airbag in response to the blood pressure measurement operation of the user. The sizes of the airbags can be classified into a plurality of levels, such as large (large, L) and small (small, S). In some embodiments, the electronic device 100 may measure and store the size of the airbag when detecting that an airbag detection condition is satisfied (for example, detecting that the airbag is used to measure blood pressure for the first time, or detecting an airbag change operation of the user). The electronic device 100 may determine the size of the airbag based on a measurement result of a Hall effect sensor. For a specific manner, refer to the related content in the embodiment shown in FIG. 1D. Details are not described herein again.

S405: The electronic device 100 sets an operating parameter of the micro air pump, a stop condition, and a wearing tightness threshold based on the performance level of the micro air pump.

The operating parameters of the micro air pump may include a voltage and a duty cycle. The electronic device 100 may supply power to the micro air pump through a component like a battery or a drive circuit. The micro air pump may be connected to two output ends of the drive circuit, for example, an output end A and an output end B. Voltages of the output end A and the output end B are different, and the voltage of the micro air pump is a differential voltage between the voltage of the output end A and the voltage of the output end B. When the voltage at the output end A is higher than the voltage at the output end B, the voltage of the micro air pump is a positive voltage. When the voltage at the output end A is lower than the voltage at the output end B, the voltage of the micro air pump is a negative voltage. In this way, the electronic device 100 may provide an alternating current for the micro air pump.

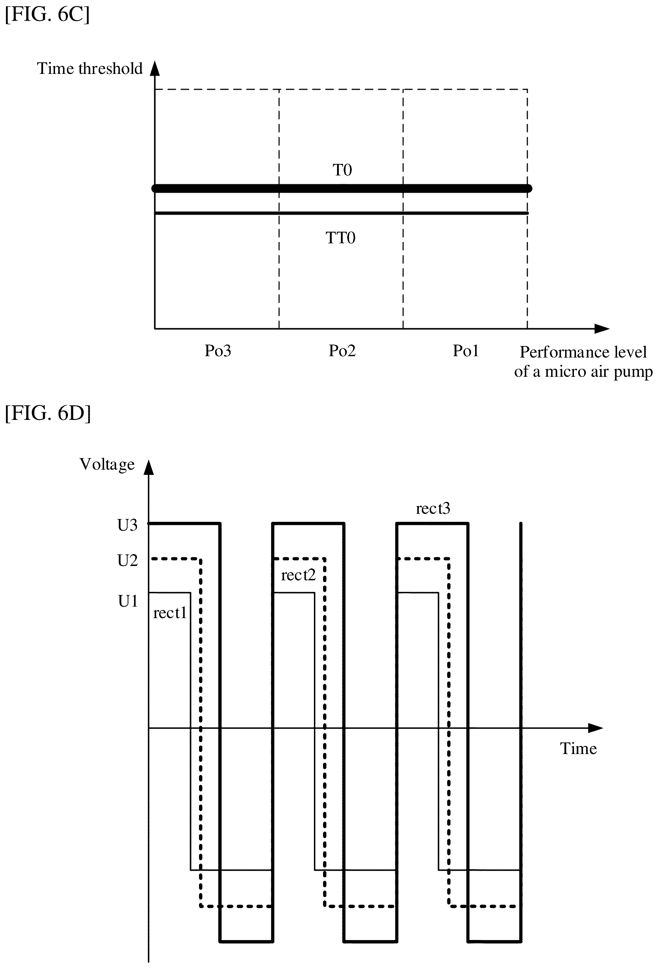

An alternating current voltage provided by the electronic device 100 for the micro air pump may be a rectangular wave. The rectangular wave is a periodic signal, and the rectangular wave includes a high potential and a low potential. The high potential and the low potential correspond to a same absolute value of a voltage. For example, for a diagram of the rectangular wave, refer to the following FIG. 6D. The voltage in the operating parameter may be a high-potential voltage or an absolute value of a low-potential voltage in the rectangular wave. The duty cycle is a proportion of duration of a high-potential voltage in a periodicity to time corresponding to the periodicity in the rectangular wave signal.

The operating parameter of the micro air pump can be used to control the rate at which the micro air pump inflates the airbag. When the duty cycle is constant, a higher voltage indicates a faster pressure rise of the airbag. When the voltage is constant, a higher duty cycle indicates a faster pressure rise of the airbag. In some other embodiments, the operating parameter of the micro air pump may further include another parameter like a frequency. This is not limited in this application.

The wearing tightness threshold may be a time threshold, or may be an air pressure threshold. The wearing tightness threshold may include a tightness threshold 1. The tightness threshold 1 may be used to determine whether wearing of the user is excessively loose, so as to determine whether to output a wearing tightness prompt, and prompt the user to tighten the wearing. In some embodiments, the wearing tightness threshold may further include a tightness threshold 2. The tightness threshold 2 may be used to determine whether wearing of the user is slightly loose, so as to determine whether a blood pressure measurement value needs to be calibrated and/or determine whether to output an error prompt. The error prompt may be used to prompt the user that there is an error in a measurement result.

In this embodiment of this application, if the wearing tightness threshold is a time threshold, the tightness threshold 1 may be referred to as a time threshold T, and the tightness threshold 2 may be referred to as a time threshold TT. Longer inflation time required for air pressure of the airbag to rise from initial air pressure to preset end air pressure indicates looser wearing. Therefore, when the wearing tightness threshold is a time threshold, the following relationship exists between the time threshold T and the time threshold TT: T>TT.

In this embodiment of this application, if the wearing tightness threshold is an air pressure threshold, the tightness threshold 1 may be referred to as an air pressure threshold Y, and the tightness threshold 2 may be referred to as an air pressure threshold YY. When time for inflating the airbag by the micro air pump reaches preset time, lower air pressure of the airbag indicates looser wearing. Therefore, when the wearing tightness threshold is an air pressure threshold, the following relationship exists between the air pressure threshold Y and the air pressure threshold YY: YY>Y.

The stop condition may be that time t for inflating the airbag by the micro air pump reaches preset duration Ts. In some other embodiments, the stop condition may alternatively be that air pressure S1 of the airbag reaches preset air pressure SS as the micro air pump inflates the airbag.

The stop condition may be set before the blood pressure measurement is started, or may be set by the electronic device 100 based on the obtained performance level of the micro air pump. It should be noted that, if the wearing tightness threshold is a time threshold, the stop condition needs to be that the air pressure S1 of the airbag reaches the preset air pressure SS as the micro air pump inflates the airbag based on a specified operating parameter. If the wearing tightness threshold is an air pressure threshold, the stop condition needs to be that time t for inflating the airbag by the micro air pump based on a specified operating parameter reaches the duration Ts.

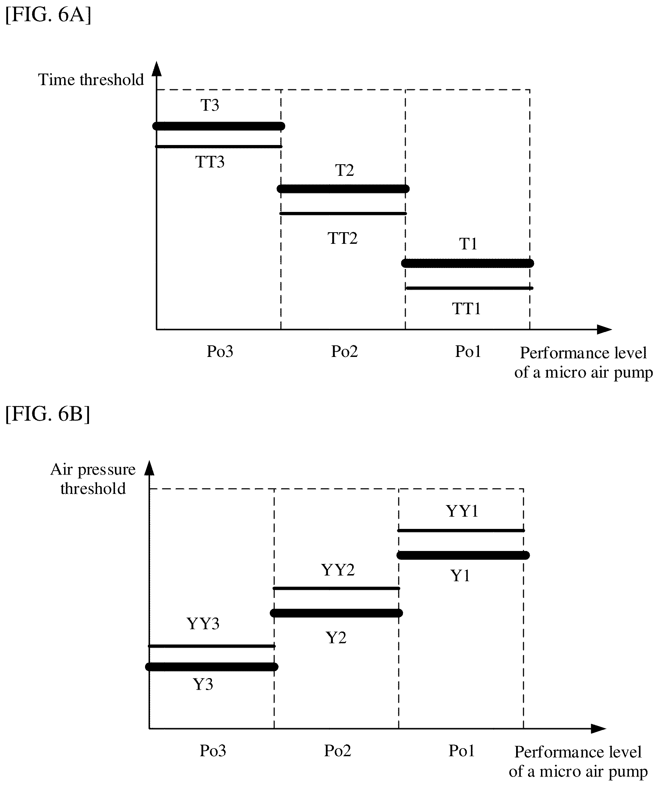

For a specific manner in which the electronic device 100 sets the operating parameter (for example, a third operating parameter or a fourth operating parameter) of the micro air pump and the wearing tightness threshold based on the performance level of the micro air pump, refer to related descriptions in the following embodiments shown in FIG. 6A to FIG. 6D. Details are not described herein.

S406: The electronic device 100 inflates the airbag through the micro air pump, where initial air pressure of the airbag is S0.

The initial air pressure S0 may be a preset air pressure value. The initial air pressure S0 may be air pressure in a non-inflated state of the airbag, for example, 0.

Before the electronic device 100 inflates the airbag through the micro air pump, the electronic device 100 may detect the air pressure of the airbag through an air pressure sensor, and determine whether the air pressure of the airbag is S0.

If it is determined that the air pressure of the airbag is S0, the electronic device 100 may start to inflate the airbag through the micro air pump.

If it is determined that the air pressure of the airbag is greater than S0, the electronic device 100 may prompt the user to perform an air release operation on the airbag, and after the air pressure of the airbag decreases to S0, prompt the user to stop air release. In this case, the electronic device 100 may start to inflate the airbag through the micro air pump.

S407: When the stop condition is satisfied, the electronic device 100 stops inflating the airbag, and determines pre-inflation data, where the pre-inflation data includes the time t for inflating the airbag by the micro air pump or the air pressure S1 of the airbag when inflating is stopped.

It may be understood that, when the stop condition is that the time for inflating the airbag by the micro air pump reaches the duration Ts, the pre-inflation data is the air pressure S1 of the airbag when inflating is stopped. When the stop condition is that the air pressure of the airbag reaches the preset air pressure SS, the pre-inflation data is the time t for inflating the airbag by the micro air pump.

S408: The electronic device 100 determines wearing tightness of the user based on the pre-inflation data and the wearing tightness threshold.

The electronic device 100 may determine the wearing tightness of the user based on the pre-inflation data and the wearing tightness threshold, and determine a subsequent to-be-performed step based on the wearing tightness of the user. The wearing tightness of the user may include excessively loose wearing and proper wearing, and optionally, may further include slightly loose wearing.

It can be learned from step S405 that, in some embodiments, the wearing tightness threshold may include the tightness threshold 1 (for example, the time threshold T or the air pressure threshold Y) and the tightness threshold 2 (for example, the time threshold TT or the air pressure threshold YY).

When the wearing tightness threshold is a time threshold, it can be learned based on a relationship between the wearing tightness threshold, the stop condition, and the pre-inflation data in step S407 that the pre-inflation data is the time t for inflating the airbag by the micro air pump when the air pressure of the airbag reaches the preset air pressure SS from the initial air pressure S0.

In this case, the electronic device 100 may determine the wearing tightness of the user based on a value relationship between the time t, the time threshold T, and the time threshold TT. When the time t is greater than or equal to the time threshold T, the electronic device 100 determines that the wearing of the user is excessively loose, and determines not to continue the blood pressure measurement, and the electronic device 100 performs the following step S409. When the time t is less than the time threshold T and is greater than or equal to the time threshold TT, the electronic device 100 determines that the user wears the electronic device 100 slightly loosely, determines to continue the blood pressure measurement, and performs the following step S413. When the time t is less than the time threshold TT, the electronic device 100 determines that the user wears the electronic device 100 properly, determines to continue the blood pressure measurement, and performs step S410.

When the wearing tightness threshold is an air pressure threshold, it can be learned based on a relationship between the wearing tightness threshold, the stop condition, and the pre-inflation data in step S407 that the pre-inflation data is the air pressure S1 of the airbag when the time for inflating the airbag by the micro air pump reaches the duration Ts starting from the air pressure of the airbag S0.

In this case, the electronic device 100 may determine the wearing tightness of the user based on a value relationship between the air pressure S1 of the airbag, the air pressure threshold Y, and the air pressure threshold YY. When the air pressure S1 is less than or equal to the air pressure threshold Y, the electronic device 100 determines that the wearing of the user is excessively loose, and determines not to continue to the blood pressure measurement, and the electronic device 100 performs the following step S409. When the air pressure S1 is less than or equal to the air pressure threshold YY and is greater than the air pressure threshold Y, the electronic device 100 determines that the user wears the electronic device 100 slightly loosely, determines to continue the blood pressure measurement, and performs the following step S413. When the air pressure S1 is greater than the air pressure threshold YY, the electronic device 100 determines that the user wears the electronic device 100 properly, determines to continue the blood pressure measurement, and performs step S410.

In some other embodiments, the wearing tightness threshold may include only the tightness threshold 1. In this case, the electronic device 100 may determine, based on the tightness threshold 1 and the pre-inflation data, whether to continue the blood pressure measurement. If it is determined to continue the measurement, step S410 is performed; or if it is determined not to continue the measurement, step S409 is performed.

S409: The electronic device 100 outputs a wearing tightness prompt, to prompt the user to adjust the wearing tightness.

When the electronic device 100 determines that the wearing of the user is excessively loose, the electronic device 100 may output the wearing tightness prompt, where the wearing tightness prompt may be used to prompt the user to adjust the wearing tightness. The wearing tightness prompt may be any one or more of the following forms: an animation prompt, a picture prompt, a voice prompt, a text prompt, and the like. For example, the wearing tightness prompt may be the related content in the embodiment shown in FIG. 2E.

In some embodiments, after the user adjusts the wearing tightness, the electronic device 100 may release air in the airbag (or remind the user to release air in the airbag), until the air pressure of the airbag is restored to the initial air pressure S0. Then, the electronic device 100 may receive and respond to the blood pressure measurement operation of the user, and re-perform step S402 and subsequent steps.

In some embodiments, after outputting the wearing tightness prompt, the electronic device 100 may release air in the airbag until the air pressure of the airbag is restored to the initial air pressure S0. In addition, the electronic device 100 may further monitor in real time whether the user wears the electronic device 100. After detecting that the user wears the electronic device 100 again, the electronic device 100 may re-perform step S406 and subsequent steps.

S410: The electronic device 100 continues to inflate the airbag through the micro air pump, to determine a blood pressure measurement value.

The blood pressure measurement value includes systolic pressure (namely, high pressure) and diastolic pressure (namely, low pressure). In some embodiments, in the process in which the electronic device 100 inflates the airbag through the micro air pump, the electronic device 100 may obtain a pulse wave signal of the user through parsing based on measurement data of the air pressure sensor. In some other embodiments, the electronic device 100 may alternatively obtain a pulse wave signal of the user through parsing based on a PPG signal collected by a PPG module. The electronic device 100 may determine the blood pressure measurement value when detecting that the pulse wave signal satisfies a value output condition. The value output condition may include that an amplitude of the pulse wave signal reaches a product of a peak value of the pulse wave signal and a preset proportion.

The following describes, with reference to a blood pressure measurement principle, the value output condition provided in this embodiment of this application.

FIG. 5 is a diagram in which the air pressure of the airbag and the pulse wave signal change with time in the process in which the electronic device 100 inflates the airbag through the micro air pump.

As shown in FIG. 5, a horizontal axis may represent time, and an increasing value in a positive direction of the horizontal axis indicates longer inflation time; and a vertical axis may represent the air pressure of the airbag, and an increasing value in a positive direction of the vertical axis indicates higher air pressure; or a vertical axis may represent a pulse wave amplitude, and an increasing value in a positive direction of the vertical axis indicates higher amplitude. In the process in which the electronic device 100 inflates the airbag through the micro air pump, a curve in which the air pressure of the airbag changes with the inflation time may be a curve P (t) shown in FIG. 5, and a curve in which the amplitude of the pulse wave signal changes with the time may be a curve U(t) shown in FIG. 5.

It can be learned from FIG. 5 that, in the process in which the micro air pump inflates the airbag, the airbag continuously pressurizes a blood vessel of the user, and an envelope of the amplitude of the pulse wave signal first increases and then decreases until the envelope completely disappears. It can be learned from experimental measurement data that, if a maximum value of the pulse wave amplitude is a peak value Um, the diastolic pressure (low pressure) of the user is air pressure of the airbag when the pulse wave amplitude reaches K1*Um before the peak value, and the systolic pressure (high pressure) of the user is air pressure of the airbag when the pulse wave amplitude reaches K2*Um after the peak value. Both K1 and K2 are real numbers greater than 0 and less than 1. For example, K1 may be 0.8, and K2 may be 0.5. It can be learned from FIG. 5 that the following three points exist on the curve U(t): a point R1 (tt0, Um), a point R2 (tt1, K1*Um), and a point R3 (tt2, K2*Um). A parameter on a left side of a bracket represents inflation time corresponding to a point, and a parameter on a right side of the bracket represents a pulse amplitude of the point.

It can be learned from the coordinates of the point R2 that, before the peak value, when the pulse wave amplitude reaches K1*Um, the inflation time is tt1. It can be learned from the coordinates of the point R3 that, after the peak value, when the pulse wave amplitude reaches K2*Um, the inflation time is tt2. In this case, it can be learned from the curve P(t) that the air pressure of the airbag is P1 when the inflation time is tt1, and the air pressure of the airbag is P2 when the inflation time is tt2. Therefore, it may be determined that the diastolic pressure of the user is P1, and the systolic pressure is P2.

It may be understood that FIG. 5 merely illustrates a pressure rise curve of the air pressure of the airbag and a change curve of the pulse wave amplitude in the process in which the micro air pump inflates the airbag. In this embodiment of this application, the pressure rise curve of the air pressure of the airbag and the change curve of the pulse wave amplitude may alternatively be different curves. This is not limited in this application.

In some embodiments, the electronic device 100 may determine the blood pressure measurement value of the user based on the change of the pulse wave amplitude in the blood pressure measurement manner in the embodiment shown in FIG. 5. In this case, the value output condition may be that the amplitude of the pulse wave of the user decreases to K2*Um after the peak value, where Um is the peak value of the pulse wave. After detecting that the value output condition is satisfied, the electronic device 100 may determine the air pressure of the airbag in the case of the pulse wave amplitude reaching K1*Um before the peak value as the diastolic pressure, and determine the air pressure of the airbag in the case of the pulse wave amplitude of the user decreasing to K2*Um after the peak value as the systolic pressure.

In some other embodiments, in addition to the foregoing proportional relationship between the pulse wave amplitude and the peak value, the electronic device 100 may further obtain the blood pressure measurement value of the user through analysis based on parameters such as a moment corresponding to the peak value of the pulse wave, rise time, a rise rate, and a rise slope of a pulse wave peak envelope, and fall time and a fall rate of the pulse wave peak envelope. In this case, the value output condition may alternatively include content related to one or more of the foregoing parameters.

S411: The electronic device 100 outputs the blood pressure measurement value.

S412: The electronic device 100 stops inflating the airbag through the micro air pump.

When obtaining the blood pressure measurement value, the electronic device 100 may stop inflating the airbag. Step S412 may be performed after step S410 is performed.

S413: The electronic device 100 continues to inflate the airbag through the micro air pump, to determine a blood pressure measurement value.

For specific content of step S413, refer to the related content of step S410.

S414: The electronic device 100 determines a blood pressure calibration value based on the blood pressure measurement value, where the blood pressure calibration value is a result obtained by calibrating the blood pressure measurement value.

When the electronic device 100 determines that the electronic device 100 is worn slightly loosely, the electronic device 100 may calibrate the blood pressure measurement value after obtaining the blood pressure measurement value, to obtain the blood pressure calibration value.

In some embodiments, the electronic device 100 may store a calibration algorithm. The calibration algorithm may be obtained by the electronic device 100 or another electronic device through training based on a plurality of groups of measurement data. Each group of measurement data may include a performance level of the micro air pump, a size of the airbag, an operating parameter of the micro air pump, initial air pressure and end air pressure of the airbag, pre-inflation time of the airbag, a blood pressure measurement value, pre-inflation time during proper wearing, a real blood pressure value, and the like. After determining the blood pressure measurement value and the pre-inflation data, the electronic device 100 may calibrate the blood pressure measurement value based on the pre-inflation data and the tightness threshold 2 according to the calibration algorithm, to obtain the blood pressure calibration value.

In some other embodiments, the electronic device 100 may store a correspondence between the pre-inflation data and a deviation value in different performance conditions (including the performance level of the micro air pump). After the electronic device 100 determines the pre-inflation data and the blood pressure measurement value, the electronic device 100 may determine the corresponding deviation value from the correspondence between the pre-inflation data and the deviation value based on the pre-inflation data, and calibrate the blood pressure measurement value based on the deviation value, to obtain the blood pressure calibration value.

S415: The electronic device 100 outputs an error prompt, to prompt the user that the measurement result is inaccurate.

For example, the error prompt may be the error prompt 221 shown in FIG. 2C, or may be displayed content in the prompt window 231 shown in FIG. 2D.

Step S414 and step S415 are both optional steps. The electronic device 100 may perform step S416 after performing step S414, or may perform step S415 after performing step S413.

S416: The electronic device 100 outputs a blood pressure measurement result, where the blood pressure measurement result is the blood pressure measurement value or the blood pressure calibration value.

When it is determined to calibrate the blood pressure measurement value, the electronic device 100 may use the blood pressure calibration value as the blood pressure measurement result for output. When it is determined not to calibrate the blood pressure measurement value, the electronic device 100 may use the blood pressure measurement value as the blood pressure measurement result for output.

S417: The electronic device 100 stops inflating the airbag.

When obtaining the blood pressure measurement value, the electronic device 100 may stop inflating the airbag. Step S417 may be performed after step S413 is performed.

According to the blood pressure measurement method provided in this embodiment of this application, the operating parameter of the micro air pump and the wearing tightness threshold may be set based on the performance level of the micro air pump, to avoid a measurement error caused by excessively loose wearing, and further avoid a measurement error caused by performance of the micro air pump or performance of the airbag, so as to improve accuracy of the blood pressure measurement value.

It may be understood that FIG. 4A to FIG. 4C merely describes an example of application of the measurement method provided in embodiments of this application in the blood pressure measurement scenario. In this embodiment of this application, the measured physiological parameter may alternatively be another physiological parameter like a heart rate or a body temperature. In addition, when the another physiological parameter is measured, the wearing tightness of the user may also be measured by using the measurement method provided in this embodiment of this application, and subsequent to-be-performed steps are determined based on the wearing tightness (for example, obtaining measurement data of the physiological parameter, calibrating the measurement data, outputting a wearing tightness prompt, and outputting an error prompt). For a relationship between the wearing tightness and the subsequent to-be-performed steps, refer to the related steps in the embodiment shown in FIG. 4A to FIG. 4C. Details are not described herein again.

The following describes a manner in which the electronic device 100 sets the operating parameter, the stop condition, and the wearing tightness threshold based on the performance level of the micro air pump according to an embodiment of this application.

The electronic device 100 may store the correspondence between the performance level and the performance data of the micro air pump. For specific content, refer to the related descriptions of step S304 shown in FIG. 3. The performance data may include the operating parameter of the micro air pump, the initial and end air pressure of the airbag, and the time for inflating the airbag by the micro air pump. Optionally, the size of the airbag and the like may be further included.

The electronic device 100 may set the operating parameter, the stop condition, and the wearing tightness threshold based on the performance level of the micro air pump and the correspondence between the performance data and the performance level of the micro air pump.

In some embodiments, the electronic device 100 may first set the operating parameter of the micro air pump. The operating parameter of the micro air pump may be an operating parameter in the performance data (for example, the embodiment shown in Table 2 or Table 3) of the micro air pump. After determining the operating parameter of the micro air pump, the electronic device 100 may determine, based on the performance level of the micro air pump and the specified operating parameter, a correspondence between the initial and end air pressure of the airbag and the time for inflating the airbag by the micro air pump when the micro air pump of the performance level operates based on the specified operating parameter, and set the stop condition and the wearing tightness threshold accordingly.

FIG. 6A is a diagram of a correspondence between the performance level of the micro air pump and the wearing tightness threshold (a time threshold) when the operating parameter is determined according to an embodiment of this application.

For example, as shown in FIG. 6A, a horizontal axis of a coordinate system may represent the performance level of the micro air pump, and an increasing value in a positive direction of the horizontal axis indicates a higher performance level. The performance of the micro air pump may be classified into a plurality of performance levels, and the plurality of performance levels sequentially include Po1, Po2, and Po3 in descending order. A vertical axis of the coordinate system may represent the time threshold, and an increasing value in a positive direction of the vertical axis indicates a larger time threshold. It can be learned from FIG. 6A that, under the preset operating parameter, when the performance level of the micro air pump is Po1, the time threshold T (namely, the tightness threshold 1) may be set to T1, and the time threshold TT (namely, the tightness threshold 2) may be set to TT1; under the preset operating parameter, when the performance level of the micro air pump is Po2, the time threshold T may be set to T2, and the time threshold TT may be set to TT2; and under the preset operating parameter, when the performance level of the micro air pump is Po3, the time threshold T may be set to T3, and the time threshold TT may be set to TT3.

It may be understood that, for the same micro air pump and the same airbag, looser wearing of the user indicates longer time consumed for the air pressure of the airbag to rise from the initial air pressure to the preset air pressure. Therefore, when the performance level of the micro air pump is determined, the time threshold T needs to be greater than the time threshold TT, that is, T1>TT1, T2>TT2, and T3>TT3. In addition, a higher performance level of the micro air pump indicates shorter time consumed for the air pressure of the airbag to rise from the initial air pressure to the preset air pressure. Therefore, the following relationship further exists between the time thresholds: T1<T2<T3, and TT1<TT2<TT3.

FIG. 6B is a diagram of a correspondence between the performance level of the micro air pump and the wearing tightness threshold (an air pressure threshold) when the operating parameter is determined according to an embodiment of this application.

For another example, as shown in FIG. 6B, a horizontal axis of a coordinate system may represent the performance level of the micro air pump, and an increasing value in a positive direction of the horizontal axis indicates a higher performance level. The performance of the micro air pump may be classified into a plurality of performance levels, and the plurality of performance levels sequentially include Po1, Po2, and Po3 in descending order. A vertical axis of the coordinate system may represent the air pressure threshold, and an increasing value in a positive direction of the vertical axis indicates a larger air pressure threshold. It can be learned from FIG. 6B that, under the operating parameter, when the performance level of the micro air pump is Po1, the air pressure threshold Y (namely, the tightness threshold 1) may be set to Y1, and the air pressure threshold YY (namely, the tightness threshold 2) may be set to YY1; under the operating parameter, when the performance level of the micro air pump is Po2, the air pressure threshold Y may be set to Y2, and the air pressure threshold YY may be set to YY2; and under the operating parameter, when the performance level of the micro air pump is Po3, the air pressure threshold Y may be set to Y3, and the air pressure threshold YY may be set to YY3.

It should be noted that, for the same micro air pump and the same airbag, looser wearing of the user indicates a lower air pressure value of the airbag when time for inflating the air pressure of the airbag from the initial air pressure reaches the preset time. Therefore, when the performance level of the micro air pump is determined, the air pressure threshold Y needs to be less than the air pressure threshold YY, that is, Y1<YY1, Y2<YY2, and Y3<YY3. In addition, a higher performance level of the micro air pump indicates a higher air pressure value of the airbag when time for inflating the air pressure of the airbag from the initial air pressure reaches the preset time. Therefore, the following relationship further exists between the air pressure thresholds: Y1>Y2>Y3, and YY1>YY2>YY3.

It may be understood that FIG. 6A and FIG. 6B merely illustrate two correspondences between the performance level of the micro air pump and the wearing tightness threshold when the operating parameter is determined. In some embodiments, the electronic device 100 may store more cases for different operating parameters, or store the foregoing content in another form like a table. This is not limited in this application.

In some other embodiments, the electronic device 100 may alternatively first determine the wearing tightness threshold and the stop condition. After determining the wearing tightness threshold and the stop condition, the electronic device 100 may determine, based on the performance level of the micro air pump, the wearing tightness threshold, and the stop condition, the operating parameter corresponding to the micro air pump of the performance level.

FIG. 6C is a diagram of a correspondence between the performance level of the micro air pump and the wearing tightness threshold (a time threshold) when the wearing tightness threshold is determined according to an embodiment of this application.

For example, as shown in FIG. 6C, for content represented by a horizontal axis and a vertical axis of a coordinate system, refer to the related content in the coordinate system shown in FIG. 6A. The performance of the micro air pump may be classified into a plurality of performance levels, and the plurality of performance levels sequentially include Po1, Po2, and Po3 in descending order. It can be learned from FIG. 6C that, when the performance level of the micro air pump is any one of the performance levels Po1, Po2, and Po3, the time threshold T used to determine whether the wearing of the user is excessively loose may be set to T0, and the time threshold TT used to determine whether the blood pressure measurement value needs to be calibrated may be set to TT0.

Similarly, when the wearing tightness threshold is an air pressure threshold, for a relationship between the wearing tightness threshold and the performance level, refer to the related content in the embodiment shown in FIG. 6C.

FIG. 6D is a diagram of a correspondence between the performance level and the operating parameter of the micro air pump when the wearing tightness threshold is determined according to an embodiment of this application.

For example, as shown in FIG. 6D, a horizontal axis of a coordinate system may represent time, and a vertical axis may represent a voltage. In addition, an increasing value in a positive direction of the vertical axis indicates a higher voltage. The performance of the micro air pump may be classified into a plurality of performance levels, and the plurality of performance levels sequentially include Po1, Po2, and Po3 in descending order. It can be learned from FIG. 6D that, when the performance level of the micro air pump is Po1, a corresponding voltage waveform is rect1, and in this case, the operating parameter includes a voltage U1 and a duty cycle B1; when the performance level of the micro air pump is Po2, a corresponding voltage waveform is rect2, and in this case, the operating parameter includes a voltage U2 and a duty cycle B2; and when the performance level of the micro air pump is Po3, a corresponding voltage waveform is rect3, and in this case, the operating parameter includes a voltage U3 and a duty cycle B3. Operating voltages corresponding to different performance levels may have the following relationship: U1<U2<U3, and duty cycles corresponding to different performance levels may have the following relationship: B1<B2<B3.

It may be understood that the embodiment shown in FIG. 6D is merely an example. In some embodiments, the electronic device 100 may store the correspondence between the performance level and the operating parameter of the micro air pump in another form like a table. In addition, the electronic device 100 may store more cases for different wearing tightness thresholds. This is not limited herein in this application.

According to the blood pressure measurement method provided in this embodiment of this application, an error caused by performance deterioration of the micro air pump can be avoided, and measurement accuracy can be improved.

It should be noted that, in some embodiments, if a plurality of airbags of different sizes may be configured for the electronic device 100, the electronic device 100 needs to first determine a size of the airbag before setting the operating parameter and the wearing tightness threshold. In this case, the electronic device 100 may store a correspondence between the performance level and the wearing tightness threshold when the micro air pump inflates airbags of different sizes based on different operating parameters, or store a correspondence between the performance level and the operating parameter of the micro air pump when the wearing tightness threshold and the size of the airbag are given, or the like. Then, the electronic device 100 may set the operating parameter of the micro air pump and the wearing tightness threshold based on the size of the airbag and the performance level of the micro air pump. For a specific manner, refer to the content in the foregoing embodiment. Details are not described herein again.

In some embodiments, the electronic device 100 may alternatively determine the wearing tightness of the user with reference to the PPG signal detected by the PPG module.

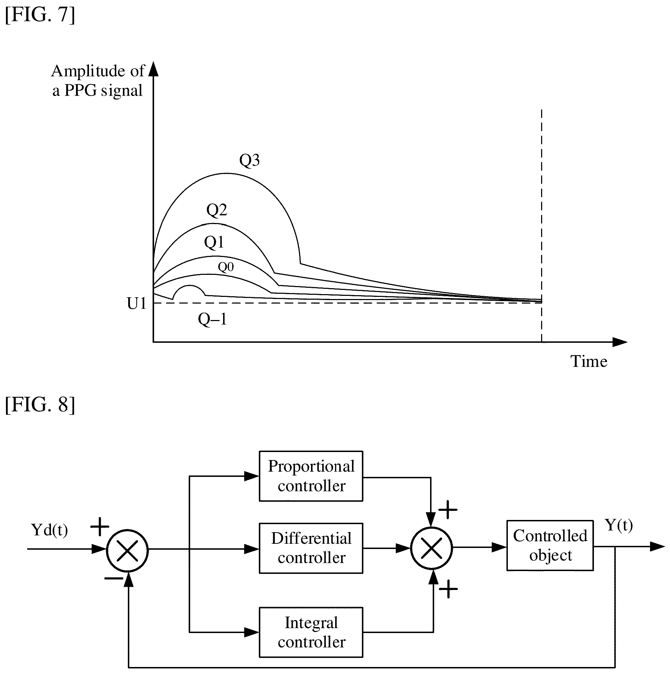

FIG. 7 is a diagram of photoplethysmography signals in the process of inflating the airbag by the micro air pump at different wearing tightness according to an embodiment of this application.

As shown in FIG. 7, the wearing tightness of the user from tight to loose may sequentially include slightly tight wearing, proper wearing, slightly loose wearing, relatively loose wearing, loose wearing, excessively loose wearing, and the like. In the case of slightly tight wearing, a curve corresponding to the PPG signal is a curve Q-1; in the case of proper wearing, a curve corresponding to the PPG signal is a curve Q0; in the case of slightly loose wearing, a curve corresponding to the PPG signal is a curve Q1; in the case of relatively loose wearing, a curve corresponding to the PPG signal is a curve Q2; and in the case of excessively loose wearing, a curve corresponding to the PPG signal is a curve Q3.

It can be learned from FIG. 7 that, in the process in which the micro air pump inflates the airbag, an amplitude of the PPG signal first increases and then decreases until the amplitude decreases to U1. In addition, looser wearing of the user indicates a larger amplitude change of the curve of the PPG signal in the process in which the micro air pump inflates the airbag; and tighter wearing of the user indicates a smaller amplitude change of the curve of the PPG signal in the process in which the micro air pump inflates the airbag.

The electronic device 100 may set a PPG threshold. When detecting that a difference between a maximum value and a minimum value of the amplitude of the PPG signal in the process in which the micro air pump inflates the airbag is greater than or equal to the preset PPG threshold, the electronic device 100 may determine that the wearing of the user is excessively loose.

In this embodiment of this application, the electronic device 100 may determine the wearing tightness of the user by using both the determining manner that is based on the pre-inflation data of the micro air pump in the embodiment shown in FIG. 4A to FIG. 4C and the determining manner that is based on the PPG signal shown in FIG. 7. If a determining result in either of the foregoing two manners is that the wearing of the user is excessively loose, the electronic device 100 may determine that the wearing of the user is excessively loose. In this case, the electronic device 100 may output a wearing tightness prompt, to prompt the user to adjust the wearing tightness. If both determining results in the foregoing two manners are not excessively loose wearing, the electronic device 100 determines not to output the wearing tightness prompt, and continues the physiological parameter measurement.

In some embodiments, the electronic device 100 may alternatively control a rise rate of the air pressure of the airbag in a proportion integral differential (proportion integral differential, PID) regulation manner, so that the air pressure of the airbag can rise steadily.

The PID algorithm is a control manner in which correction is performed based on an output feedback of a controlled object, and a deviation between an actual measured value and a planned value is corrected based on a quota or standard. PID is short for proportion (proportion), integral (integral), and differential (differential coefficient), which represent three control algorithms respectively. A combination of the three algorithms can effectively correct the deviation of the controlled object, so that the controlled object can reach a stable state.

FIG. 8 is a diagram of a PID control principle according to an embodiment of this application.

As shown in FIG. 8, a PID control system may include a proportional controller, an integral controller, and a differential controller. A signal Y(t) may represent a current value of the controlled object, and a signal Yd(t) may represent a target value preset for the controlled object. An input signal is a deviation between the target value and the current value of the controlled object. After the input signal is separately regulated by the proportional controller, the integral controller, and the differential controller, a regulation manner for the controlled object may be determined. After the controlled object is regulated, the deviation between the current value and the preset target value of the controlled object may be recalculated, and it is determined whether PID regulation needs to be performed again.

In this embodiment of this application, an air pressure value of the airbag may be the controlled object. The electronic device 100 may regulate the air pressure of the airbag in the PID regulation manner by changing the operating parameter of the micro air pump or a status of an air valve. In this way, after the value output condition is satisfied, the air pressure of the airbag can be stable around an air pressure value, to avoid uncomfortable use experience brought to the user due to excessively high air pressure.

In some embodiments, a flow monitoring sensor may be further disposed in the watch body 11 shown in FIG. 1A, and the flow monitoring sensor may be configured to measure a volume of an air flow inflated by the micro air pump into the airbag. The electronic device 100 may determine the performance level of the micro air pump based on detection data of the flow monitoring sensor in the performance detection phase of the micro air pump, or determine the wearing tightness of the user based on detection data of the flow monitoring sensor in the pre-inflation phase. In some embodiments, the electronic device 100 may alternatively determine the wearing tightness of the user with reference to detection data of the air pressure sensor and the flow monitoring sensor.

This can improve accuracy of determining the wearing tightness of the user.

For example, FIG. 9A is a diagram of a connection relationship between a flow monitoring sensor and another component related to the wearing tightness measurement in a watch body according to an embodiment of this application.

As shown in FIG. 9A, a watch body 11B may include a flow monitoring sensor. An electrical connection may be disposed between the flow monitoring sensor and a processor, and the processor may control the flow monitoring sensor to detect an air flow in the watch body 11B. For another component inside the watch body 11B, refer to the related content of the watch body 11A shown in FIG. 1C. In a process in which the micro air pump inflates an airbag 13 through an air path connection, the air flow inside the surface 11B is affected. Under a condition of a same operating parameter, better performance of the micro air pump indicates a larger air flow detected by the flow monitoring sensor.

For example, FIG. 9B is a diagram of another connection relationship between a flow monitoring sensor and another component related to the wearing tightness measurement in a watch body according to an embodiment of this application.

As shown in FIG. 9B, a watch body 11C may include a flow monitoring sensor, and the flow monitoring sensor may be disposed on an air path connection between the micro air pump and the airbag. An electrical connection may be further disposed between the flow monitoring sensor and a processor, and the processor may control the flow monitoring sensor to detect an air flow of the air path connection between the micro air pump and the airbag 13. For another component inside the watch body 11C, refer to the related content of the watch body 11A shown in FIG. 1C. In a process in which the micro air pump inflates an airbag 13 through the air path connection, the flow monitoring sensor may detect another flow of inflating the airbag 13 by the micro air pump. Under a condition of a same operating parameter, better performance of the micro air pump indicates a larger air flow detected by the flow monitoring sensor.

In some embodiments, in the performance detection phase of the micro air pump, the electronic device 100 may monitor, based on the flow monitoring sensor, data of the air flow in the watch body 11B, or monitor, based on the flow monitoring sensor, data of the air flow for inflating the airbag 13 by the micro air pump, and determine the performance level of the micro air pump based on the operating parameter of the micro air pump and the air flow data monitored by the flow monitoring sensor.

In some other embodiments, when the stop condition in the embodiment shown in FIG. 4A to FIG. 4C is that the air pressure of the airbag reaches the preset air pressure SS, the electronic device 100 may also set a flow threshold based on the performance level of the micro air pump, and determine the wearing tightness of the user based on a comparison result between the air flow data collected by the flow monitoring sensor and the flow threshold. When the air flow data is greater than or equal to the flow threshold, it may be determined that the wearing of the user is excessively loose. When the air flow data is less than the flow threshold, it may be determined that the wearing of the user is not excessively loose. The flow threshold may further include a plurality of sub-flow thresholds, which are used to further determine whether the user wears the electronic device 100 slightly loosely, proper, or the like.

In this embodiment of this application, the electronic device 100 may determine the wearing tightness of the user by using both the determining manner that is based on the pre-inflation data of the micro air pump in the embodiment shown in FIG. 4A to FIG. 4C and the determining manner that is based on the flow monitoring sensor shown in FIG. 9A (or FIG. 9B). If a determining result in either of the foregoing two manners is that the wearing of the user is excessively loose, the electronic device 100 may determine that the wearing of the user is excessively loose. In this case, the electronic device 100 may output a wearing tightness prompt, to prompt the user to adjust the wearing tightness. If both determining results in the foregoing two manners are not excessively loose wearing, the electronic device 100 determines not to output the wearing tightness prompt, and continues the physiological parameter measurement. After the wearing tightness of the user is determined, for subsequent steps performed by the electronic device 100, refer to the related steps in the embodiment shown in FIG. 4A to FIG. 4C. Details are not described herein again.

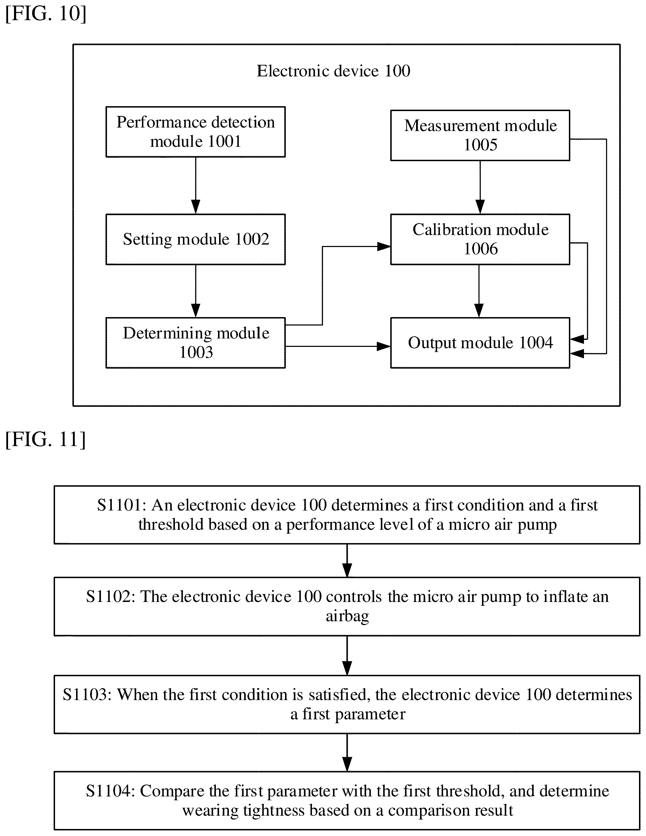

FIG. 10 is a diagram of functional modules of an electronic device 100 according to an embodiment of this application.

As shown in FIG. 10, the electronic device 100 may include a performance detection module 1001, a setting module 1002, a determining module 1003, an output module 1004, a measurement module 1005, and a calibration module 1006.

The performance detection module 1001 may detect performance of a micro air pump in the electronic device 100, and store a performance level of the micro air pump. In some embodiments, after determining that the stored performance level of the micro air pump changes, the performance detection module 1001 may further send a changed performance level of the micro air pump to the setting module 1002. In some other embodiments, the performance detection module 1001 may alternatively send the performance level of the micro air pump to the setting module 1002 in response to a physiological parameter measurement operation (for example, a blood pressure detection operation or a heart rate detection operation) of a user. In some embodiments, the performance detection module 1001 may also detect and store a size of an airbag in the electronic device 100, and send the size of the airbag to the setting module 1002.

The setting module 1002 may receive the performance level that is of the micro air pump and that is sent by the performance detection module 1001, and set an operating parameter of the micro air pump and a wearing tightness threshold (for example, a tightness threshold 1 and a tightness threshold 2) based on the performance level of the micro air pump. The setting module 1002 may further send the specified operating parameter and the wearing tightness threshold to the determining module 1003.

The determining module 1003 may control the micro air pump to inflate the airbag based on the specified operating parameter, obtain pre-inflation data, and determine wearing tightness of the user based on the pre-inflation data and the wearing tightness threshold. For a specific determining manner, refer to the related steps in the embodiment shown in FIG. 4A to FIG. 4C. Details are not described herein again. In some other embodiments, the determining module 1003 may alternatively determine the wearing tightness of the user based on a PPG signal and a preset PPG change threshold. In some other embodiments, the determining module 1003 may alternatively determine the wearing tightness of the user based on flow data collected by a flow monitoring sensor and a preset flow threshold.

When the determining module 1003 determines that the wearing of the user is excessively loose, the output module 1004 may output a wearing tightness prompt, to prompt the user to adjust the wearing tightness. When the determining module 1003 determines that the user wears the electronic device 100 slightly loosely, the output module 1004 may output an error prompt, to prompt the user that a measurement result is inaccurate. The output module 1004 may further output a detection result (for example, a blood pressure measurement value or a blood pressure calibration value obtained through calibration) of a physiological parameter. In some embodiments, the electronic device 100 may further perform wearing detection. If it is detected that the electronic device 100 is not worn by the user, the output module 1004 may further output a wearing prompt, to prompt the user to wear the electronic device 100. In some embodiments, the output module 1004 may further output an operation guide to guide the user to measure performance of the micro air pump.

The measurement module 1005 may obtain measurement data. In a blood pressure measurement scenario, the measurement module 1005 may obtain measurement data of an air pressure sensor, analyze the obtained measurement data to obtain an amplitude change of a pulse wave, and obtain a blood pressure measurement value based on the amplitude change of the pulse wave and a preset value output condition.

When the determining module 1003 determines that the measurement data (for example, the blood pressure measurement value) needs to be calibrated, the calibration module 1006 may obtain the blood pressure measurement value measured by the measurement module 1005, and obtain a blood pressure calibration value based on the blood pressure measurement value and a calibration algorithm.

It may be understood that the embodiment shown in FIG. 10 is merely an example. In this embodiment of this application, the electronic device 100 may further include more or fewer modules than those in the embodiment shown in FIG. 10, or a plurality of functional modules shown in FIG. 10 are integrated into one functional module, or one functional module shown in FIG. 10 is divided into a plurality of functional modules. This is not limited in this application.

The following describes a measurement method provided in an embodiment of this application.

As shown in FIG. 11, a specific procedure of the measurement method provided in this embodiment of this application may include the following steps.

S1101: An electronic device 100 determines a first condition and a first threshold based on a performance level of a micro air pump.

For the performance level of the micro air pump and a manner of determining the performance level of the micro air pump, refer to the related descriptions in the embodiment shown in FIG. 3.

The first condition may be the stop condition in step S407 shown in FIG. 4A. The first threshold may be the wearing tightness threshold in the embodiment shown in FIG. 4A to FIG. 4C.

When the first condition is that time for inflating an airbag by the micro air pump reaches fifth duration, the first threshold may include a fifth air pressure value. Optionally, in this case, the first threshold may further include a sixth air pressure value. The fifth duration may be the preset duration Ts in the embodiment shown in FIG. 4A to FIG. 4C, the fifth air pressure value may be the air pressure threshold Y in the embodiment shown in FIG. 4A to FIG. 4C, and the sixth air pressure value may be the air pressure threshold YY in the embodiment shown in FIG. 4A to FIG. 4C.

When the first condition is that air pressure of an airbag reaches a seventh air pressure value, the first threshold may include sixth duration. Optionally, in this case, the first threshold may further include seventh duration. The seventh air pressure value may be the preset air pressure SS in the embodiment shown in FIG. 4A to FIG. 4C, the sixth duration may be the time threshold T in the embodiment shown in FIG. 4A to FIG. 4C, and the seventh duration may be the time threshold TT in the embodiment shown in FIG. 4A to FIG. 4C.

S1102: The electronic device 100 controls the micro air pump to inflate the airbag.

Before step S1102 is performed, the electronic device 100 may determine whether a user wears the electronic device 100. For a specific determining manner, refer to the related content of step S402 shown in FIG. 4A.

S1103: When the first condition is satisfied, the electronic device 100 determines a first parameter.

When the first condition is that the time for inflating the airbag by the micro air pump reaches the fifth duration, the first parameter may be air pressure of the airbag when the first condition is satisfied. For example, the first parameter may be the air pressure S1 of the airbag in the embodiment shown in FIG. 4A to FIG. 4C.

When the first condition is that the air pressure of the airbag reaches the seventh air pressure value, the first parameter may be duration of inflating the airbag by the micro air pump when the first condition is satisfied. For example, the first parameter may be the time t for inflating the airbag by the micro air pump in the embodiment shown in FIG. 4A to FIG. 4C.

Other specific content of the first parameter may be the pre-inflation data in step S407 shown in FIG. 4A.

S1104: Compare the first parameter with the first threshold, and determine wearing tightness based on a comparison result.

When the first threshold includes only one threshold (for example, includes only the fifth air pressure value, or includes only the sixth duration), the electronic device 100 may determine, based on the first parameter and the first threshold, whether the wearing of the user is excessively loose.

When the first threshold includes a plurality of sub-thresholds (for example, includes the fifth air pressure value and the sixth air pressure value, or includes the sixth duration and the seventh duration), the electronic device 100 may determine the wearing tightness of the user based on the first parameter and the first threshold. The wearing tightness includes excessively loose wearing, slightly loose wearing, proper wearing, and the like.

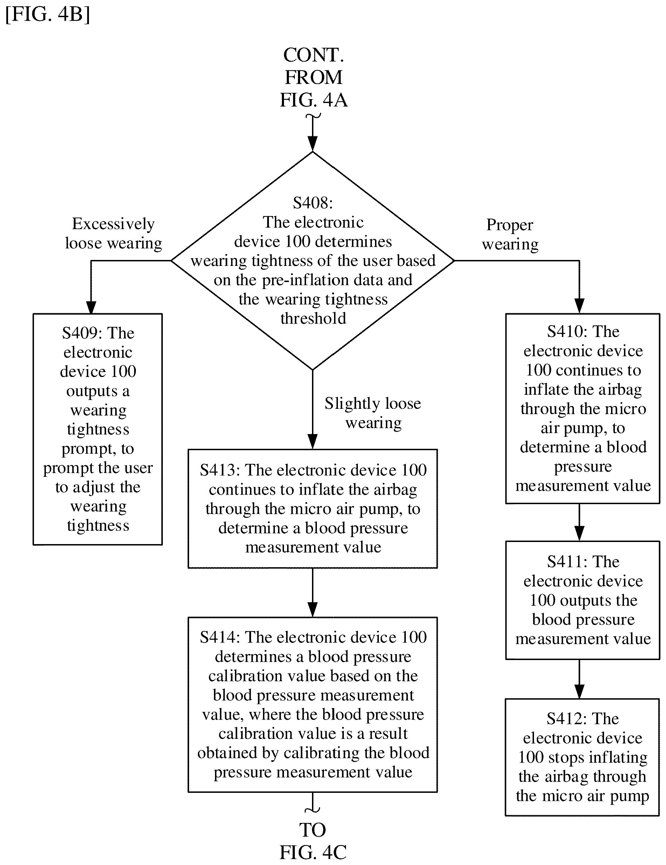

For a specific determining manner and other content of step S1104, refer to the related content of step S408 shown in FIG. 4B.

In this way, the wearing tightness of the user can be determined in a physiological parameter measurement, to avoid an error caused by the wearing tightness in the physiological parameter measurement process. In addition, performance of the micro air pump is also considered in the wearing tightness measurement of the user, so that a measurement error caused by performance deterioration of the micro air pump can be avoided.

In some embodiments, the electronic device 100 may alternatively determine the wearing tightness of the user by combining the comparison result with the wearing tightness determining manner in any one of the embodiments in FIG. 7, FIG. 9A, and FIG. 9B. When a result of determining the wearing tightness of the user in any one of the foregoing manners is excessively loose wearing, the electronic device 100 determines that the wearing of the user is excessively loose. When no result of determining the wearing tightness of the user in the foregoing manners is excessively loose wearing, the electronic device 100 may determine to continue the physiological parameter measurement. For a specific manner, refer to the related descriptions in the embodiments shown in FIG. 7 and FIG. 9B. Details are not described herein again.

In this way, the wearing tightness of the user may be determined with reference to inflation data of the micro air pump for the airbag, monitoring data of a PPG module, and monitoring data of a flow monitoring sensor, to improve measurement accuracy.

This application provides a measurement method. The electronic device 100 may obtain an environment parameter, and when starting wearing tightness detection, the electronic device 100 may determine an operating parameter, a stop condition, and a wearing tightness threshold based on the environment parameter. The electronic device 100 inflates the airbag based on the operating parameter through the micro air pump, to obtain pre-inflation data, where the pre-inflation data includes air pressure of the airbag and inflation time in an inflation process. When detecting that the stop condition is satisfied, the electronic device 100 may determine wearing tightness based on the pre-inflation data and the wearing tightness threshold. The environment parameter may include but is not limited to any one or more of the following: the performance level of the micro air pump, a measurement posture, a model of the airbag, and the like.

In this way, the electronic device 100 may determine wearing tightness of the user in different scenarios, and may further prompt the user to adjust the wearing tightness, to avoid an error caused by different wearing tightness when another physiological parameter (for example, blood pressure) is measured.

The following describes another connection relationship of hardware related to the wearing tightness measurement in the electronic device 100 provided in embodiments of this application.

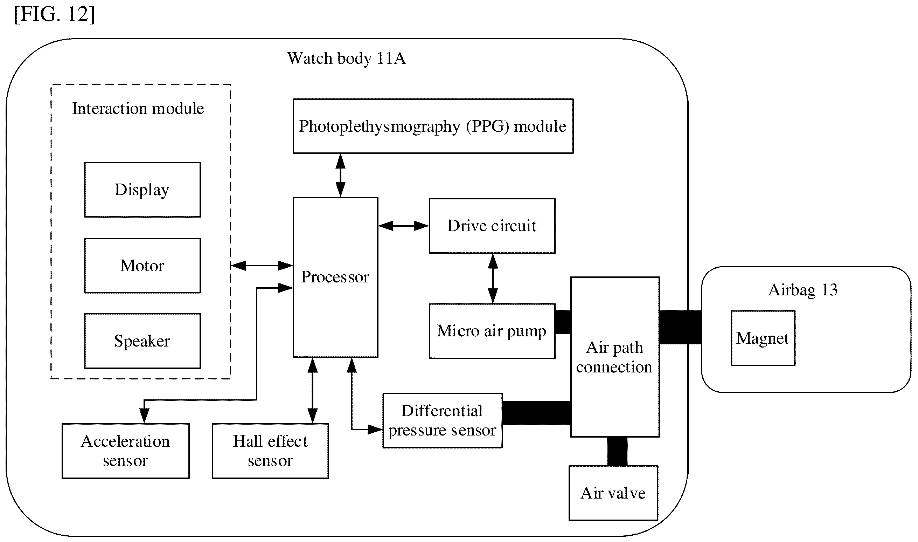

FIG. 12 is a diagram of the connection relationship of the hardware related to the wearing tightness measurement according to an embodiment of this application.

In this embodiment of this application, the watch body 11 shown in FIG. 1A may be a watch body 11A shown in FIG. 12. As shown in FIG. 12, the watch body 11A may include a processor, a drive circuit, a micro air pump, a differential pressure sensor, an air path connection, and the like. In some embodiments, the watch body 11A may further include any one or more of a photoplethysmography (photoplethysmography, PPG) module, an acceleration sensor, a display, a motor, a speaker, a Hall effect sensor, and an air valve. An airbag 13 may be connected to the micro air pump in the watch body 11 through the air path connection. Optionally, a magnet may be disposed in the airbag 13, and the magnet may be disposed on a side that is of the airbag 13 and that is close to the watch body 11.

For specific function descriptions of components such as the processor, the drive circuit, the micro air pump, the air path connection, the PPG module, the Hall effect sensor, and the air valve, refer to the related descriptions in the embodiment shown in FIG. 1C. Details are not described herein again. In addition, the differential pressure sensor may be one of the air pressure sensors shown in FIG. 1C, and is configured to measure the air pressure of the airbag.

The display may be the display 194 in the embodiment shown in FIG. 1B. Any one or more of the display, the motor, and the speaker may form an interaction module. The interaction module may be configured to output a measurement result of a physiological parameter 1 (for example, blood pressure), and may be further configured to output a measurement result of the wearing tightness or the like.

In some embodiments, the acceleration sensor may be configured to measure the measurement posture of the user, for example, a measurement posture of the user when the physiological parameter like the blood pressure is measured. In some other embodiments, the acceleration sensor may be configured to measure a motion status of the user (for example, determine whether the user is in a static state).

It may be understood that the embodiment shown in FIG. 12 merely illustrates some hardware. For other hardware, refer to the related content in the embodiment shown in FIG. 1B.

In some embodiments, the magnet may be disposed in the airbag 13, and the electronic device 100 may include the Hall effect sensor. The electronic device 100 may detect the magnet in the airbag 13 through the Hall effect sensor, to determine a size of the airbag.

In some application scenarios, the performance level of the micro air pump and the model of the airbag in the foregoing embodiments may also be referred to as environment parameters. It can be learned from the foregoing embodiments that both the performance level of the micro air pump and the model of the airbag affect the wearing tightness measurement. Therefore, in the embodiment shown in FIG. 11, that the electronic device 100 determines the first condition and the first threshold based on the performance level of the micro air pump may also mean that the electronic device 100 determines the first condition and the first threshold based on the environment parameter. The environment parameter may include but is not limited to any one or more of the following: the performance level of the micro air pump, the measurement posture, the model of the airbag, and the like. Specific content of the environment parameter is not limited in this application.

The following describes a procedure of a method for measuring the wearing tightness based on the environment parameter according to an embodiment of this application.

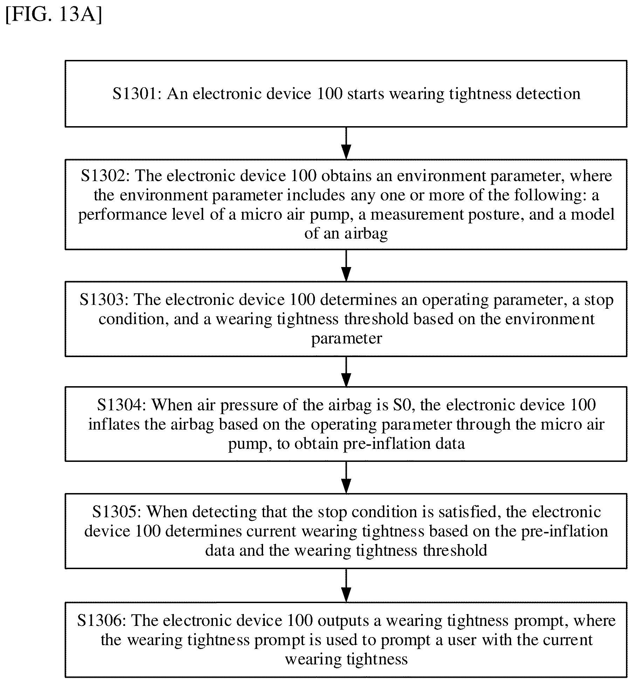

FIG. 13A is a schematic flowchart of a measurement method according to an embodiment of this application.

As shown in FIG. 13A, a specific procedure of the measurement method provided in this embodiment of this application may include the following steps.

S1301: An electronic device 100 starts wearing tightness detection.

In some embodiments, the electronic device 100 may receive and respond to an operation of starting wearing tightness detection by a user (for example, a tap operation performed by the user on a wearing tightness detection control, or an operation of starting detection of a physiological parameter like blood pressure by the user), and start wearing tightness detection.

S1302: The electronic device 100 obtains an environment parameter, where the environment parameter includes any one or more of the following: a performance level of a micro air pump, a measurement posture, and a model of an airbag.

In some embodiments, the electronic device 100 may perform step S1302 after starting the wearing tightness detection. In some other embodiments, the electronic device 100 may alternatively obtain one or more environment parameters before starting the wearing tightness detection.

The micro air pump may be classified into a plurality of levels, for example, three levels: strong (Po1), medium (Po2), and weak (Po3), based on strength of an inflation capability. It may be understood that, in some embodiments, the micro air pump may be further classified into more or fewer levels based on the inflation capability. This is not limited in this application.

The electronic device 100 may obtain the performance level of the micro air pump when detecting that a performance detection condition of the micro air pump is satisfied. The performance detection condition of the micro air pump may include but is not limited to any one or more of the following: The user wears the electronic device 100 for the first time, the electronic device 100 is restored to factory settings, duration from previous performance detection of the micro air pump reaches a preset quantity of days, and the like.

In some embodiments, the electronic device 100 may inflate the airbag based on a fixed operating parameter (for example, a voltage amplitude and a duty cycle that are provided for the micro air pump) through the micro air pump, measure inflation duration consumed for air pressure of the airbag to rise from initial air pressure to preset end air pressure, and determine the performance level of the micro air pump based on the inflation duration. When the operating parameter, the initial air pressure, and the end air pressure are determined, longer inflation duration indicates a lower performance level of the micro air pump. For example, for a specific procedure in which the electronic device 100 determines the performance level of the micro air pump in the foregoing manner, refer to the related descriptions in the embodiment shown in FIG. 3. Details are not described herein again.

In some other embodiments, the electronic device 100 may inflate the airbag based on a fixed operating parameter, fixed initial air pressure, and fixed inflation duration through the micro air pump, measure a value of the air pressure of the airbag when the preset inflation time ends, and determine the performance level of the micro air pump based on the value of the air pressure of the airbag. When the operating parameter, the initial air pressure, and the inflation time are determined, higher air pressure of the airbag when the inflation ends indicates a higher performance level of the micro air pump.

In some other embodiments, the electronic device 100 may alternatively use fixed initial air pressure and fixed end air pressure, control linear pressure rise of the micro air pump in a PID manner, measure a pressure rise duty cycle of the air pressure of the airbag that rises from the initial air pressure to the end air pressure, and determine the performance level of the micro air pump based on the pressure rise duty cycle. When the initial air pressure and the end air pressure are determined, a higher pressure rise duty cycle indicates a lower performance level of the micro air pump.

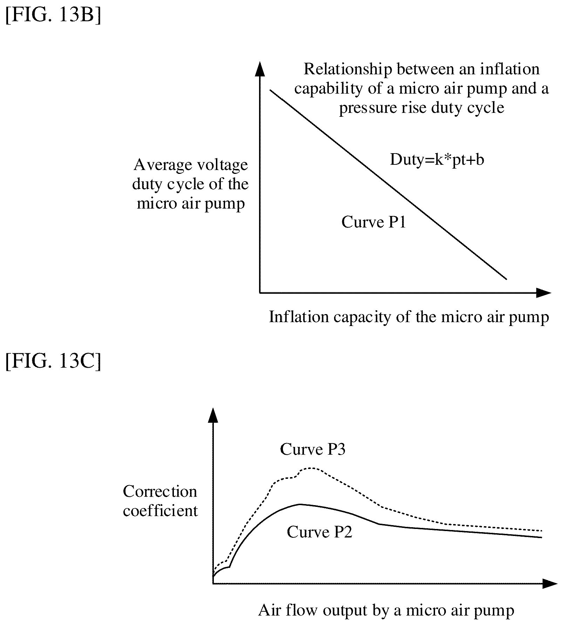

For example, FIG. 13B is a diagram of a correspondence between the inflation capability of the micro air pump and the pressure rise duty cycle according to an embodiment of this application.

As shown in FIG. 13B, in a two-dimensional coordinate system, a horizontal axis represents the inflation capability of the micro air pump, that is, an air flow output by the micro air pump in a unit time; and a vertical axis represents an average voltage duty cycle of the micro air pump in a process in which the airbag rises from the initial air pressure to the end air pressure. A curve P1 may represent a relationship between the average voltage duty cycle of the micro air pump and the inflation capability of the micro air pump in the process in which the air pressure of the airbag rises from the initial air pressure to the end air pressure. In some embodiments, a function expression of the curve P1 may be the following Formula (1): duty=k*pt+b Formula (1)

In Formula (1), duty represents the average voltage duty cycle of the micro air pump, k is a negative constant, pt represents the air flow output by the micro air pump in the unit time, and b is a normal number. It can be learned from Formula (1) that, when the initial air pressure and the end air pressure are determined, the average voltage duty cycle of the micro air pump is inversely proportional to the inflation capability of the micro air pump.

It may be understood that FIG. 13B merely shows an example of a relationship between the average voltage duty cycle of the micro air pump and the inflation capability of the micro air pump. In this embodiment of this application, the relationship between the average voltage duty cycle of the micro air pump and the inflation capability of the micro air pump may alternatively be different from that in the foregoing embodiment. This is not limited in this application.

In some embodiments, when starting the wearing tightness detection, the electronic device 100 may detect the measurement posture of the user through one or more sensors such as an acceleration sensor and a gyroscope sensor.

The electronic device 100 may store a correspondence between a plurality of measurement postures, a wearing manner of the electronic device 100, and an acceleration signal. The electronic device 100 may determine the wearing manner of the electronic device 100 based on an operation of selecting the wearing manner by the user. The wearing manner of the electronic device 100 may include: The display faces outward, the display faces inward, and the like. That the display faces outward means that when the user wears the electronic device 100, the display of the electronic device 100 is located on an outer side of a user arm. That the display faces inward means that when the user wears the electronic device 100, the display of the electronic device 100 is located on an inner side of a user arm.

For example, the following Table 4 shows a relationship between the wearing manner of the electronic device 100, a feature of the acceleration signal, and the measurement posture provided in this embodiment of this application.

**Table 4**

| Wearing manner of the electronic device 100 | Feature of an acceleration signal | Measurement posture |
|---|---|---|
| A display faces outward | Feature 1 | Posture 1 |
| A display faces inward | Feature 2 | Posture 1 |
| A display faces outward | Feature 2 | Posture 2 |
| A display faces inward | Feature 1 | Posture 2 |

As shown in Table 4, the electronic device 100 may store a correspondence between the wearing manner of the electronic device 100, the feature of the acceleration signal, and the measurement posture. For example, when the wearing manner of the electronic device 100 is that the display faces outward, and the feature of the acceleration signal includes the feature 1, the measurement posture is the posture 1; when the wearing manner of the electronic device 100 is that the display faces inward, and the feature of the acceleration signal includes the feature 2, the measurement posture is the posture 1; when the wearing manner of the electronic device 100 is that the display faces outward, and the feature of the acceleration signal includes the feature 2, the measurement posture is the posture 2; when the wearing manner of the electronic device 100 is that the display faces inward, and the feature of the acceleration signal includes the feature 1, the measurement posture is the posture 2; and the like.

It may be understood that the embodiment shown in Table 4 is merely an example. In this embodiment of this application, the electronic device 100 may store more wearing manners, features of acceleration signals, measurement postures, and the like than those in the foregoing embodiment, and correspondences between the wearing manners, the features of the acceleration signals, and the measurement postures may include more or less correspondences than that in the foregoing embodiment, or include a correspondence different from that in the foregoing embodiment. This is not limited in this application.

In a possible implementation, the electronic device 100 may collect the acceleration signal through the acceleration sensor, determine the feature of the acceleration signal, and determine the wearing manner of the electronic device 100 based on the operation of selecting the wearing manner by the user (for example, a selection operation performed by the user on an outward-facing control of the display). Then, the electronic device 100 may determine a current measurement posture based on the correspondence between the wearing manner of the electronic device 100, the feature of the acceleration signal, and the measurement posture.

In addition, in some embodiments, the electronic device 100 may further determine the measurement posture based on a correspondence between the wearing manner of the electronic device 100, a gyroscope signal, and the measurement posture. For a specific determining manner, refer to the related descriptions in the foregoing embodiment. Details are not described herein again.

It should be noted that, in some embodiments, different measurement postures may correspond to different correction coefficients. The electronic device 100 may store a correspondence between different measurement postures and correction coefficients, and the correction coefficient may be used to construct an air expansion model in subsequent embodiments. For example, FIG. 13C is a diagram of a relationship between different measurement postures and correction coefficients according to an embodiment of this application.

As shown in FIG. 13C, in a two-dimensional coordinate system, a horizontal axis may represent an air flow output by the micro air pump, a vertical axis may represent the correction coefficient, a curve P2 may represent a relationship between the air flow output by the micro air pump and the correction coefficient when the measurement posture of the user is the posture 1, and a curve P3 may represent a relationship between the air flow output by the micro air pump and the correction coefficient when the measurement posture of the user is the posture 2.

It may be understood that the embodiment shown in FIG. 13C is merely an example for description. Different measurement postures correspond to different correction coefficients. In this embodiment of this application, the electronic device 100 may alternatively store more or fewer measurement postures or store measurement postures different from those in the foregoing embodiment, and the relationship between different measurement postures and correction coefficients may include more or less than that in the foregoing embodiment, or include a relationship different from that in the foregoing embodiment. This is not limited in this application.

In some other embodiments, a plurality of measurement postures stored in the electronic device 100 may include a standard measurement posture (for example, the posture 1), and the standard measurement posture may be a posture with a minimum measurement error. For example, an arm wearing the electronic device 100 is closely attached to the chest at an angle of 45° upward, and a palm is closely attached to a heart position. The electronic device 100 may determine, based on the measured measurement posture, whether the measurement posture is the standard measurement posture. If the current measurement posture is not the standard measurement posture, the electronic device 100 may output a posture change prompt, where the posture change prompt is used to prompt the user to change the measurement posture to the standard measurement posture.

In some embodiments, the model of the airbag may include large (large, L) and small (small, S). Magnets of different polarities may be disposed in airbags of different models. Therefore, the electronic device 100 may measure a polarity of the airbag based on a Hall effect sensor, and determine the model of the airbag based on the polarity of the airbag. For a specific manner in which the electronic device 100 determines the model of the airbag based on the Hall effect sensor, refer to the related content in the embodiment shown in FIG. 1D. Details are not described herein again. It may be understood that, this embodiment herein merely describes an example in which the model of the airbag may include a plurality of models. In this embodiment of this application, the model of the airbag may alternatively include more or fewer than those in the foregoing embodiment or include a model different from those in the foregoing embodiment, for example, middle (middle, M). This is not limited in this application.

It should be noted that the electronic device 100 may measure the model of the airbag when the user wears the electronic device 100 for the first time, or may measure the model of the airbag when detecting that the user changes the airbag. This is not limited in this application.

It should be noted that, when the electronic device 100 can adapt to a plurality of airbags of different types, before detecting the performance level of the micro air pump, the electronic device 100 may first measure the model of the airbag, and then detect the performance level of the micro air pump after determining the model of the airbag.

S1303: The electronic device 100 determines the operating parameter, a stop condition, and a wearing tightness threshold based on the environment parameter.

In this embodiment of this application, the operating parameter may include a voltage (voltage amplitude) and a duty cycle. It should be noted that, in a process in which the micro air pump inflates the airbag, the operating parameter may be constant, or may regularly change. The operating parameter of the micro air pump can be used to control a rate at which the micro air pump inflates the airbag. When the duty cycle is constant, a higher voltage amplitude indicates a faster pressure rise of the airbag. When the voltage amplitude is constant, a higher duty cycle indicates a faster pressure rise of the airbag. In some other embodiments, the operating parameter of the micro air pump may further include another parameter like a frequency. This is not limited in this application.

For other specific content of the operating parameter of the micro air pump, refer to the related descriptions in step S405 shown in FIG. 4A. Details are not described herein again.

In this embodiment of this application, for the stop condition, refer to the related descriptions of the stop condition in step S405 shown in FIG. 4A. For example, the stop condition may be that time t for inflating the airbag by the micro air pump reaches preset duration Ts. In some other embodiments, the stop condition may alternatively be that air pressure S1 of the airbag reaches preset air pressure SS as the micro air pump inflates the airbag. It may be understood that the two stop conditions herein are merely some examples. In some embodiments, the stop condition may alternatively include that a voltage duty cycle of the micro air pump increases to a preset duty cycle, or the like. This is not limited herein in this application.

In this embodiment of this application, the wearing tightness threshold may include but is not limited to any one or more of the following: a time threshold, an air pressure threshold, a duty cycle threshold, a tightness coefficient threshold of the airbag expansion model, and the like. The wearing tightness threshold may include a tightness threshold 1, and optionally, may further include a tightness threshold 2. The tightness threshold 1 may be used to determine whether the wearing of the user is excessively loose, and the tightness threshold 2 may be used to determine whether the user wears the electronic device 100 properly.

In some embodiments, if the wearing tightness threshold is a time threshold, the tightness threshold 1 may be referred to as a time threshold T, and the tightness threshold 2 may be referred to as a time threshold TT. In this case, the stop condition may be that the air pressure of the airbag rises from fixed air pressure S0 to the preset air pressure SS. When the operating parameter is constant, longer inflation time required for the air pressure of the airbag to rise from the air pressure S0 pressure to the preset air pressure SS indicates looser wearing. Therefore, when the wearing tightness threshold is a time threshold, the following relationship exists between the time threshold T and the time threshold TT: T>TT.

In some embodiments, if the wearing tightness threshold is an air pressure threshold, the tightness threshold 1 may be referred to as an air pressure threshold Y, and the tightness threshold 2 may be referred to as an air pressure threshold YY. In this case, the stop condition may be that the time for inflating the airbag by the micro air pump reaches the preset duration Ts. When the operating parameter is constant, and the time for inflating the airbag by the micro air pump reaches the preset time Ts, lower air pressure of the airbag indicates looser wearing. Therefore, when the wearing tightness threshold is an air pressure threshold, the following relationship exists between the air pressure threshold Y and the air pressure threshold YY: YY>Y.

In some embodiments, if the wearing tightness threshold is a duty cycle threshold, the tightness threshold 1 may be referred to as a duty cycle threshold zth2, and the tightness threshold 2 may be referred to as a duty cycle threshold zth1. In this case, the stop condition may be that the air pressure of the airbag rises from fixed air pressure S0 to the preset air pressure SS. In a process in which the air pressure of the airbag rises from the air pressure S0 to the preset air pressure SS, a higher voltage duty cycle of the micro air pump indicates looser wearing. Therefore, when the wearing tightness threshold is a duty cycle threshold, the following relationship exists between the duty cycle threshold zth1 and the duty cycle threshold zth2: zth2>zth1.

In some embodiments, if the wearing tightness threshold is a tightness coefficient threshold of the airbag expansion model, the tightness threshold 1 may be referred to as a tightness coefficient threshold rth2, and the tightness threshold 2 may be referred to as a tightness coefficient threshold rth1. It should be noted that a tightness coefficient of the airbag expansion model may be used to represent the wearing tightness of the user. A larger tightness coefficient indicates looser wearing of the user. Therefore, when the wearing tightness threshold is the tightness coefficient threshold of the airbag expansion model, the following relationship exists between the tightness coefficient threshold rth1 and the tightness coefficient threshold rth2: rth2>rth1. The tightness coefficient of the airbag expansion model may be determined based on any one or more parameters such as the air pressure of the airbag, the voltage duty cycle of the micro air pump, a rise rate of the voltage duty cycle of the micro air pump, and inflation duration in an inflation process. In some embodiments, the stop condition may be that the time for inflating the airbag by the micro air pump reaches the preset duration Ts. In some other embodiments, the stop condition may alternatively be that the air pressure of the airbag rises from the fixed air pressure S0 to the preset air pressure SS, or the like.

It should be noted that, in some embodiments, the stop condition is related to the wearing tightness threshold. When the wearing tightness threshold is different parameters, the stop condition is accordingly different. For example, if the wearing tightness threshold is a time threshold, the stop condition may be that the air pressure of the airbag rises from the fixed air pressure S0 to the preset air pressure SS. If the wearing tightness threshold is an air pressure threshold, the stop condition may be that the time for inflating the airbag by the micro air pump reaches the preset duration Ts. If the wearing tightness threshold is a duty cycle threshold, the stop condition may be that the air pressure of the airbag rises from the fixed air pressure S0 to the preset air pressure SS. If the wearing tightness threshold is a tightness coefficient threshold of the airbag expansion model, the stop condition may be that the time for inflating the airbag by the micro air pump reaches the preset duration Ts, or the stop condition may be that the air pressure of the airbag rises from the fixed air pressure S0 to the preset air pressure SS, or the like. It may be understood that, the foregoing embodiment merely describes an example in which when the wearing tightness threshold is different parameters, stop conditions may be the same or different. In some other embodiments, the wearing tightness threshold may alternatively be a parameter different from that in the foregoing embodiment, the stop condition may alternatively include a condition different from that in the foregoing embodiment, and the relationship between the wearing tightness threshold and the stop condition may alternatively include more or less content than that in the foregoing embodiment, or include content different from that in the foregoing embodiment. This is not limited in this application.

The following describes manners of determining the operating parameter, the stop condition, and the wearing tightness threshold.

In some embodiments, the operating parameter may be preset, that is, set before the environment parameter is obtained. Specifically, the voltage amplitude provided by the electronic device 100 for the micro air pump may be preset, and the voltage amplitude may be related to a battery voltage of the electronic device 100, for example, equal to the battery voltage of the electronic device 100. The voltage duty cycle may be a preset constant value, for example, 50%, 30%, or 75%; or the duty cycle may change based on a preset start value (for example, 5%) and a preset rise rate.

In some other embodiments, the electronic device 100 determines the operating parameter based on the environment parameter and the stop condition. The electronic device 100 may store a relationship between the performance level of the micro air pump, the operating parameter, the model of the airbag, the air pressure of the airbag, and the inflation time. When the stop condition has been determined, the electronic device 100 may determine the operating parameter based on the stop condition, the model of the airbag, the performance level of the micro air pump, and the relationship between the performance level of the micro air pump, the operating parameter, the model of the airbag, the air pressure of the airbag, and the inflation time.

In some embodiments, the stop condition may be preset. In some other embodiments, the stop condition may alternatively be determined by the electronic device 100 based on the environment parameter and the operating parameter. The electronic device 100 may store a relationship between the performance level of the micro air pump, the operating parameter, the model of the airbag, the air pressure of the airbag, and the inflation time. When the operating parameter has been determined, the electronic device 100 may determine the stop condition based on the operating parameter, the model of the airbag, the performance level of the micro air pump, and the relationship between the performance level of the micro air pump, the operating parameter, the model of the airbag, the air pressure of the airbag, and the inflation time.

For example, Table 5 shows a correspondence that is between the performance level of the micro air pump, the operating parameter, the model of the airbag, the air pressure of the airbag, and the inflation time and that is stored in the electronic device 100 according to this embodiment of this application.

**Table 5**

| Operating parameter | Initial air pressure | End air pressure | Inflation time | Performance level of a micro air pump | Model of an airbag |
|---|---|---|---|---|---|
| An operating voltage is 15 V, and a duty cycle is 50% | CS0 | CS1 | [0, ct1) | Strong | L |
| An operating voltage is 15 V, and a duty cycle is 50% | CS0 | CS1 | [ct1, ct2) | Medium | L |
| An operating voltage is 15 V, and a duty cycle is 50% | CS0 | CS1 | [ct2, +∞) | Low | L |
| An operating voltage is 13 V, and a duty cycle is 30% | CS0 | CS1 | [0, ct3) | Strong | S |
| An operating voltage is 13 V, and a duty cycle is 30% | CS0 | CS1 | [ct3, ct4) | Medium | S |
| An operating voltage is 13 V, and a duty cycle is 30% | CS0 | CS1 | [ct4, +∞) | Low | S |

As shown in Table 5, the electronic device 100 may store the correspondence between the performance level of the micro air pump, the operating parameter, the model of the airbag, the air pressure of the airbag, and the inflation time. For example, when the performance level of the micro air pump is strong and the model of the airbag is L, if the operating parameter is the operating voltage 15 V and the duty cycle 50%, the inflation time consumed for the air pressure of the airbag to rise from the initial air pressure CS0 to CS1 is less than ct1. When the performance level of the micro air pump is medium, and the model of the airbag is L, if the operating parameter is the operating voltage 15 V and the duty cycle 50%, the inflation time consumed for the air pressure of the airbag to rise from the initial air pressure CS0 to CS1 is greater than or equal to ct1 and less than ct2. When the performance level of the micro air pump is weak and the model of the airbag is L, if the operating parameter is the operating voltage 15 V and the duty cycle 50%, the inflation time consumed for the air pressure of the airbag to rise from the initial air pressure CS0 to CS1 is greater than or equal to ct2. When the performance level of the micro air pump is strong and the model of the airbag is S, if the operating parameter is the operating voltage 13 V and the duty cycle 30%, the inflation time consumed for the air pressure of the airbag to rise from the initial air pressure CS0 to CS1 is less than ct3. When the performance level of the micro air pump is medium and the model of the airbag is S, if the operating parameter is the operating voltage 13 V and the duty cycle 30%, the inflation time consumed for the air pressure of the airbag to rise from the initial air pressure CS0 to CS1 is greater than or equal to ct3 and less than ct4. When the performance level of the micro air pump is weak and the model of the airbag is S, if the operating parameter is the operating voltage 13 V and the duty cycle 30%, the inflation time consumed for the air pressure of the airbag to rise from the initial air pressure CS0 to CS1 is greater than or equal to ct4.

It may be understood that Table 5 is merely an example. In this embodiment of this application, the electronic device 100 may alternatively store more or less content than that in Table 5, or store content different from that in Table 5. This is not limited in this application.

After determining the environment parameter, the operating parameter, and the stop condition, the electronic device 100 may determine the wearing tightness threshold based on the environment parameter, the operating parameter, and the stop condition. In some embodiments, the electronic device 100 may store correspondences between different environment parameters, operating parameters, stop conditions, and wearing tightness thresholds. The electronic device 100 may determine the wearing tightness threshold from the correspondence based on the environment parameter, the operating parameter, and the stop condition. It may be understood that this embodiment herein merely describes an example of a manner of determining the wearing tightness threshold. In this embodiment of this application, the electronic device 100 may alternatively determine the wearing tightness threshold in another manner. This is not limited in this application.

S1304: When the air pressure of the airbag is S0, the electronic device 100 inflates the airbag based on the operating parameter through the micro air pump, to obtain pre-inflation data.

The air pressure value S0 may be preset initial air pressure. For example, the air pressure value S0 may be 0. Before supplying power to the micro air pump, the electronic device 100 may determine the air pressure of the airbag through an air pressure sensor (for example, a differential pressure sensor). When the air pressure of the airbag is S0, the electronic device 100 may inflate the airbag based on the operating parameter through the micro air pump. When the air pressure of the airbag is not S0, the electronic device 100 may control the airbag to release air, or control the micro air pump to inflate the airbag, to adjust the air pressure of the airbag to S0. In this way, it can be ensured that the initial air pressure of the airbag is the preset value S0, to avoid an error caused by different initial air pressure.

In some embodiments, the pre-inflation data includes the air pressure of the airbag and the inflation time in the inflation process. In some other embodiments, the pre-inflation data may further include the voltage amplitude and/or the voltage duty cycle in the inflation process.

S1305: When detecting that the stop condition is satisfied, the electronic device 100 determines current wearing tightness based on the pre-inflation data and the wearing tightness threshold.

The wearing tightness may include proper wearing, slightly loose wearing (also referred to as little loose wearing), excessively loose wearing, and the like in an order from tight to loose. In some other embodiments, the wearing tightness may alternatively include more or fewer degrees than those in the foregoing embodiment, or include a degree different from those in the foregoing embodiment. This is not limited in this application.

In some embodiments, the wearing tightness threshold may be a time threshold. In this case, the electronic device 100 may determine the wearing tightness based on the inflation time in the pre-inflation data and the time threshold.

In some embodiments, the wearing tightness threshold may be an air pressure threshold. In this case, the electronic device 100 may determine the wearing tightness based on the air pressure of the airbag in the pre-inflation data and the air pressure threshold.

In some embodiments, the wearing tightness threshold may be a duty cycle threshold. In this case, the electronic device 100 may determine the wearing tightness based on the voltage duty cycle in the pre-inflation data and the duty cycle threshold.

In some embodiments, the wearing tightness threshold may be a tightness coefficient threshold. In this case, the electronic device 100 may determine a tightness coefficient based on one or more parameters such as the air pressure of the airbag, the inflation time, and the duty cycle in the pre-inflation data, and determine the wearing tightness based on the tightness coefficient and the tightness coefficient threshold. For a specific calculation manner of the tightness coefficient, refer to the related descriptions in the embodiment shown in FIG. 5. Details are not described herein.

It may be understood that the foregoing embodiment is merely an example for description. When the wearing tightness threshold is different parameters, the electronic device 100 may determine the wearing tightness based on different parameters in the pre-inflation data and the wearing tightness threshold. In this embodiment of this application, the wearing tightness threshold may alternatively be a parameter different from that in the foregoing embodiment. This is not limited in this application.

For example, for a specific procedure in which the electronic device 100 determines the current wearing tightness based on the pre-inflation data and the wearing tightness threshold, refer to the related content in the embodiment shown in FIG. 4A to FIG. 4C or FIG. 5. Details are not described herein in this application.

S1306: The electronic device 100 outputs a wearing tightness prompt, where the wearing tightness prompt is used to prompt the user with the current wearing tightness.

Step S1306 is an optional step.

In some embodiments, when determining that the current wearing tightness is excessively loose wearing, the electronic device 100 may output an excessively-loose-wearing prompt, where the excessively-loose-wearing prompt is used to prompt the user that the electronic device 100 is worn excessively loosely currently.

In some embodiments, when determining that the current wearing tightness is slightly loose wearing, the electronic device 100 may output a slightly-loose-wearing prompt, where the slightly-loose-wearing prompt is used to prompt the user that the electronic device 100 is worn slightly loosely currently.

In some embodiments, when determining that the current wearing tightness is proper wearing, the electronic device 100 may output a proper-wearing prompt, where the proper-wearing prompt is used to prompt the user that the electronic device 100 is worn properly currently. In some other embodiments, when determining that the current wearing tightness is proper wearing, the electronic device 100 may not output the wearing tightness prompt.

It may be understood that the excessively-loose-wearing prompt, the slightly-loose-wearing prompt, and the proper-wearing prompt are all wearing tightness prompts.

It should be noted that the electronic device 100 may output the wearing tightness prompt in any one or more manners such as displaying on a display, audio broadcast, vibration, and indicator blinking. A specific output manner of the wearing tightness prompt is not limited in this application.

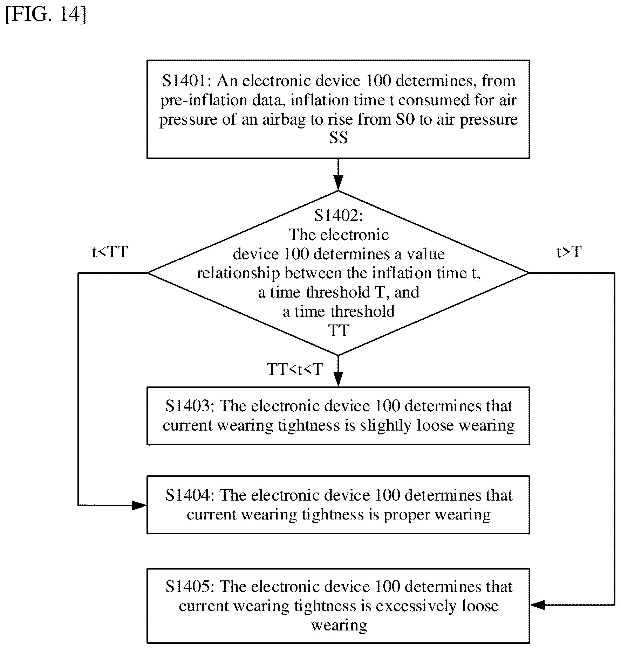

FIG. 14 is a schematic flowchart of determining, by the electronic device 100, the current wearing tightness based on the pre-inflation data and the wearing tightness threshold according to an embodiment of this application.

For example, as shown in FIG. 14, the stop condition is that the air pressure of the airbag rises from the air pressure S0 to the air pressure SS, the micro air pump uses a constant operating parameter, and the wearing tightness threshold is a time threshold. A specific procedure in which the electronic device 100 determines the current wearing tightness based on the pre-inflation data and the wearing tightness threshold may include the following steps.

S1401: The electronic device 100 determines, from the pre-inflation data, the inflation time t consumed for the air pressure of the airbag to rise from S0 to the air pressure SS.

S1402: The electronic device 100 determines a value relationship between the inflation time t, the time threshold TT, and the time threshold T.

When t<TT, the electronic device 100 performs the following step S1404.

When TT<t<T, the electronic device 100 performs the following step S1403.

When t>T, the electronic device 100 performs the following step S1405.

S1403: The electronic device 100 determines that the current wearing tightness is slightly loose wearing.

S1404: The electronic device 100 determines that the current wearing tightness is proper wearing.

S1405: The electronic device 100 determines that the current wearing tightness is excessively loose wearing.

According to the measurement method provided in this embodiment of this application, the electronic device 100 may determine the current wearing tightness of the user.

It may be understood that the embodiment shown in FIG. 14 is merely an example. In this embodiment of this application, the wearing tightness threshold may alternatively be another parameter like an air pressure threshold or a duty cycle threshold. In this case, the electronic device 100 may alternatively compare a corresponding parameter in the pre-inflation data with the wearing tightness threshold, and determine the wearing tightness based on a comparison result. For a specific manner, refer to the embodiment shown in FIG. 14. This is not limited in this application.

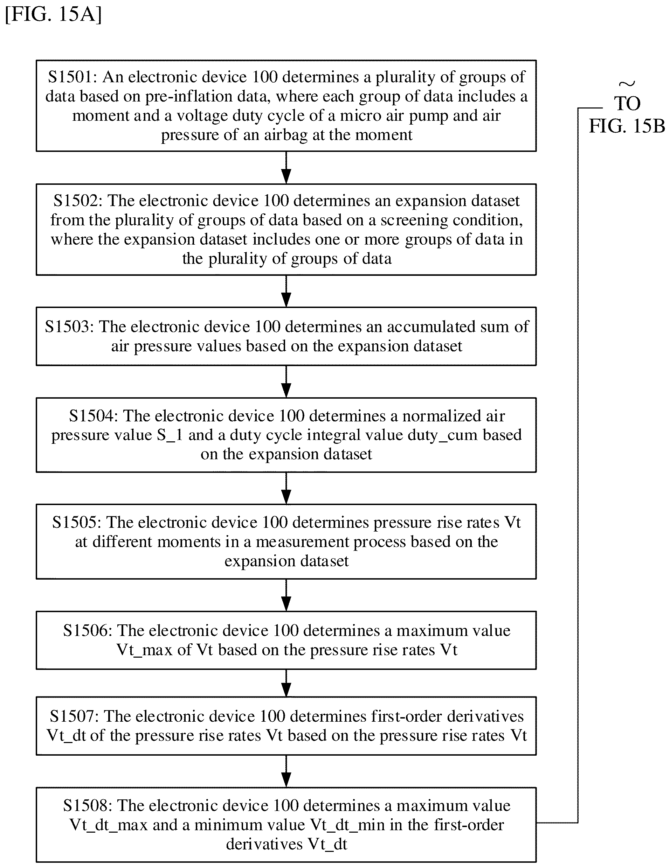

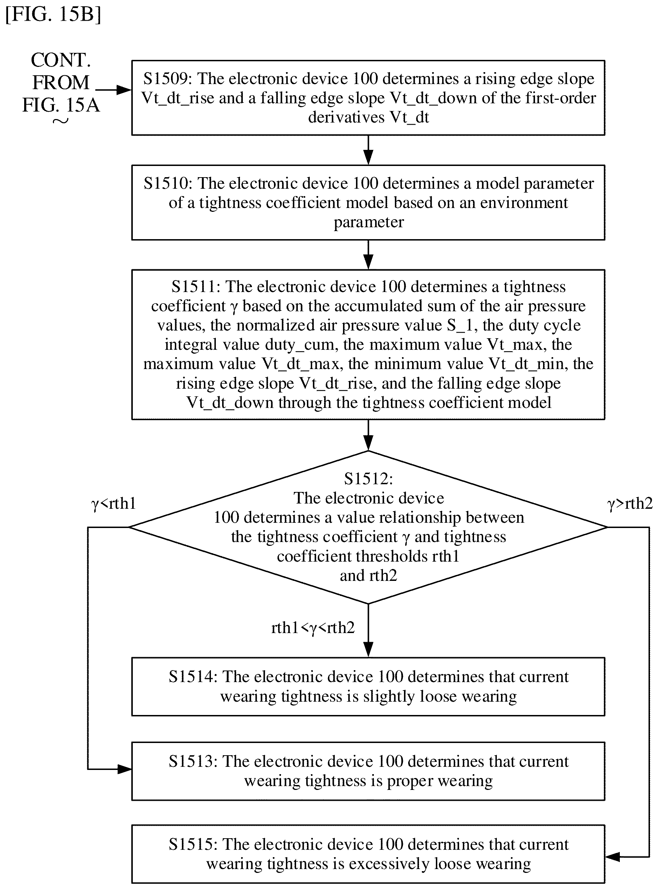

FIG. 15A and FIG. 15B are another schematic flowchart of determining, by the electronic device 100, the current wearing tightness based on the pre-inflation data and the wearing tightness threshold according to an embodiment of this application.

For example, as shown in FIG. 15A and FIG. 15B, the stop condition is that the air pressure of the airbag rises from the air pressure S0 to the air pressure SS, and the wearing tightness threshold is a tightness coefficient threshold of the airbag expansion model. A specific procedure in which the electronic device 100 determines the current wearing tightness based on the pre-inflation data and the wearing tightness threshold may include the following steps.

S1501: The electronic device 100 determines a plurality of groups of data based on the pre-inflation data, where each group of data includes a moment and a voltage duty cycle of the micro air pump and air pressure of the airbag at the moment.

In some embodiments, the pre-inflation data may include operating parameters (namely, voltage amplitudes and voltage duty cycles) and air pressure of the airbag at different moments in the inflation process. Therefore, after obtaining the pre-inflation data, the electronic device 100 may determine the plurality of groups of data based on the pre-inflation data. Each group of data includes the moment, a duty cycle, and an air pressure value, and each group of data may represent the voltage duty cycle and the air pressure of the airbag at the moment.

S1502: The electronic device 100 determines an expansion dataset from the plurality of groups of data based on a screening condition, where the expansion dataset includes one or more groups of data in the plurality of groups of data.

In some embodiments, the screening condition may be a limitation on values of the duty cycle and the air pressure value of the airbag. For example, the screening condition may include that the duty cycle is less than 50% and the air pressure of the airbag is greater than 5 millimeters of mercury.

S1503: The electronic device 100 determines an accumulated sum of air pressure values based on the expansion dataset.

There may be one or more accumulated sums of air pressure values.

In some embodiments, the electronic device 100 may perform, based on a preset air pressure value limiting condition (for example, an air pressure value is smaller than 30 millimeters of mercury), accumulation calculation on air pressure values that satisfy the air pressure value limiting condition in the expansion dataset, to obtain an accumulated sum.

In some other embodiments, a plurality of different air pressure value limiting conditions (for example, an air pressure value is smaller than 30 millimeters of mercury and an air pressure value is smaller than 60 millimeters of mercury) may be set for the electronic device 100. The electronic device 100 may separately perform, based on the plurality of air pressure value limiting conditions, accumulation calculation on data that satisfies different air pressure value limiting conditions in the expansion dataset, to obtain a plurality of accumulated sums.

S1504: The electronic device 100 determines a normalized air pressure value S_1 and a duty cycle integral value duty_cum based on the expansion dataset.

The electronic device 100 may perform normalized calculation on the air pressure value in the expansion dataset, to obtain the normalized air pressure value S_1.

The electronic device 100 may further perform integral calculation on the duty cycle in the expansion dataset, to obtain the duty cycle integral value duty_cum.

S1505: The electronic device 100 determines pressure rise rates Vt at different moments in the measurement process based on the expansion dataset.

S1506: The electronic device 100 determines a maximum value Vt_max of Vt based on the pressure rise rates Vt.

S1507: The electronic device 100 determines first-order derivatives Vt_dt of the pressure rise rates Vt based on the pressure rise rates Vt.

S1508: The electronic device 100 determines a maximum value Vt_dt_max and a minimum value Vt_dt_min in the first-order derivatives Vt_dt.

S1509: The electronic device 100 determines a rising edge slope Vt_dt_rise and a falling edge slope Vt_dt_down of the first-order derivatives Vt_dt.

S1510: The electronic device 100 determines a model parameter of a tightness coefficient model based on the environment parameter.

It should be noted that the determining in step S1510 may be performed after the environment parameter of the electronic device 100 is obtained. In some embodiments, step S1510 may be performed before step S1501 is performed, or may be performed after step S1501 is performed. An execution sequence number of step S1510 is not limited in this application.

The model parameter may include but is not limited to any one or more of the following: an airbag correction coefficient, a measurement posture correction coefficient, a micro air pump performance correction coefficient, and the like. The electronic device 100 may determine the airbag correction coefficient based on the model of the airbag, determine the measurement posture correction coefficient based on the measurement posture, and determine the micro air pump performance correction coefficient based on the performance level of the micro air pump.

S1511: The electronic device 100 determines a tightness coefficient γ based on the accumulated sum of the air pressure values, the normalized air pressure value S_1, the duty cycle integral value duty_cum, the maximum value Vt_max, the maximum value Vt_dt_max, the minimum value Vt_dt_min, the rising edge slope Vt_dt_rise, and the falling edge slope Vt_dt_down through the tightness coefficient model.

In some embodiments, the electronic device 100 may store one or more tightness coefficient models. An input of the tightness coefficient model may include but is not limited to any one or more of the following parameters: the accumulated sum of the air pressure values, the normalized air pressure value S_1, the duty cycle integral value duty_cum, the maximum value Vt_max, the maximum value Vt_dt_max, the minimum value Vt_dt_min, the rising edge slope Vt_dt_rise, the falling edge slope Vt_dt_down, and the like. An output of the tightness coefficient model may be the tightness coefficient γ.

S1512: The electronic device 100 determines a value relationship between the tightness coefficient γ and the tightness coefficient thresholds rth1 and rth2.

When γ<rth1, the electronic device 100 may perform the following step S1513.

When rth1<γ<rth2, the electronic device 100 may perform the following step S1514.

When γ>rth2, the electronic device 100 may perform the following step S1515.

S1513: The electronic device 100 determines that the current wearing tightness is proper wearing.

S1514: The electronic device 100 determines that the current wearing tightness is slightly loose wearing.

S1515: The electronic device 100 determines that the current wearing tightness is excessively loose wearing.

It may be understood that the embodiment shown in FIG. 15A and FIG. 15B merely describes an example of a method for calculating the tightness coefficient based on the airbag expansion model and determining the wearing tightness of the user based on the tightness coefficient. In this embodiment of this application, the electronic device 100 may alternatively determine the tightness coefficient in a manner different from that in the foregoing embodiment, and determine the wearing tightness of the user. This is not limited in this application.

In some application scenarios, a difference between a parameter in the pre-inflation data and the wearing tightness threshold is small, and the electronic device 100 cannot accurately determine the current wearing tightness of the user based on the pre-inflation data and the wearing tightness threshold. In this case, the electronic device 100 may determine the tightness coefficient of the airbag expansion model based on the pre-inflation data, and determine the current wearing tightness of the user based on the tightness coefficient and the tightness coefficient threshold.

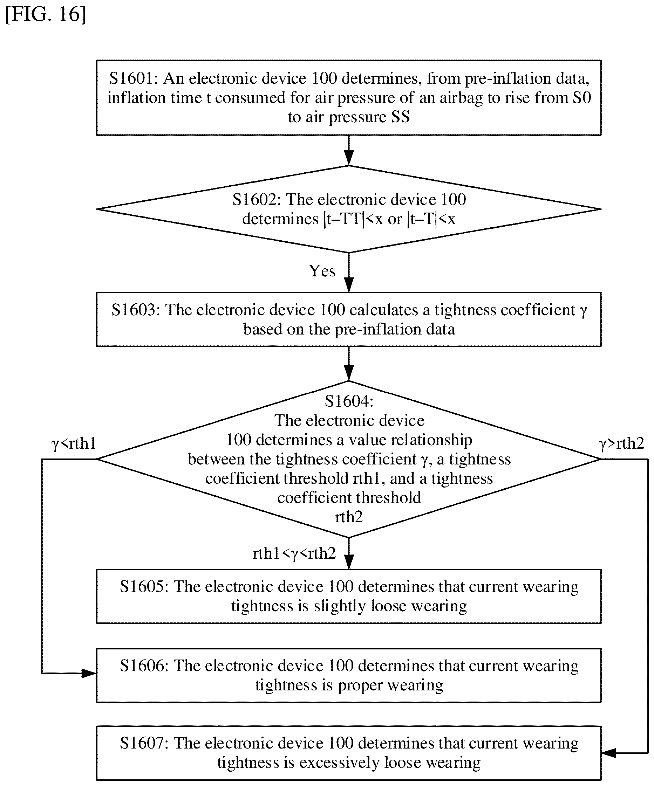

FIG. 16 is another schematic flowchart of determining, by the electronic device 100, the current wearing tightness based on the pre-inflation data and the wearing tightness threshold according to an embodiment of this application.

For example, as shown in FIG. 16, the stop condition is that the air pressure of the airbag rises from the air pressure S0 to the air pressure SS, the micro air pump uses a constant operating parameter, and the wearing tightness threshold is a time threshold. A specific procedure in which the electronic device 100 determines the current wearing tightness based on the pre-inflation data and the wearing tightness threshold may include the following steps.

S1601: The electronic device 100 determines, from the pre-inflation data, the inflation time t consumed for the air pressure of the airbag to rise from S0 to the air pressure SS.

S1602: The electronic device 100 determines |t-TT|<x or |t-T|<x.

If |t-TT|<x, or |t-T|<x, the electronic device 100 may perform the following step S1603 and subsequent steps.

If |t-TT|≥x, and |t-T|≥x, the electronic device 100 may determine the current wearing tightness of the user in the manner in the embodiment shown in FIG. 14. For details, refer to the related content of step S1402 to step S1405 shown in FIG. 14.

S1603: The electronic device 100 calculates a tightness coefficient γ based on the pre-inflation data.

For a specific calculation manner of the tightness coefficient γ, refer to the related content in the embodiment shown in FIG. 15A and FIG. 15B. Details are not described herein again in this application.

S1604: The electronic device 100 determines a value relationship between the tightness coefficient γ, the tightness coefficient threshold rth1, and the tightness coefficient threshold rth2.

When γ<rth1, the electronic device 100 performs the following step S1606.

When rth1<γ<rth2, the electronic device 100 performs the following step S1605.

When γ>rth2, the electronic device 100 performs the following step S1607.

S1605: The electronic device 100 determines that the current wearing tightness is slightly loose wearing.

S1606: The electronic device 100 determines that the current wearing tightness is proper wearing.

S1607: The electronic device 100 determines that the current wearing tightness is excessively loose wearing.

According to the measurement method provided in this embodiment of this application, when the current wearing tightness of the user cannot be determined based on a single parameter, the electronic device 100 may determine the current wearing tightness of the user based on the tightness coefficient of the airbag expansion model.

It may be understood that the embodiment shown in FIG. 16 is merely an example. In this embodiment of this application, the wearing tightness threshold may alternatively be another parameter like an air pressure threshold or a duty cycle threshold. In this case, the electronic device 100 may alternatively determine, based on a difference between a corresponding parameter in the pre-inflation data and the wearing tightness threshold, whether the electronic device 100 can determine the current wearing tightness based on the single parameter. When it is determined that the current wearing tightness cannot be determined based on the single parameter, the electronic device 100 may determine the current wearing tightness of the user by using the tightness coefficient of the airbag expansion model. For a specific manner, refer to the embodiment shown in FIG. 16. This is not limited in this application.

In some application scenarios, when measuring the physiological parameter 1 (for example, the blood pressure), the electronic device 100 may first measure the current wearing tightness of the user, then determine, based on the current wearing tightness, whether to prompt the user to adjust the wearing tightness, and may further calibrate a measurement value of the physiological parameter 1 based on the current wearing tightness.

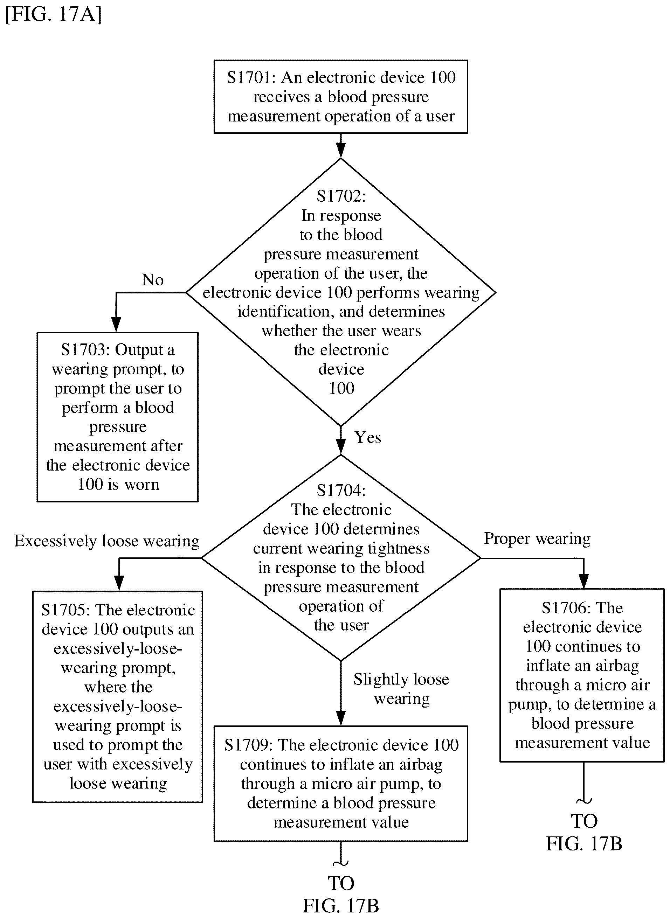

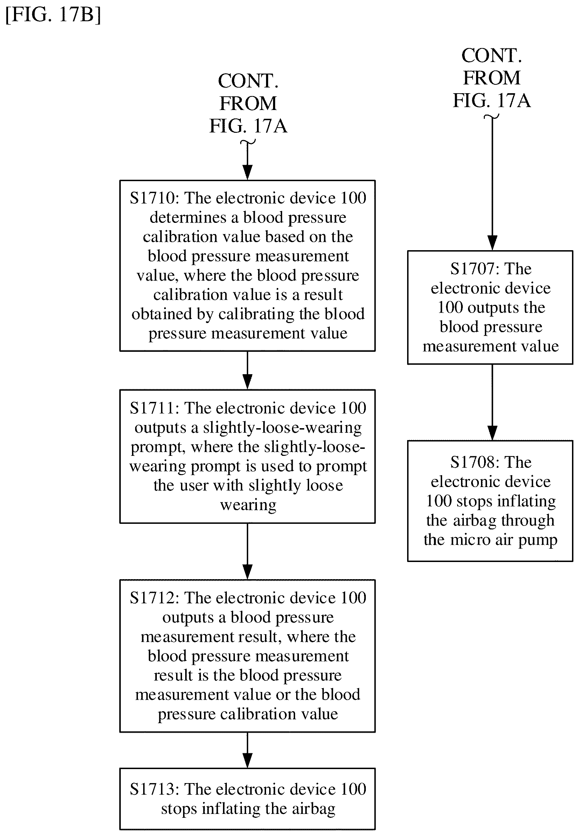

FIG. 17A and FIG. 17B are a schematic flowchart of measuring the physiological parameter 1 (for example, the blood pressure) by the electronic device 100 according to an embodiment of this application.

For example, the physiological parameter 1 is the blood pressure. As shown in FIG. 17A and FIG. 17B, a specific procedure of measuring the blood pressure by the electronic device 100 may include the following steps.

S1701: The electronic device 100 receives a blood pressure measurement operation of the user.

For example, the blood pressure measurement operation of the user may be a tap operation on the measurement control 212 on the blood pressure measurement interface 210 shown in FIG. 2B.

S1702: In response to the blood pressure measurement operation of the user, the electronic device 100 performs wearing identification, and determines whether the user wears the electronic device 100.

For a manner in which the electronic device 100 determines whether the user wears the electronic device 100, refer to the related content of step S301 shown in FIG. 3. Details are not described herein again.

If the electronic device 100 determines that the user wears the electronic device 100, the electronic device 100 may perform the following step S1704.

If the electronic device 100 determines that the user does not wear the electronic device 100, the electronic device 100 performs the following step S1703.

S1703: Output a wearing prompt, to prompt the user to perform a blood pressure measurement after the electronic device 100 is worn.

When it is determined that the user does not wear the electronic device 100, the electronic device 100 may output the wearing prompt, where the wearing prompt is used to prompt the user to perform a blood pressure measurement after the electronic device 100 is worn.

Step S1702 and step S1703 are optional steps. In some embodiments, the electronic device 100 may alternatively perform step S1704 after performing step S1701.

S1704: The electronic device 100 determines the current wearing tightness in response to the blood pressure measurement operation of the user.

In some embodiments, the wearing tightness may include excessively loose wearing, slightly loose wearing, proper wearing, and the like.

For a specific procedure in which the electronic device 100 determines the current wearing tightness, refer to the related content in the embodiment shown in FIG. 13A. Details are not described herein again.

If the wearing tightness is excessively loose wearing, the electronic device 100 may perform the following step S1705.

If the wearing tightness is proper wearing, the electronic device 100 may perform the following step S1706 to step S1708.

If the wearing tightness is slightly loose wearing, the electronic device 100 may perform the following step S1709 to step S1713.

S1705: The electronic device 100 outputs an excessively-loose-wearing prompt, where the excessively-loose-wearing prompt is used to prompt the user with excessively loose wearing.

When the electronic device 100 determines that the wearing of the user is excessively loose, the electronic device 100 may output the excessively-loose-wearing prompt. The excessively-loose-wearing prompt may be used to prompt the user that the electronic device 100 is worn excessively loosely currently, and may be further used to prompt to adjust the wearing tightness. The excessively-loose-wearing prompt may be any one or more of the following forms: an animation prompt, a picture prompt, a voice prompt, a text prompt, and the like.

In some embodiments, after the user adjusts the wearing tightness, the electronic device 100 may release air in the airbag (or remind the user to release air in the airbag), until the air pressure of the airbag is restored to the preset air pressure S0. Then, the electronic device 100 may receive and respond to the blood pressure measurement operation of the user, and re-perform step S1704.

S1706: The electronic device 100 continues to inflate the airbag through the micro air pump, to determine a blood pressure measurement value.

The blood pressure measurement value includes systolic pressure (namely, high pressure) and diastolic pressure (namely, low pressure). In some embodiments, in the process in which the electronic device 100 inflates the airbag through the micro air pump, the electronic device 100 may obtain a pulse wave signal of the user through parsing based on measurement data of the air pressure sensor. In some other embodiments, the electronic device 100 may alternatively obtain a pulse wave signal of the user through parsing based on a PPG signal collected by a PPG module. The electronic device 100 may determine the blood pressure measurement value when detecting that the pulse wave signal satisfies a value output condition. The value output condition may include that an amplitude of the pulse wave signal reaches a product of a peak value of the pulse wave signal and a preset proportion.

For specific descriptions of the value output condition, refer to the related content in the embodiment shown in FIG. 5. Details are not described herein again.

S1707: The electronic device 100 outputs the blood pressure measurement value.

S1708: The electronic device 100 stops inflating the airbag through the micro air pump.

When obtaining the blood pressure measurement value, the electronic device 100 may stop inflating the airbag. Step S1712 may be performed after step S1710 is performed.

S1709: The electronic device 100 continues to inflate the airbag through the micro air pump, to determine a blood pressure measurement value.

For specific content of step S1709, refer to the related content of step S1706.

S1710: The electronic device 100 determines a blood pressure calibration value based on the blood pressure measurement value, where the blood pressure calibration value is a result obtained by calibrating the blood pressure measurement value.

When the electronic device 100 determines that the electronic device 100 is worn slightly loosely, the electronic device 100 may calibrate the blood pressure measurement value after obtaining the blood pressure measurement value, to obtain the blood pressure calibration value.

In some embodiments, the electronic device 100 may store a calibration algorithm. The calibration algorithm may be obtained by the electronic device 100 or another electronic device through training based on a plurality of groups of measurement data. Each group of measurement data may include a performance level of the micro air pump, a size of the airbag, an operating parameter of the micro air pump, initial air pressure and end air pressure of the airbag, pre-inflation time of the airbag, a blood pressure measurement value, pre-inflation time when the electronic device 100 is worn properly, a real blood pressure value, and the like. After determining the blood pressure measurement value and the pre-inflation data, the electronic device 100 may calibrate the blood pressure measurement value based on the pre-inflation data and the tightness threshold 2 according to the calibration algorithm, to obtain the blood pressure calibration value.

In some other embodiments, the electronic device 100 may store a correspondence between the pre-inflation data and a deviation value in different performance conditions (including the performance level of the micro air pump). After the electronic device 100 determines the pre-inflation data and the blood pressure measurement value, the electronic device 100 may determine the corresponding deviation value from the correspondence between the pre-inflation data and the deviation value based on the pre-inflation data, and calibrate the blood pressure measurement value based on the deviation value, to obtain the blood pressure calibration value.

S1711: The electronic device 100 outputs a slightly-loose-wearing prompt, where the slightly-loose-wearing prompt is used to prompt the user with slightly loose wearing.

Step S1710 and step S1711 are both optional steps. After performing step S1709, the electronic device 100 may perform either one or more of step S1710 and step S1711, or may perform the following step S1712 after performing step S1709.

S1712: The electronic device 100 outputs a blood pressure measurement result, where the blood pressure measurement result is the blood pressure measurement value or the blood pressure calibration value.

When it is determined to calibrate the blood pressure measurement value, the electronic device 100 may use the blood pressure calibration value as the blood pressure measurement result for output. When it is determined not to calibrate the blood pressure measurement value, the electronic device 100 may use the blood pressure measurement value as the blood pressure measurement result for output.

S1713: The electronic device 100 stops inflating the airbag.

When obtaining the blood pressure measurement value, the electronic device 100 may stop inflating the airbag. In some embodiments, step S1713 may be performed after step S1709 is performed. In some other embodiments, step S1713 may alternatively be performed after step S1712.

According to the blood pressure measurement method provided in this embodiment of this application, the operating parameter of the micro air pump and the wearing tightness threshold may be set based on the performance level of the micro air pump, to avoid a measurement error caused by excessively loose wearing, and further avoid a measurement error caused by performance of the micro air pump or performance of the airbag, so as to improve accuracy of the blood pressure measurement value.

It may be understood that FIG. 17A and FIG. 17B merely describe an example of application of the measurement method provided in embodiments of this application in the blood pressure measurement scenario. In this embodiment of this application, the measured physiological parameter may alternatively be another physiological parameter like a heart rate or a body temperature. In addition, when the another physiological parameter is measured, the wearing tightness of the user may also be measured by using the measurement method provided in this embodiment of this application, and subsequent to-be-performed steps are determined based on the wearing tightness (for example, obtaining measurement data of the physiological parameter, calibrating the measurement data, outputting a wearing tightness prompt, and outputting an error prompt). For a relationship between the wearing tightness and the subsequent to-be-performed steps, refer to the related steps in the embodiment shown in FIG. 17A and FIG. 17B. Details are not described herein again.

In some application scenarios, the electronic device 100 may determine the wearing tightness of the user based on measurement data of the physiological parameter 1 in the process of measuring the physiological parameter 1 (for example, the blood pressure).

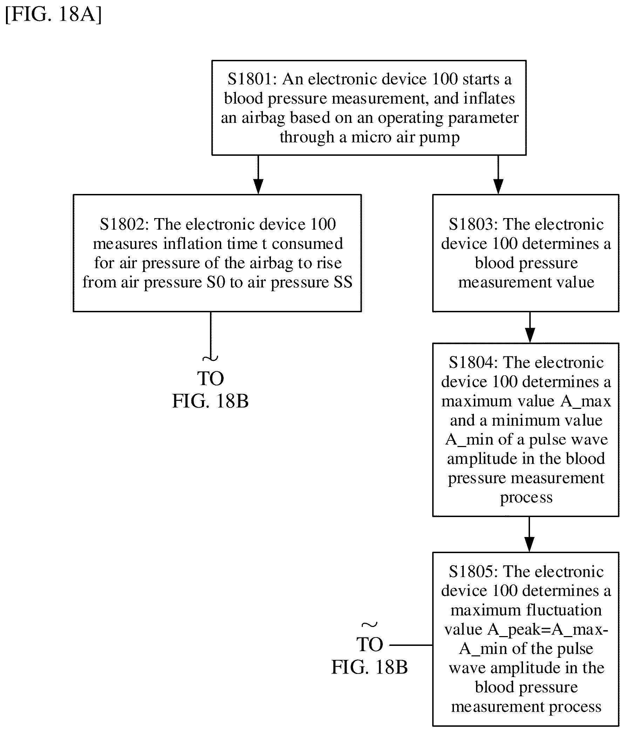

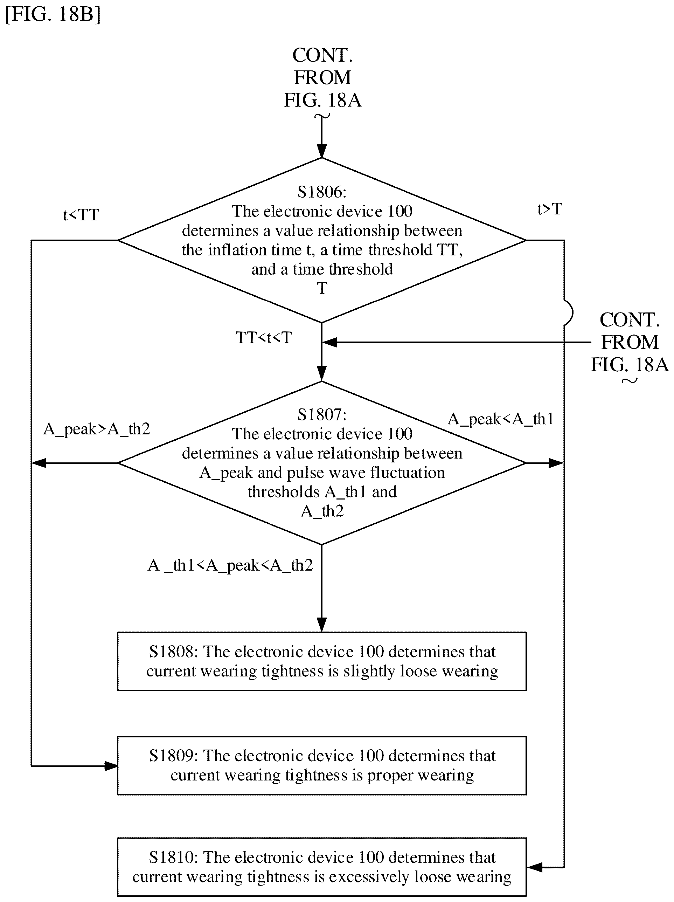

FIG. 18A and FIG. 18B are a schematic flowchart of measuring the physiological parameter 1 and the wearing tightness of the user by the electronic device 100 according to an embodiment of this application.

For example, the physiological parameter 1 is the blood pressure, the wearing tightness threshold is a time threshold, and the stop condition is that the air pressure of the airbag rises from the air pressure S0 to the air pressure SS. As shown in FIG. 18A and FIG. 18B, a specific procedure in which the electronic device 100 measures the blood pressure and the wearing tightness of the user may include the following steps.

S1801: The electronic device 100 starts a blood pressure measurement, and inflates the airbag based on the operating parameter through the micro air pump.

The electronic device 100 may start the blood pressure measurement in response to a blood pressure measurement operation of the user.

The operating parameter may be preset, or may be set based on the environment parameter. For a specific setting manner, refer to the related descriptions in the embodiment shown in FIG. 13A. Details are not described herein again. It should be noted that, the operating parameter of the micro air pump may be constant, or may change.

S1802: The electronic device 100 measures the inflation time t consumed for the air pressure of the airbag to rise from the air pressure S0 to the air pressure SS.

In the process in which the electronic device 100 inflates the airbag based on the operating parameter through the micro air pump, the electronic device 100 may measure the air pressure of the airbag through the air pressure sensor (for example, the differential pressure sensor), and determine the inflation time t consumed for the air pressure of the airbag to rise from the air pressure S0 to the air pressure SS.

It should be noted that step S1802 and the following step S1803 may be steps that are performed in parallel. An execution sequence of step S1802 and step S1803 is not limited in this application.

S1803: The electronic device 100 determines a blood pressure measurement value.

In the process in which the electronic device 100 inflates the airbag based on the operating parameter through the micro air pump, the electronic device 100 may determine the blood pressure measurement value. For a manner of determining the blood pressure measurement value by the electronic device 100, refer to the related content of step S1706 shown in FIG. 17A. Details are not described herein again.

S 1804: The electronic device 100 determines a maximum value A_max and a minimum value A_min of a pulse wave amplitude in the blood pressure measurement process.

In the blood pressure measurement process, the electronic device 100 may obtain a pulse wave signal of the user. The electronic device 100 may determine the maximum value A_max and the minimum value A_min of the pulse wave amplitude in the blood pressure measurement process based on the pulse wave signal.

S1805: The electronic device 100 determines a maximum fluctuation value A_peak=A_max-A_min of the pulse wave amplitude in the blood pressure measurement process.

S1806: The electronic device 100 determines a value relationship between the inflation time t, the time threshold TT, and the time threshold T.

Step S1806 is an optional step.

In some embodiments, after determining the inflation time t consumed for the air pressure of the airbag to rise from the air pressure S0 to the air pressure SS, the electronic device 100 may perform step S1806.

When t<TT, the electronic device 100 performs the following step S1809.

When TT<t<T, the electronic device 100 performs the following step S1807.

When t>T, the electronic device 100 performs the following step S1810.

S1807: The electronic device 100 determines a value relationship between A_peak and pulse wave fluctuation thresholds A_th1 and A_th2.

In some embodiments, after step S1805 is performed, the electronic device 100 may perform step S1807.

The pulse wave fluctuation threshold A_th1 and the pulse wave fluctuation threshold A_th2 may be preset values, and A_th1<A_th2. It should be noted that a larger pulse wave fluctuation amplitude indicates better quality of a pulse wave signal collected by the electronic device 100, namely, tighter wearing tightness of the user.

When A_peak>A_th2, the electronic device 100 performs the following step S1809.

When A_th1<A_peak<A_th2, the electronic device 100 performs the following step S1808.

When A_peak<A_th1, the electronic device 100 performs the following step S1810.

S1808: The electronic device 100 determines that the current wearing tightness is slightly loose wearing.

S1809: The electronic device 100 determines that the current wearing tightness is proper wearing.

S1810: The electronic device 100 determines that the current wearing tightness is excessively loose wearing.

It may be understood that the embodiment shown in FIG. 18A and FIG. 18B is merely an example. In this embodiment of this application, the physiological parameter 1 may alternatively be a physiological parameter other than the blood pressure, the measurement data of the physiological parameter 1 may alternatively be data other than the pulse wave amplitude, and the wearing tightness threshold and the stop condition may alternatively be content different from that in the foregoing embodiment. This is not limited in this application. In addition, in some other embodiments, the electronic device 100 may not perform step S1806, but directly perform step S1807, to determine the current wearing tightness based on the measurement data of the physiological parameter 1. This is not limited in this application.

According to the measurement method provided in this embodiment of this application, the wearing tightness can be determined based on the measurement data of the physiological parameter 1 while the physiological parameter 1 is measured, so that accuracy of the wearing tightness can be improved, and an error introduced by the wearing tightness to the measurement of the physiological parameter 1 can be further reduced.

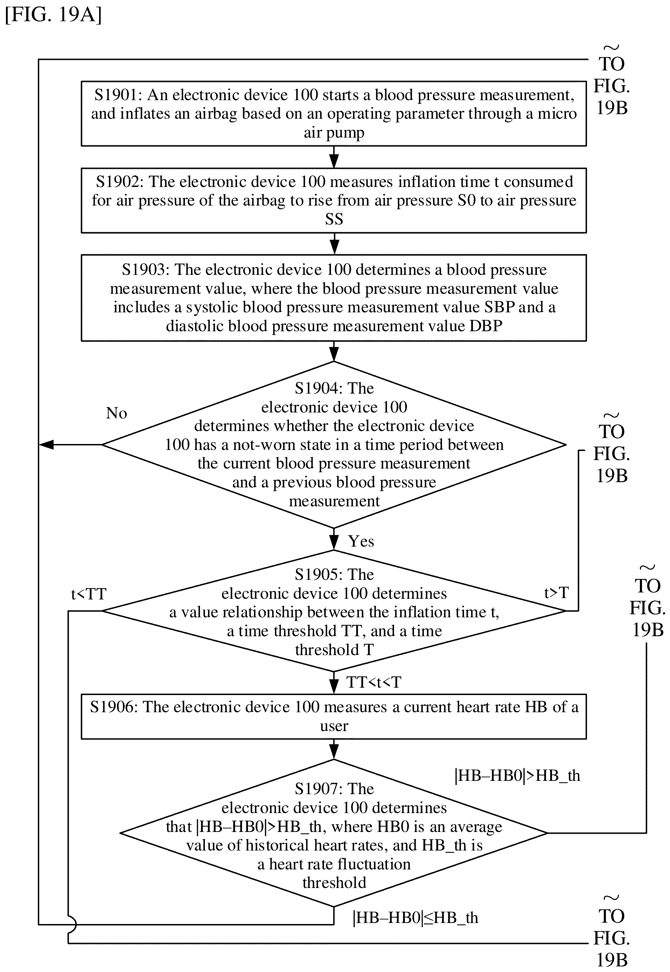

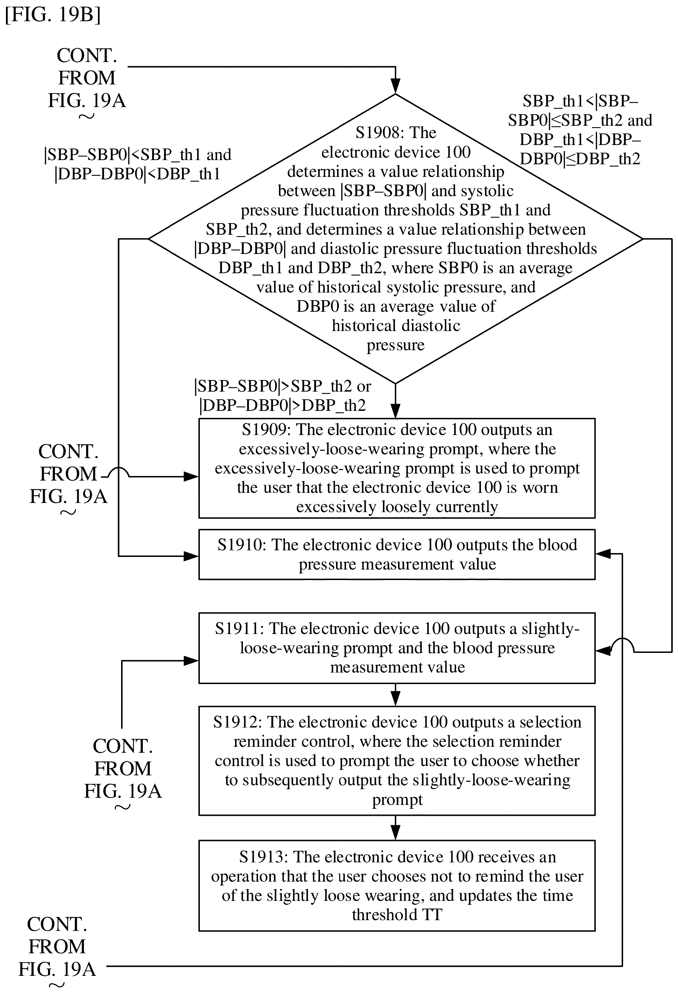

FIG. 19A and FIG. 19B are another schematic flowchart of measuring the physiological parameter 1 by the electronic device 100 according to an embodiment of this application.

For example, the physiological parameter 1 is the blood pressure. As shown in FIG. 19A and FIG. 19B, a specific procedure of measuring the blood pressure by the electronic device 100 may include the following steps.

S1901: The electronic device 100 starts a blood pressure measurement, and inflates the airbag based on the operating parameter through the micro air pump.

S1902: The electronic device 100 measures the inflation time t consumed for the air pressure of the airbag to rise from the air pressure S0 to the air pressure SS.

For specific content of step S1901 and step S1902, refer to the related content in the embodiment shown in FIG. 18A and FIG. 18B. Details are not described herein again.

S1903: The electronic device 100 determines a blood pressure measurement value, where the blood pressure measurement value includes a systolic blood pressure measurement value SBP and a diastolic blood pressure measurement value DBP.

S1904: The electronic device 100 determines whether the electronic device 100 has a not-worn state in a time period between the current blood pressure measurement and a previous blood pressure measurement.

If the electronic device 100 does not have the not-worn state, that is, the electronic device 100 is not removed by the user after the previous blood pressure measurement, the electronic device 100 may determine that the user currently wearing the electronic device 100 is the same as a user wearing the electronic device 100 in the previous blood pressure measurement. In this case, the electronic device 100 may perform the following step S1908.

If the electronic device 100 has the not-worn state, that is, the electronic device 100 has been removed by the user after the previous blood pressure measurement, the electronic device 100 cannot determine whether the user currently wearing the electronic device 100 is the same as a user wearing the electronic device 100 in the previous blood pressure measurement. In this case, the electronic device 100 may perform the following step S1905.

S1905: The electronic device 100 determines a value relationship between the inflation time t, the time threshold TT, and the time threshold T.

When t>T, the electronic device 100 may perform the following step S1909.

When TT<t<T, the electronic device 100 may perform the following step S1906.

When t<TT, the electronic device 100 may perform the following step S1910.

S1906: The electronic device 100 measures a current heart rate HB of the user.

In some embodiments, the electronic device 100 may measure the heart rate of the user through the PPG module. In some other embodiments, the electronic device 100 may alternatively measure the heart rate of the user through another component. This is not limited in this application.

S1907: The electronic device 100 determines that |HB-HB0|>HB_th, where HB0 is an average value of historical heart rates, and HB_th is a heart rate fluctuation threshold.

When |HB-HB0|>HB_th, the electronic device 100 determines that the user currently wearing the electronic device 100 is different from the user wearing the electronic device 100 in the previous blood pressure measurement. In this case, the electronic device 100 may perform the following step S1911.

When |HB-HB0|≤HB_th, the electronic device 100 determines that the user currently wearing the electronic device 100 is the same as the user wearing the electronic device 100 in the previous blood pressure measurement. In this case, the electronic device 100 may perform the following step S1908.

It may be understood that this embodiment herein is merely an example for describing a case that it may be determined, based on a fluctuation between the heart rate and a historically measured heart rate, whether the user currently wearing the electronic device 100 is the same as the user in the previous blood pressure measurement. In this embodiment of this application, the electronic device 100 may alternatively determine, by using a fluctuation of a physiological parameter (for example, blood oxygen saturation, body temperature, or electrocardiogram) other than the heart rate relative to a historical value, or using fluctuations of a plurality of physiological parameters relative to historical values, whether the user currently wearing the electronic device 100 is the same as the user in the previous blood pressure measurement. This is not limited herein in this application.

S1908: The electronic device 100 determines a value relationship between |SBP-SBP0| and systolic pressure fluctuation thresholds SBP_th1 and SBP_th2, and determines a value relationship between |DBP-DBP0| and diastolic pressure fluctuation thresholds DBP_th1 and DBP_th2, where SBP0 is an average value of historical systolic pressure, and DBP0 is an average value of historical diastolic pressure.

When |SBP-SBP0|>SBP_th2 or |DBP-DBP0|>DBP_th2, the electronic device 100 may determine that the electronic device 100 is worn excessively loosely. In this case, the electronic device 100 may perform the following step S1909.

When |SBP-SBPO|<SBP_th1 and [DBP-DBP0|<DBP_th1, the electronic device 100 may determine that the electronic device 100 is worn properly. In this case, the electronic device 100 may perform the following step S1910.

When SBP_th1<|SBP-SBP0|≤SBP_th2 and DBP_th1<|DBP-DBP0|≤DBP_th2, the electronic device 100 may perform the following step S1911.

It may be understood that, herein is merely an example for describing a case that the wearing tightness of the user may be determined by using a fluctuation of the blood pressure measurement value relative to the historical blood pressure measurement value. In some embodiments, the electronic device 100 may alternatively determine the wearing tightness of the user based on a fluctuation of one of the systolic pressure and the diastolic pressure. This is not limited herein in this application.

S1909: The electronic device 100 outputs an excessively-loose-wearing prompt, where the excessively-loose-wearing prompt is used to prompt the user that the electronic device 100 is worn excessively loosely currently.

The electronic device 100 may output the excessively-loose-wearing prompt in any one or more manners such as displaying on a display, voice broadcast, vibration, and indicator blinking.

The excessively-loose-wearing prompt is used to prompt the user that the electronic device 100 is worn excessively loosely currently, and may be further used to prompt to adjust the wearing tightness. In some embodiments, the electronic device 100 may prompt, based on historical measurement data of the wearing tightness, the user to tighten the strap one or two notches.

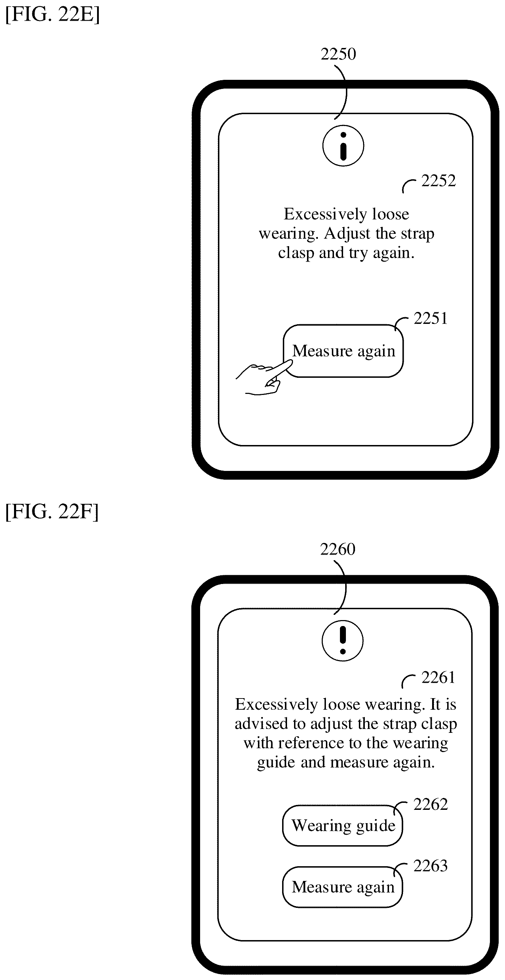

For example, for an interface on which the electronic device 100 outputs the excessively-loose-wearing prompt, refer to related descriptions in the following embodiments shown in FIG. 22E and FIG. 22F.

S1910: The electronic device 100 outputs the blood pressure measurement value.

The electronic device 100 may output the blood pressure measurement value in any one or more manners such as displaying on a display, audio broadcast, vibration, and indicator blinking.

In some embodiments, when outputting the blood pressure measurement value, the electronic device 100 may output any one or more of content such as a heart rate of the user and a blood pressure value range. This is not limited in this application.

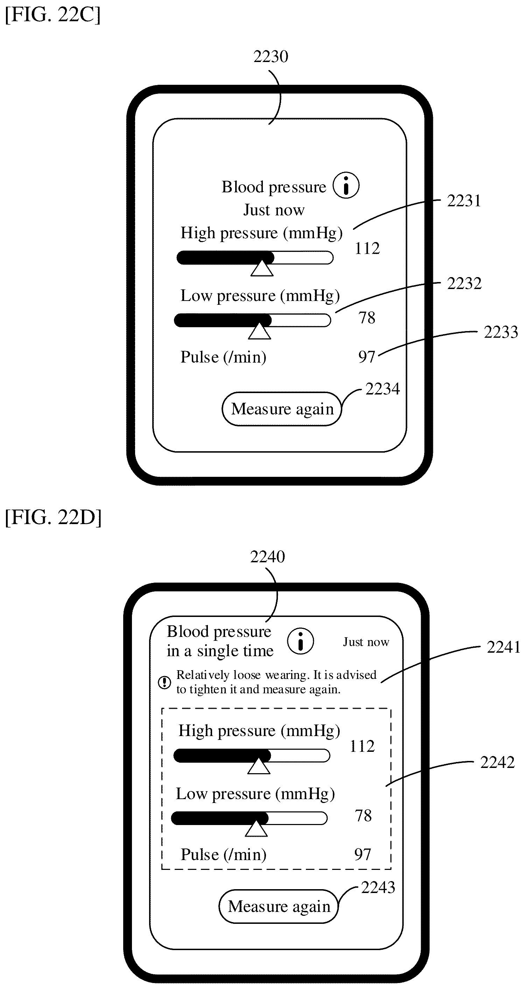

For example, for an interface on which the electronic device 100 outputs the blood pressure measurement value, refer to related descriptions in the following embodiments shown in FIG. 22C and FIG. 22D. Details are not described herein.

S1911: The electronic device 100 outputs a slightly-loose-wearing prompt and the blood pressure measurement value.

The electronic device 100 may output the blood pressure measurement value and the slightly-loose-wearing prompt in any one or more manners such as displaying on a display, audio broadcast, vibration, and indicator blinking. It should be noted that the electronic device 100 may output the blood pressure measurement value and the slightly-loose-wearing prompt at the same time, or may first output the blood pressure measurement value and then output the slightly-loose-wearing prompt, or first output the slightly-loose-wearing prompt and then output the blood pressure measurement value.

In some other embodiments, the electronic device 100 may further calibrate the blood pressure measurement value based on the wearing tightness, to determine a blood pressure calibration value. Then, the electronic device 100 may output the blood pressure calibration value.

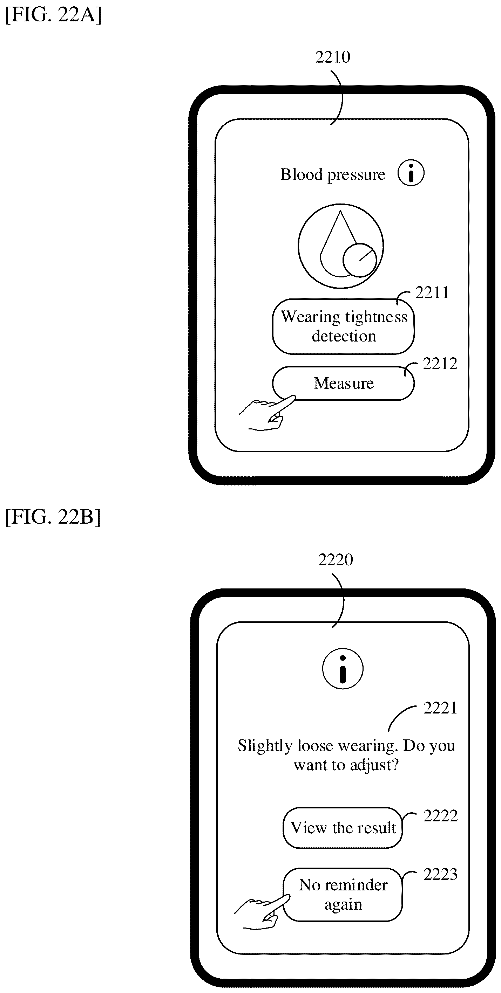

For example, for a specific interface on which the electronic device 100 outputs the slightly-loose-wearing prompt and the blood pressure measurement value, refer to related descriptions in the following embodiments shown in FIG. 22B to FIG. 22D.

S1912: The electronic device 100 outputs a selection reminder control, where the selection reminder control is used to prompt the user to choose whether to subsequently output the slightly-loose-wearing prompt.

In some embodiments, the selection reminder control may include a no-reminder-again control, and the no-reminder-again control is used to trigger the electronic device 100 to update a value of the time threshold TT in step S1905.

For example, for specific content of the selection reminder control, refer to related descriptions in the following embodiment shown in FIG. 22B.

S1913: The electronic device 100 receives an operation that the user chooses not to remind the user of the slightly loose wearing, and updates the time threshold TT.

For example, the operation that the user chooses not to remind the user of the slightly loose wearing may be an operation performed by the user on the no-reminder-again control in the selection reminder control.

Updating the time threshold TT specifically refers to increasing a value of the time threshold TT. It should be noted that, if the wearing tightness threshold in step S1905 is the air pressure threshold Y and the air pressure threshold YY, in step S1913, the electronic device 100 may reduce a value of YY, and the like. It may be understood that, this embodiment herein is merely an example for describing a case that the tightness threshold 2 that is in the wearing tightness threshold and that is used to determine whether the user wears the electronic device 100 properly or slightly loosely may be updated, so that when the user uses the same wearing tightness, no slightly-loose-wearing prompt is output.

In addition, the operation that the user chooses not to remind the user of the slightly loose wearing does not affect the fluctuation threshold of the blood pressure measurement value in step S1908. In other words, if the electronic device 100 determines, based on step S1908, that the user wears the electronic device 100 slightly loosely, the electronic device 100 may further continue to output the slightly-loose-wearing prompt.

In this way, an output frequency of the slightly-loose-wearing prompt can be reduced, and a large error caused by the slightly loose wearing to the blood pressure (or another physiological parameter) measurement can be avoided, to ensure both a user requirement and measurement accuracy.

In some embodiments, the electronic device 100 may alternatively determine a current wearing tightness measurement result based on whether the user removes the electronic device 100 after the previous wearing tightness measurement and a previous wearing tightness measurement result, and determine whether to output the wearing tightness prompt.

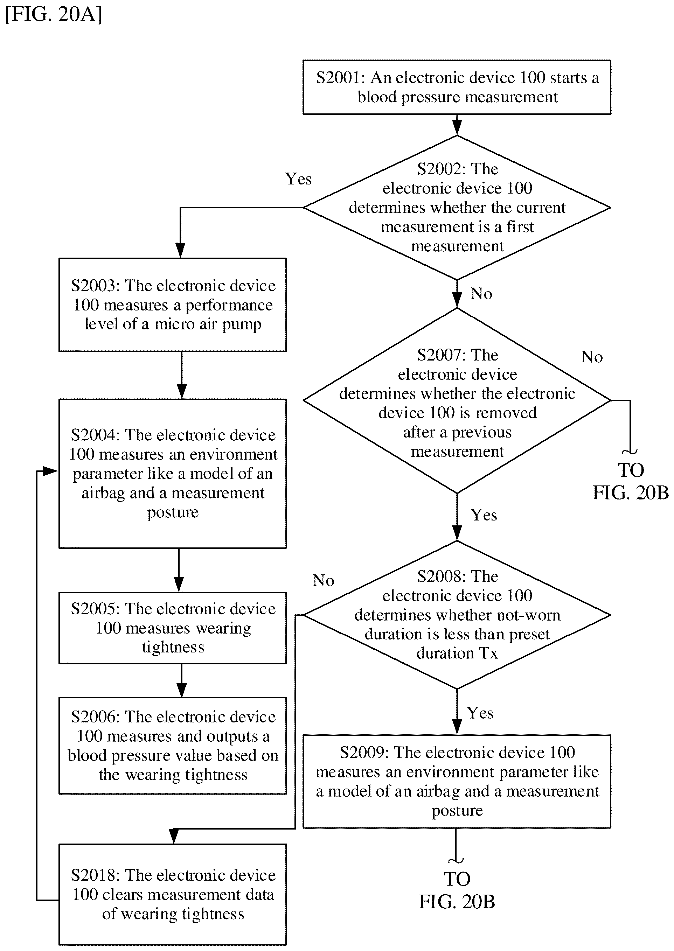

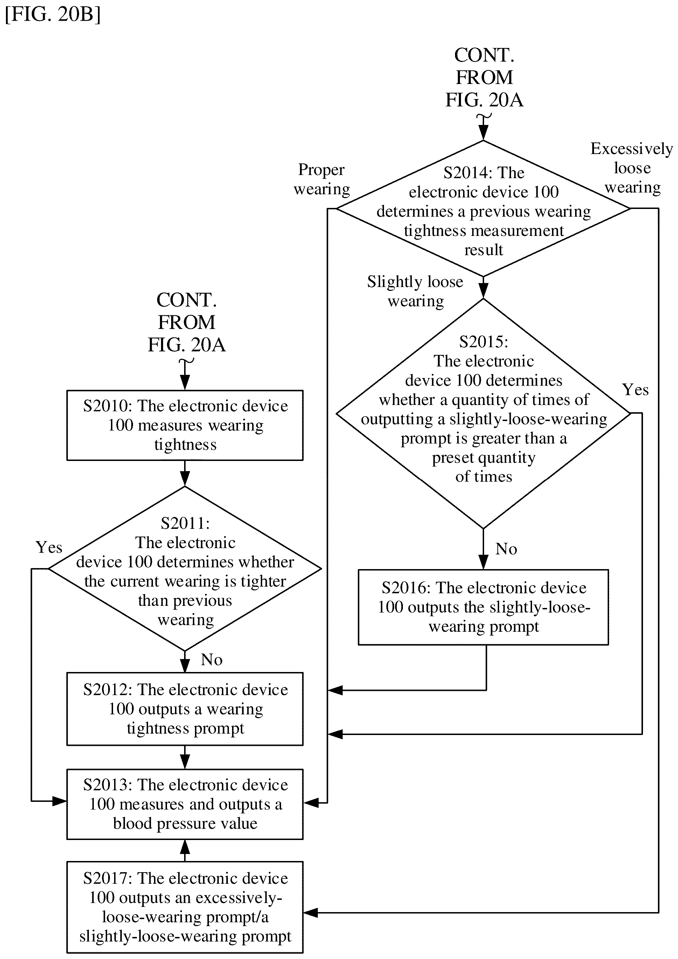

For example, the blood pressure measurement is used as an example. FIG. 20A and FIG. 20B are a schematic flowchart of a blood pressure measurement method according to an embodiment of this application.

As shown in FIG. 20A and FIG. 20B, a specific procedure of the blood pressure measurement method may include the following steps.

S2001: The electronic device 100 starts a blood pressure measurement.

The blood pressure measurement can trigger the electronic device 100 to perform a wearing tightness measurement. In some other embodiments, the blood pressure measurement in step S2001 may alternatively be replaced with another physiological parameter measurement or the like that can trigger the electronic device 100 to perform the wearing tightness measurement. This is not limited in this application.

S2002: The electronic device 100 determines whether the current measurement is a first measurement.

In some embodiments, after determining to perform the wearing tightness measurement, the electronic device 100 may determine whether the current wearing tightness measurement is the first measurement.

In some other embodiments, after determining to perform the blood pressure measurement, the electronic device 100 may alternatively determine whether the current blood pressure measurement is a first blood pressure measurement.

When the current measurement is the first measurement, the electronic device 100 may perform the following step S2003.

When the current measurement is not the first measurement, the electronic device 100 may perform the following step S2007.

S2003: The electronic device 100 measures a performance level of a micro air pump.

When wearing tightness is measured for the first time (or blood pressure is measured for the first time), the electronic device 100 needs to measure performance of the micro air pump. For a specific manner of measuring the performance level of the micro air pump, refer to the related descriptions in the embodiment in FIG. 3 and the like.

In some embodiments, the electronic device 100 may perform step S2003 and step S2004 in parallel, or may first perform step S2003 and then perform step S2004, or may first perform step S2004 and then perform step S2003. A specific execution sequence of step S2003 and step S2004 is not limited in this application.

S2004: The electronic device 100 measures an environment parameter like a model of an airbag and a measurement posture.

For specific content of step S2004, refer to the related descriptions in step S1302 shown in FIG. 13A.

S2005: The electronic device 100 measures the wearing tightness.

After obtaining the environment parameter, the electronic device 100 may measure the wearing tightness based on the environment parameter. The environment parameter may include data obtained in step S2003 and step S2004, for example, any one or more of the performance level of the micro air pump, the model of the airbag, and the measurement posture.

For a specific manner in which the electronic device 100 measures the wearing tightness based on the environment parameter, refer to the related descriptions in step S 1303 to step S1305 shown in FIG. 13A.

S2006: The electronic device 100 measures and outputs a blood pressure value based on the wearing tightness.

After determining the wearing tightness, the electronic device 100 may determine, based on the current wearing tightness, whether to measure blood pressure.

If the current wearing tightness is excessively loose wearing, the electronic device 100 may output an excessively-loose-wearing prompt, to prompt the user with excessively loose wearing and the blood pressure measurement cannot be normally performed.

If the current wearing tightness is slightly loose wearing, the electronic device 100 may measure and output a blood pressure value. Optionally, a slightly-loose-wearing prompt may be further output, and the slightly-loose-wearing prompt is used to prompt the user that the electronic device 100 is worn slightly loosely currently, and an error may exist in the blood pressure measurement.

If the current wearing tightness is proper wearing, the electronic device 100 may measure and output a blood pressure value.

It may be understood that, for a specific process in which the electronic device 100 measures and outputs the blood pressure value based on the wearing tightness, refer to the related descriptions in the embodiment shown in FIG. 17A and FIG. 17B. Details are not described herein again.

S2007: The electronic device 100 determines whether the electronic device 100 is removed after a previous measurement.

If the current wearing tightness measurement (or blood pressure measurement) is not the first measurement, the electronic device 100 may determine whether the user removes the electronic device 100 after the previous wearing tightness measurement (or blood pressure measurement) (that is, whether the electronic device 100 has a not-worn state after the previous measurement).

The electronic device 100 may monitor a wearing status of the electronic device 100. The wearing status indicates whether the user wears the electronic device 100.

If the electronic device 100 has been removed by the user, the electronic device 100 may perform the following step S2008.

If the electronic device 100 has not been removed by the user, it indicates that the electronic device 100 is always in a worn state after the previous measurement. In this case, the electronic device 100 may perform the following step S2014.

S2008: The electronic device 100 determines whether not-worn duration is less than preset duration Tx.

The not-worn duration is total duration in which the electronic device 100 is not worn by the user in a time period from the previous wearing tightness measurement to the current wearing tightness measurement.

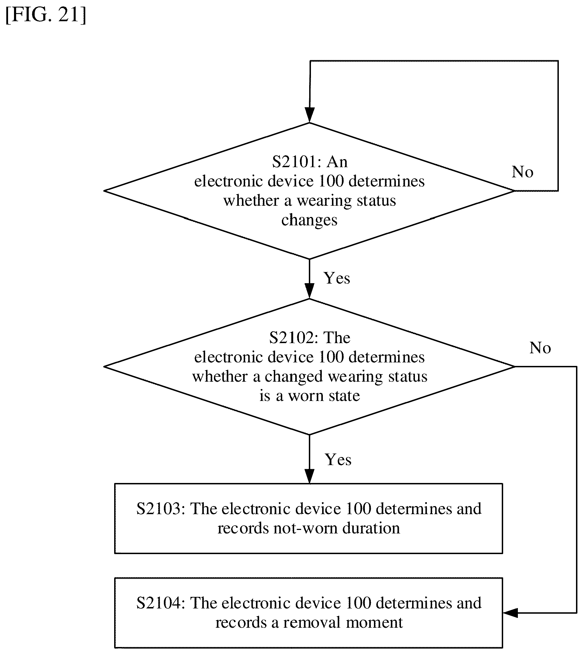

In some embodiments, the electronic device 100 monitors the wearing status of the electronic device 100, and records the not-worn duration of the electronic device 100. For a specific manner of determining the not-worn duration, refer to related steps in the following embodiment shown in FIG. 21. Details are not described herein.

In some application scenarios, if a previous wearing tightness measurement result is slightly loose wearing or excessively loose wearing, the electronic device 100 may output the slightly-loose-wearing prompt or the excessively-loose-wearing prompt, to prompt the user to tighten the wearing. In this case, the user may remove the electronic device 100 and tighten the wearing. In this case, the not-worn duration is short. Therefore, in some embodiments, the not-worn duration may be used to determine whether the user adjusts the wearing tightness.

When the not-worn duration is less than the preset duration Tx, it may be considered that the user adjusts the wearing tightness. In this case, the electronic device 100 may perform the following step S2009.

When the not-worn duration is greater than or equal to the preset duration Tx, the electronic device 100 may perform the following step S2018.

S2009: The electronic device 100 measures an environment parameter like a model of an airbag and a measurement posture.

S2010: The electronic device 100 measures the wearing tightness.

For specific content of step S2009 and step S2010, refer to related content of step S2004 and step S2005.

S2011: The electronic device 100 determines whether the electronic device 100 is worn more tightly currently than previously.

If the electronic device 100 is worn more tightly currently than previously, the electronic device 100 may perform the following step S2013.

If the electronic device 100 is worn more loosely currently than or at a same tightness as previously, the electronic device 100 may perform the following step S2012.

S2012: The electronic device 100 outputs a wearing tightness prompt.

The wearing tightness prompt may include the slightly-loose-wearing prompt and the excessively-loose-wearing prompt, and optionally, may further include a proper-wearing prompt. The wearing tightness prompt is used to prompt the user with the current wearing tightness (for example, slightly loose wearing, excessively loose wearing, or proper wearing).

When the electronic device 100 is worn more loosely currently than or at a same tightness as previously, the electronic device 100 may output the wearing tightness prompt based on the measurement result in step S2010. If the measurement result is slightly loose wearing, the electronic device 100 may output the slightly-loose-wearing prompt, to prompt the user that the electronic device 100 is worn slightly loosely currently. If the measurement result is excessively loose wearing, the electronic device 100 may output the excessively-loose-wearing prompt, to prompt the user that the electronic device 100 is worn excessively loosely currently, and the measurement is performed after the electronic device 100 is tightened/orn tightly. If the measurement result is proper wearing, the electronic device 100 may alternatively output the proper-wearing prompt, to prompt the user that the electronic device 100 is worn properly currently.

When the measurement result is slightly loose wearing or proper wearing, the electronic device 100 may further perform the following step S2013.

S2013: The electronic device 100 measures and outputs a blood pressure value.

For specific content of step S2013, refer to the related descriptions in step S2006.

S2014: The electronic device 100 determines the previous wearing tightness measurement result.

If the electronic device 100 has been removed by the user after the previous measurement, the electronic device 100 may determine whether the previous wearing tightness measurement result is proper wearing.

If the previous wearing tightness measurement result is proper wearing, the electronic device 100 may perform step S2013, to measure and output the blood pressure value.

If the previous wearing tightness measurement result is slightly loose wearing, the electronic device 100 may perform the following step S2015.

If the previous wearing tightness measurement result is excessively loose wearing, the electronic device 100 may perform the following step S2017.

S2015: The electronic device 100 determines whether a quantity of times of outputting the slightly-loose-wearing prompt is greater than a preset quantity of times.

If the previous wearing tightness measurement result is slightly loose wearing, the electronic device 100 may detect a total quantity of times that the electronic device 100 outputs the slightly-loose prompt after the user removes the electronic device 100 and wears the electronic device 100 again last time. The total quantity of times is a quantity of times of outputting the slightly-loose-wearing prompt.

If the quantity of times of outputting the slightly-loose-wearing prompt is greater than the preset quantity of times, it indicates that the user may not want to change the current wearing tightness. In this case, the electronic device 100 may perform step S2013 to measure and output the blood pressure value.

If the quantity of times of outputting the slightly-loose-wearing prompt is less than or equal to the preset quantity of times, the electronic device 100 may perform the following step S2016, to output the slightly-loose-wearing prompt.

S2016: The electronic device 100 outputs the slightly-loose-wearing prompt.

For specific content of step S2016, refer to the related content of step S1711 shown in FIG. 17B.

S2017: The electronic device 100 outputs the excessively-loose-wearing prompt/the slightly-loose-wearing prompt.

The excessively-loose-wearing prompt in step S2017 may be used to prompt the user that the electronic device 100 is worn excessively loosely currently.

In some other embodiments, when the electronic device 100 determines the excessively loose wearing for two consecutive times, it may also be considered that the current wearing tightness is comfortable tightness of the user. In this case, the electronic device 100 may alternatively output the slightly-loose-wearing prompt, to prompt the user that the current wearing tightness may affect the blood pressure measurement value.

After performing step S2017, the electronic device 100 may perform step S2013, to measure and output the blood pressure value.

S2018: The electronic device 100 clears measurement data of the wearing tightness.

Step S2018 is an optional step.

When the not-worn duration is greater than the preset duration Tx, the electronic device 100 may clear the measurement data of the wearing tightness. The measurement data of the wearing tightness includes wearing tightness measured by the electronic device 100 in one or more times. In this way, memory space of the electronic device 100 can be saved.

After step S2018 is performed, the electronic device 100 may perform step S2004 to step S2006.

According to the blood pressure measurement method provided in this embodiment of this application, the current wearing tightness measurement result may be determined based on the previous wearing tightness measurement result.

It may be understood that the embodiment shown in FIG. 20A and FIG. 20B is merely an example. In this embodiment of this application, another physiological parameter measurement related to the wearing tightness measurement may also use a method similar to that in the embodiment shown in FIG. 20A and FIG. 20B. This is not limited in this application.

The following describes a method for measuring the not-worn duration according to an embodiment of this application.

FIG. 21 is a schematic flowchart of the method for measuring the not-worn duration according to an embodiment of this application.

As shown in FIG. 21, a specific procedure of the method for measuring the not-worn duration may include the following steps.

S2101: The electronic device 100 determines whether the wearing status changes.

The wearing status may include two states: the worn state and the not-worn state.

The electronic device 100 may monitor the wearing status. For a specific manner of monitoring the wearing status by the electronic device 100, refer to the related descriptions in step S301 shown in FIG. 3.

Step S2101 may be a step that is repeatedly (for example, periodically) performed.

If the electronic device 100 detects that the wearing status changes from the worn state to the not-worn state, or detects that the wearing status changes from the not-worn state to the worn state, it indicates that the wearing status changes.

If the wearing status changes, the electronic device 100 may perform the following step S2102.

If the wearing status does not change, the electronic device 100 may continue to monitor the wearing status, and repeatedly perform step S2101.

S2102: The electronic device 100 determines whether a changed wearing status is the worn state.

If the changed wearing status is the worn state, the electronic device 100 may perform the following step S2103.

If the changed wearing status is the not-worn state, the electronic device 100 may perform the following step S2104.

S2103: The electronic device 100 determines and records the not-worn duration.

When the electronic device 100 detects that the wearing status changes the not-worn state to the worn state, the electronic device 100 may determine duration of the not-worn state based on a previously recorded removal moment and a current moment. In some embodiments, the duration of the not-worn state is the not-worn duration.

In some other embodiments, the not-worn duration may alternatively be a sum of duration of one or more not-worn states after the previous wearing tightness measurement. In this case, the electronic device 100 may determine duration of each not-worn state after the previous wear tightness measurement, and update the not-worn duration.

S2104: The electronic device 100 determines and records a removal moment.

When detecting that the wearing status changes from the worn state to the not-worn state, the electronic device 100 may determine the current moment as a current removal moment.

In this way, when detecting that the user performs wearing again, the electronic device 100 may determine duration of the current not-worn state based on the removal moment, and determine the not-worn duration.

The following uses the blood pressure measurement scenario as an example to describe a group of interfaces for performing the measurement method by the electronic device 100 provided in this embodiment of this application

FIG. 22A to FIG. 22F are diagrams of a group of interfaces for measuring blood pressure by the electronic device 100 according to an embodiment of this application.

For example, the electronic device 100 may display the home screen 200 shown in FIG. 2A, and the home screen 200 includes a blood pressure application icon 201. The electronic device 100 may receive and respond to a tap operation performed by the user on the blood pressure application icon 201, and display a blood pressure measurement interface 2210 shown in FIG. 22A.

As shown in FIG. 22A, the blood pressure measurement interface 2210 may include a wearing tightness detection control 2211 and a measurement control 2212. The wearing tightness detection control 2211 may be used to trigger the electronic device 100 to detect the current wearing tightness. The measurement control 2212 may be used to trigger the electronic device 100 to measure current blood pressure of the user. It should be noted that, in some embodiments, the measurement control 2212 may alternatively trigger the electronic device 100 to detect the current wearing tightness while measuring the blood pressure.

The electronic device 100 may receive and respond to a tap operation performed by the user on the measurement control 2212, and measure the current wearing tightness of the user.

If the current wearing tightness of the user is slightly loose wearing, the electronic device 100 may display a slightly-loose prompt interface 2220 shown in FIG. 22B.

As shown in FIG. 22B, the slightly-loose prompt interface 2220 may include a slightly-loose-wearing prompt 2221. The slightly-loose-wearing prompt is used to prompt the user that the electronic device 100 is worn slightly loosely currently, and may be further used to query the user whether to adjust the wearing tightness. In some embodiments, the slightly-loose prompt interface 2220 may further include a selection reminder control, for example, a result view control 2222 and a no-reminder-again control 2223. The result view control 2222 is used to trigger the electronic device 100 to output a blood pressure value (for example, a blood pressure measurement value or a blood pressure calibration value). The no-reminder-again control 2223 may be used to trigger the electronic device 100 to stop displaying the slightly-loose prompt interface 2220, and display the blood pressure value. In addition, in some embodiments, after a tap operation performed by the user on the no-reminder-again control 2223 is received, if it is detected again that the user wears the electronic device 100 slightly loosely, the electronic device 100 does not display the slightly-loose prompt interface 2220.

The electronic device 100 may receive and respond to the tap operation performed by the user on the no-reminder-again control 2223 or the result view control 2222. Alternatively, the electronic device 100 may detect that display duration of the slightly-loose prompt interface 2220 reaches preset duration, or receive a tap operation performed by the user on the measurement control 2212. In this case, if the electronic device 100 determines that the current wearing tightness of the user is proper wearing, the electronic device 100 may display an output interface 2230 shown in FIG. 22C.

As shown in FIG. 22C, the output interface 2230 may include a blood pressure value, and the blood pressure value may include a high pressure value 2231 and a low pressure value 2232, respectively corresponding to systolic pressure and diastolic pressure in the current measurement. Optionally, the output interface 2230 may further include a heart rate 2233, and the heart rate 2233 is used to prompt the user with a heart rate measured in the blood pressure measurement process. In some embodiments, the output interface 2230 may further include a measure-again control 2234, and the measure-again control 2234 is configured to trigger the electronic device 100 to measure and output the blood pressure of the user again.

In some embodiments, in response to the received tap operation performed by the user on the measurement control 2212, the electronic device 100 may alternatively display the blood pressure value and the wearing tightness prompt, where the wearing tightness prompt may indicate the current wearing tightness. In this way, the wearing tightness may be output when the blood pressure value is output. In another possible implementation, when detecting that the current wearing tightness is slightly loose wearing, the electronic device 100 may alternatively output the slightly-loose prompt and the blood pressure value at the same time.

For example, when determining that the electronic device 100 is worn slightly loosely, the electronic device 100 may display an output interface 2240 shown in FIG. 22D. As shown in FIG. 22D, the output interface 2240 may include a slightly-loose prompt 2241, a blood pressure value 2242, and a measure-again control 2243. The slightly-loose prompt 2241 may be used to prompt the user that the current wearing tightness is slightly loose wearing. The slightly-loose-wearing prompt 2241 may include a text, for example, "Relatively loose wearing. It is advised to tighten it and measure again." For specific content of the blood pressure value 2242 and the measure-again control 2243, refer to the related descriptions in the embodiment shown in FIG. 22C. Details are not described herein again.

In some embodiments, when the tap operation performed by the user on the measurement control 2212 is received, if the electronic device 100 determines that the current wearing tightness of the user is excessively loose wearing, the electronic device 100 may display an excessively-loose prompt interface 2250 shown in FIG. 22E.

As shown in FIG. 22E, the excessively-loose prompt interface 2250 may include a measure-again control 2251 and an excessively-loose-wearing prompt 2252. The measure-again control 2251 is used to trigger the electronic device 100 to measure the blood pressure of the user again. The excessively-loose-wearing prompt 2252 may be used to prompt the user that the electronic device 100 is worn excessively loosely currently, and the blood pressure cannot be normally measured. For example, the excessively-loose-wearing prompt 2252 may include a text like "Excessively loose wearing. Adjust the strap clasp and try again".

In some other embodiments, when the tap operation performed by the user on the measurement control 2212 is received, if the electronic device 100 determines that the current wearing tightness of the user is excessively loose wearing, the electronic device 100 may display an excessively-loose prompt interface 2260 shown in FIG. 22F.

As shown in FIG. 22F, the excessively-loose prompt interface 2260 may include an excessively-loose-wearing prompt 2261, a wearing guide control 2262, and a measure-again control 2263. The excessively-loose-wearing prompt 2261 may be used to prompt the user with excessively loose wearing, and prompt the user to adjust the wearing tightness with reference to a wearing guide. For example, the excessively-loose-wearing prompt 2261 may include a text "Excessively loose wearing. It is advised to adjust the strap clasp with reference to the wearing guide and measure again." The wearing guide control 2262 may be used to trigger the electronic device 100 to display the wearing guide, and the wearing guide is used to guide the user to adjust the wearing tightness. The measure-again control 2263 may be used to trigger the electronic device 100 to measure the blood pressure again. It may be understood that FIG. 22A to FIG. 22E merely describe an example of application of the measurement method provided in embodiments of this application in the blood pressure measurement scenario. In some embodiments, the control used to trigger performance detection of the micro air pump may alternatively be displayed on an interface for measuring another physiological parameter (for example, the heart rate or the body temperature). This is not limited in this application.

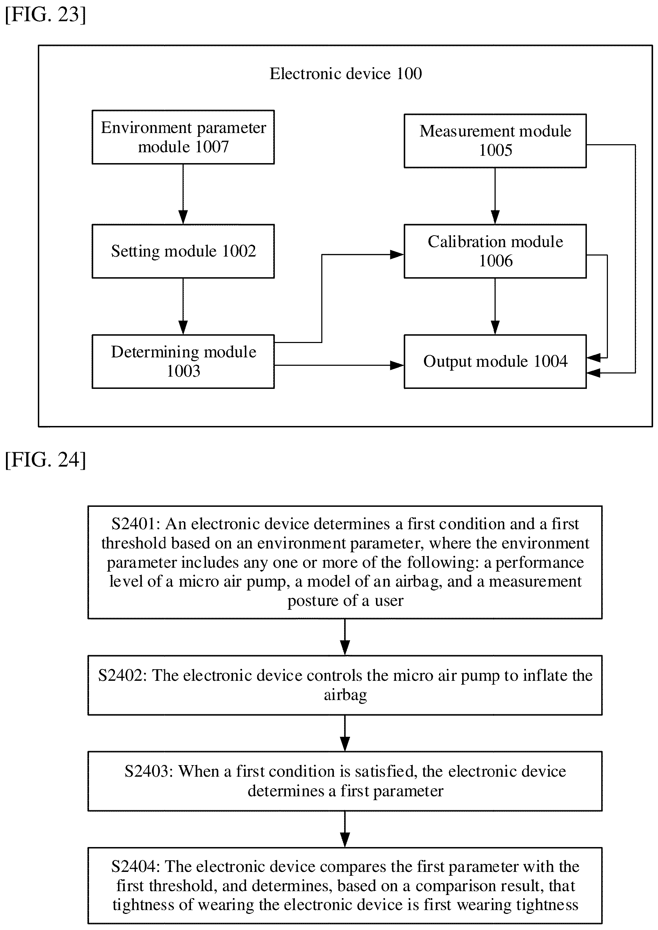

FIG. 23 is a diagram of functional modules of an electronic device 100 according to an embodiment of this application.

As shown in FIG. 23, the electronic device 100 may include an environment parameter module 1007, a setting module 1002, a determining module 1003, an output module 1004, a measurement module 1005, and a calibration module 1006.

For specific functions of the setting module 1002, the determining module 1003, the output module 1004, the measurement module 1005, and the calibration module 1006, refer to the related descriptions in the embodiment shown in FIG. 10. Details are not described herein again.

The environment parameter module 1007 obtains an environment parameter. The environment parameter may include any one or more of the following: a model of an airbag, a performance level of a micro air pump, a measurement posture, and the like. The environment parameter module 1007 may send the obtained environment parameter to the setting module 1002. In a possible implementation, the environment parameter module 1007 may include the performance detection module 1001 shown in FIG. 10.

It may be understood that the embodiment shown in FIG. 23 is merely an example. In this embodiment of this application, the electronic device 100 may further include more or fewer modules than those in the embodiment shown in FIG. 23, or a plurality of functional modules shown in FIG. 23 are integrated into one functional module, or one functional module shown in FIG. 23 is divided into a plurality of functional modules. This is not limited in this application.

FIG. 24 is a schematic flowchart of another measurement method according to an embodiment of this application.

As shown in FIG. 24, a specific procedure of the another measurement method may include the following steps.

S2401: An electronic device determines a first condition and a first threshold based on an environment parameter, where the environment parameter includes any one or more of the following: a performance level of a micro air pump, a model of an airbag, and a measurement posture of a user.

The electronic device includes the micro air pump and the airbag. In this embodiment of this application, the electronic device may be the electronic device 100 in the foregoing embodiment.

For specific content and a measurement manner of the environment parameter, refer to the related descriptions in the embodiment shown in FIG. 13A.

The first condition may be the stop condition in the embodiment shown in FIG. 13A, and the first threshold may be the wearing tightness threshold in the embodiment shown in FIG. 13A.

S2402: The electronic device controls the micro air pump to inflate the airbag.

S2403: When a first condition is satisfied, the electronic device determines a first parameter.

In a possible implementation, the first condition is that inflation time of the micro air pump reaches fifth duration, the first threshold includes a fifth air pressure value, and the first parameter is air pressure of the airbag when the first condition is satisfied.

In a possible implementation, the first condition is that the air pressure of the airbag reaches a seventh air pressure value, the first threshold includes sixth duration, and the first parameter is inflation time of the micro air pump when the first condition is satisfied. For example, in the embodiment shown in FIG. 14, the seventh air pressure value may be the air pressure SS, and the sixth duration may include the time threshold T and the time threshold TT.

It may be understood that the foregoing two implementations are merely two examples. In this embodiment of this application, the first condition, the first threshold, and the first parameter may be different from those in the foregoing examples. For details, refer to the related descriptions in the embodiment shown in FIG. 13A. Details are not described herein again.

S2404: The electronic device compares the first parameter with the first threshold, and determines, based on a comparison result, that tightness of wearing the electronic device is first wearing tightness.

For a specific manner in which the electronic device determines the first wearing tightness based on the first parameter and the first threshold, refer to the related descriptions of step S1305 shown in FIG. 13A. Details are not described herein again.

In this way, the wearing tightness of the user can be measured based on the environment parameter, to avoid a measurement error caused by different environment parameters, and improve measurement accuracy of the wearing tightness.

In a possible implementation, the wearing tightness includes proper wearing, slightly loose wearing, and excessively loose wearing.

It should be noted that, in embodiments of this application, slightly loose wearing may also be referred to as little loose wearing.

In a possible implementation, the method further includes: determining, based on the environment parameter, that an operating parameter of the micro air pump is a first operating parameter; and controlling the micro air pump to inflate the airbag specifically includes: controlling the micro air pump to inflate the airbag based on the first operating parameter.

In this way, the operating parameter of the micro air pump can be set based on the environment parameter.

In a possible implementation, the electronic device further includes a Hall effect sensor; and before determining the first condition and the first threshold based on the environment parameter, the method further includes: measuring the model of the airbag based on the Hall effect sensor.

For a specific manner in which the electronic device measures the model of the airbag based on the Hall effect sensor, refer to the related descriptions of step S1302 shown in FIG. 13A, or refer to the related descriptions in the embodiment shown in FIG. 1D.

In this way, the model of the airbag can be measured.

In a possible implementation, the electronic device further includes an inertial measurement sensor, the inertial measurement sensor includes an acceleration sensor and/or a gyroscope sensor, and before determining the first condition and the first threshold based on the environment parameter, the method further includes: measuring the measurement posture of the user based on the inertial measurement sensor.

For a specific manner in which the electronic device measures the measurement posture of the user based on the inertial measurement sensor, refer to the related descriptions of step S1302 shown in FIG. 13A.

In this way, the measurement posture of the user can be obtained.

In a possible implementation, before determining the first condition and the first threshold based on the environment parameter, the method further includes: setting the operating parameter of the micro air pump to the first operating parameter; setting initial air pressure of the airbag to a first air pressure value, and setting end air pressure of the airbag to a second air pressure value; controlling the micro air pump to inflate the airbag based on the first operating parameter, and obtaining first duration consumed for air pressure of the airbag to change from the first air pressure value to the second air pressure value; and determining the performance level of the micro air pump based on the first operating parameter, the first air pressure value, the second air pressure value, and the first duration.

For a specific manner of measuring the performance level of the micro air pump, refer to the related descriptions in the embodiment shown in FIG. 3.

In this way, the performance level of the micro air pump can be measured.

In a possible implementation, measuring the model of the airbag based on the Hall effect sensor specifically includes: when a first measurement condition is satisfied, measuring the model of the airbag based on the Hall effect sensor, where the first measurement condition includes any one or more of the following: The first wearing tightness is measured for a first time, and the user removes and mounts the airbag.

In this way, when the first measurement condition is satisfied, the measurement of the model of the airbag may be triggered. There is no need to measure the model of the airbag each time the wearing tightness is measured, so that power consumption is reduced.

In a possible implementation, obtaining the performance level of the micro air pump specifically includes: when a second measurement condition is satisfied, obtaining the performance level of the micro air pump, where the second measurement condition includes any one or more of the following: The first wearing tightness is measured for a first time, and a time interval from measuring the performance level of the micro air pump last time reaches a first time interval.

In this way, when the second measurement condition is satisfied, the measurement of the performance level of the micro air pump may be triggered. There is no need to measure the performance level of the micro air pump each time the wearing tightness is measured, so that power consumption is reduced.

In a possible implementation, if the first wearing tightness is proper wearing, the method further includes: controlling the micro air pump to inflate the airbag to obtain a first blood pressure measurement value; and outputting the first blood pressure measurement value.

In a possible implementation, the wearing tightness includes proper wearing, slightly loose wearing, and excessively loose wearing; and if the first wearing tightness is slightly loose wearing, the method further includes: obtaining a maximum fluctuation value of a pulse wave amplitude; and determining second wearing tightness based on the maximum fluctuation value of the pulse wave amplitude and a preset fluctuation threshold.

For a specific manner of determining the wearing tightness of the user based on the fluctuation of the pulse wave amplitude, refer to the related steps in the embodiment shown in FIG. 18A and FIG. 18B.

In this way, in the case of slightly loose wearing, the wearing tightness of the user may be further determined based on fluctuation of the pulse wave amplitude.

In a possible implementation, if the first wearing tightness is slightly loose wearing, the method further includes: displaying a slightly-loose-wearing prompt, where the slightly-loose-wearing prompt includes a first control, the first control is used to trigger the electronic device not to display the slightly-loose-wearing prompt when slightly loose wearing is detected, and the slightly-loose-wearing prompt is used to prompt the user that the first wearing tightness is slightly loose wearing.

For example, the first control may be the selection reminder control in the embodiment shown in FIG. 19A and FIG. 19B. For another example, the first control may also be the no-reminder-again control 2223 in the embodiment shown in FIG. 22B.

In this way, in the case of slightly loose wearing, the slightly-loose-wearing prompt may be output. In addition, the first control used to trigger the electronic device not to display the slightly-loose-wearing prompt may be further displayed.

In a possible implementation, the method further includes: receiving and responding to an operation on the first control, and stopping displaying the slightly-loose-wearing prompt; receiving an operation of measuring the tightness of wearing the electronic device; determining the first condition and the first threshold based on the environment parameter; controlling the micro air pump to inflate the airbag; when the first condition is satisfied, determining a second parameter; comparing the second parameter with the first threshold, and determining, based on a comparison result, that the tightness of wearing the electronic device is third wearing tightness; and if the third wearing tightness is slightly loose wearing, skipping displaying the slightly-loose-wearing prompt.

In this way, based on a user operation, the slightly-loose-wearing prompt may not be displayed.

In a possible implementation, the method further includes: before determining the first condition and the first threshold based on the environment parameter, receiving an operation of starting a wearing tightness measurement; detecting that the electronic device has a not-worn state between a current wearing tightness measurement and a previous wearing tightness measurement; and if the first tightness is tighter than fourth tightness measured last time, skipping displaying the slightly-loose-wearing prompt.

For specific content of this possible implementation, refer to the related content in the embodiment shown in FIG. 20A and FIG. 20B.

In this way, if the electronic device is removed by the user between two wearing tightness measurements, and the wearing tightness measured this time is tighter than that measured last time, no slightly-loose-wearing prompt is output regardless of whether the wearing tightness measured this time is slightly loose wearing.

In a possible implementation, the method further includes: if the first tightness is tighter than the fourth tightness measured last time, measuring and outputting a second blood pressure measurement value.

For specific content of this possible implementation, refer to the related content in the embodiment shown in FIG. 20A and FIG. 20B.

In this way, when the wearing tightness measured this time is tighter than that measured last time, the electronic device determines that the electronic device is worn properly, and measures and outputs a blood pressure value.

In a possible implementation, duration of the not-worn state of the electronic device is less than preset removal duration.

For specific content of this possible implementation, refer to the related content in the embodiment shown in FIG. 20A and FIG. 20B.

In this way, the electronic device may determine, based on a value relationship between the not-worn duration and the preset removal duration, whether the user adjusts the wearing tightness. If the not-worn duration is less than the preset removal duration, the electronic device may determine that the user adjusts the wearing tightness. If the not-worn duration is greater than or equal to the preset removal duration, the electronic device may determine that the user removes the electronic device.

Implementations of this application may be randomly combined to achieve different technical effect.

All or some of the foregoing embodiments may be implemented by using software, hardware, firmware, or any combination thereof. When software is used for implementation, all or some of the methods may be implemented in a form of a computer program product. The computer program product includes one or more computer instructions. When the computer program instructions are loaded and executed on a computer, the procedures or functions according to this application are all or partially generated. The computer may be a general-purpose computer, a dedicated computer, a computer network, or other programmable apparatuses. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

A person of ordinary skill in the art may understand that all or some of the processes of the methods in embodiments may be implemented by a computer program instructing related hardware. The program may be stored in a computer-readable storage medium. When the program runs, the processes of the methods in embodiments are performed. The storage medium includes any medium that can store program code, such as a ROM, a random access memory RAM, a magnetic disk, or an optical disc.

In summary, what is described above is merely embodiments of the technical solutions of this application, but is not intended to limit the protection scope of this application. Any modification, equivalent replacement, or improvement made according to the disclosure of this application shall fall within the protection scope of this application.

## Claims

1. A measurement method, applied to an electronic device, wherein the electronic device comprises a micro air pump and an airbag, and the method comprises:
determining a first condition and a first threshold based on an environment parameter, wherein the environment parameter comprises any one or more of the following: a performance level of the micro air pump, a model of the airbag, and a measurement posture of a user;
controlling the micro air pump to inflate the airbag;
when the first condition is satisfied, determining a first parameter; and
comparing the first parameter with the first threshold, and determining, based on a comparison result, that tightness of wearing the electronic device is first wearing tightness.

2. The method according to claim 1, wherein the wearing tightness comprises proper wearing, slightly loose wearing, and excessively loose wearing.

3. The method according to claim 1 or 2, wherein the method further comprises:
determining, based on the environment parameter, that an operating parameter of the micro air pump is a first operating parameter; and
controlling the micro air pump to inflate the airbag specifically comprises:
controlling the micro air pump to inflate the airbag based on the first operating parameter.

4. The method according to any one of claims 1 to 3, wherein the electronic device further comprises a Hall effect sensor, and before determining the first condition and the first threshold based on the environment parameter, the method further comprises:
measuring the model of the airbag based on the Hall effect sensor.

5. The method according to any one of claims 1 to 4, wherein the electronic device further comprises an inertial measurement sensor, the inertial measurement sensor comprises an acceleration sensor and/or a gyroscope sensor, and before determining the first condition and the first threshold based on the environment parameter, the method further comprises:
measuring the measurement posture of the user based on the inertial measurement sensor.

6. The method according to any one of claims 1 to 5, wherein before determining the first condition and the first threshold based on the environment parameter, the method further comprises:
setting the operating parameter of the micro air pump to the first operating parameter;
setting initial air pressure of the airbag to a first air pressure value and end air pressure of the airbag to a second air pressure value;
controlling the micro air pump to inflate the airbag based on the first operating parameter, and obtaining first duration consumed for air pressure of the airbag to change from the first air pressure value to the second air pressure value; and
determining the performance level of the micro air pump based on the first operating parameter, the first air pressure value, the second air pressure value, and the first duration.

7. The method according to claim 4, wherein measuring the model of the airbag based on the Hall effect sensor specifically comprises:
when a first measurement condition is satisfied, measuring the model of the airbag based on the Hall effect sensor, wherein the first measurement condition comprises any one or more of the following: the first wearing tightness is measured for a first time, and the user removes and mounts the airbag.

8. The method according to claim 6, wherein obtaining the performance level of the micro air pump specifically comprises:
when a second measurement condition is satisfied, obtaining the performance level of the micro air pump, wherein the second measurement condition comprises any one or more of the following: the first wearing tightness is measured for a first time, and a time interval from measuring the performance level of the micro air pump last time reaches a first time interval.

9. The method according to any one of claims 1 to 8, wherein the first condition is that inflation time of the micro air pump reaches fifth duration, the first threshold comprises a fifth air pressure value, and the first parameter is air pressure of the airbag when the first condition is satisfied.

10. The method according to any one of claims 1 to 8, wherein the first condition is that the air pressure of the airbag reaches a seventh air pressure value, the first threshold comprises sixth duration, and the first parameter is inflation time of the micro air pump when the first condition is satisfied.

11. The method according to any one of claims 1 to 10, wherein if the first wearing tightness is proper wearing, the method further comprises:
controlling the micro air pump to inflate the airbag to obtain a first blood pressure measurement value; and
outputting the first blood pressure measurement value.

12. The method according to any one of claims 1 to 11, wherein the wearing tightness comprises proper wearing, slightly loose wearing, and excessively loose wearing; and if the first wearing tightness is slightly loose wearing, the method further comprises:
obtaining a maximum fluctuation value of a pulse wave amplitude; and
determining second wearing tightness based on the maximum fluctuation value of the pulse wave amplitude and a preset fluctuation threshold.

13. The method according to any one of claims 1 to 12, wherein if the first wearing tightness is slightly loose wearing, the method further comprises:
displaying a slightly-loose-wearing prompt, wherein the slightly-loose-wearing prompt comprises a first control, the first control is used to trigger the electronic device not to display the slightly-loose-wearing prompt when slightly loose wearing is detected, and the slightly-loose-wearing prompt is used to prompt the user that the first wearing tightness is slightly loose wearing.

14. The method according to claim 13, wherein the method further comprises:
receiving and responding to an operation on the first control, and stopping displaying the slightly-loose-wearing prompt;
receiving an operation of measuring the tightness of wearing the electronic device;
determining the first condition and the first threshold based on the environment parameter;
controlling the micro air pump to inflate the airbag;
when the first condition is satisfied, determining a second parameter;
comparing the second parameter with the first threshold, and determining, based on a comparison result, that the tightness of wearing the electronic device is third wearing tightness; and
if the third wearing tightness is slightly loose wearing, skipping displaying the slightly-loose-wearing prompt.

15. The method according to any one of claims 1 to 14, wherein the method further comprises:
before determining the first condition and the first threshold based on the environment parameter, receiving an operation of starting a wearing tightness measurement;
detecting that the electronic device has a not-worn state between the current wearing tightness measurement and a previous wearing tightness measurement; and
if the first tightness is tighter than fourth tightness measured last time, skipping displaying the slightly-loose-wearing prompt.

16. The method according to claim 15, wherein the method further comprises:
if the first tightness is tighter than the fourth tightness measured last time, measuring and outputting a second blood pressure measurement value.

17. The method according to claim 15 or 16, wherein duration of the not-worn state of the electronic device is less than preset removal duration.

18. An electronic device, comprising one or more processors, one or more memories, a micro air pump, and an airbag, wherein the one or more memories are coupled to the one or more processors, the one or more memories are configured to store computer instructions, and when the one or more processors execute the computer instructions, the measurement method according to any one of claims 1 to 17 is implemented.

19. A computer-readable storage medium, comprising computer instructions, wherein when the computer instructions are executed by a processor, the measurement method according to any one of claims 1 to 17 is implemented.

20. A chip system, wherein the chip system comprises a processing circuit and an interface circuit, the interface circuit is configured to receive code instructions and transmit the code instructions to the processing circuit, and the processing circuit is configured to run the code instructions to perform the measurement method according to any one of claims 1 to 17.

21. A computer program product, comprising computer instructions, wherein when the computer program is executed by a processor, the measurement method according to any one of claims 1 to 17 is implemented.
